# EUROPÄISCHE PATENTSCHRIFT

(11) **EP 2 121 765 B1**
(45) Veröffentlichungstag und Bekanntmachung des Hinweises auf die Patenterteilung: **05.06.2013**
(21) Anmeldenummer: 07870193.5
(22) Anmeldetag: 21.12.2007
(51) Int. Cl.: C07K 19/00, C12N 15/62, C12N 9/88, C07K 14/315, G01N 33/569

(54) **VERFAHREN UND MITTEL ZUR ANREICHERUNG, ENTFERNUNG UND ZUM NACHWEIS VON GRAM-POSITIVEN BAKTERIEN**
METHOD AND MEANS FOR CONCENTRATING, REMOVING, AND DETECTING GRAM-POSITIVE BACTERIA
PROCEDE ET AGENT D'ENRICHISSEMENT, D'ELIMINATION ET DE DETECTION DE BACTERIES GRAM POSITIF

(30) Priorität: 22.12.2006 DE 102006061002
(43) Veröffentlichungstag der Anmeldung: 25.11.2009
(73) Patentinhaber: Biomérieux, 69280 Marcy L'etoile (FR)
(72) Erfinder: BEISSINGER, Martina, 93055 Regensburg (DE); MILLER, Stefan, 93055 Regensburg (DE); PHILIPP, Anja, 93053 Regensburg (DE); SCHÜTZ, Michael, 93059 Regensburg (DE)
(74) Vertreter: Dehmel, Albrecht
(86) Internationale Anmeldenummer: PCT/DE2007/002320
(87) Internationale Veröffentlichungsnummer: WO 2008/077397

(56) Entgegenhaltungen:
- WO-A-90/14431
- WO-A-2004/005482
- WO-A-2004/027020
- WO-A-2004/088321
- WO-A-2004/106367
- WO-A-2007/022768
- DE-A1- 19 837 751
- US-A- 5 252 466
- JOCKEL P ET AL: "Membrane topology of the beta-subunit of the oxaloacetate decarboxylase Na+ pump from Klebsiella pneumoniae." BIOCHEMISTRY 12 OCT 1999, Bd. 38, Nr. 41, 12. Oktober 1999 (1999-10-12), Seiten 13461-13472, XP002488770 ISSN: 0006-2960
- CONSLER T G ET AL: "Properties and purification of an active biotinylated lactose permease from Escherichia coli." PROCEEDINGS OF THE NATIONAL ACADEMY OF SCIENCES OF THE UNITED STATES OF AMERICA 1 AUG 1993, Bd. 90, Nr. 15, 1. August 1993 (1993-08-01), Seiten 6934-6938, XP002488771 ISSN: 0027-8424

### SEQUENCE LISTING

<110> PROFOS AG
<120> Verfahren und Mittel zur Anreicherung, Entfernung und zum Nachweis von gram-positiven Bakterien
<130> PRO-025 PCT
<140> unknown
   <141> 2007-12-21
<150> DE 10 2006 061 002.4 <151> 2006-12-22
<160> 56
<170> PatentIn version 3.3
<210> 1
   <211> 66
   <212> PRT
   <213> Artificial
<220>
   <223> JS-Tag
<400> 1
   Val Thr Ala Pro Leu Ala Gly Thr Ile Trp Lys Val Leu Ala Ser Glu
   1               5                   10                  15
   Gly Gln Thr Val Ala Ala Gly Glu Val Leu Leu Ile Leu Glu Ala Met
               20                  25                  30
   Lys Met Glu Thr Glu Ile Arg Ala Ala Gln Ala Gly Thr Val Arg Gly
           35                  40                  45
   Ile Ala Val Lys Ala Gly Asp Ala Val Ala Val Gly Glu Thr Leu Met
       50                  55                  60
   Thr Leu
   65
<210> 2
   <211> 66
   <212> PRT
   <213> Klebsiella pneumoniae
<400> 2
   Val Thr Ala Pro Leu Ala Gly Thr Ile Trp Lys Val Leu Ala Ser Glu
   1               5                   10                  15
   Gly Gln Thr Val Ala Ala Gly Glu Val Leu Leu Ile Leu Glu Ala Met
               20                  25                  30
   Lys Met Glu Thr Glu Ile Arg Ala Ala Gln Ala Gly Thr Val Arg Gly
           35                  40                  45
   Ile Ala Val Lys Ala Gly Asp Ala Val Ala Val Gly Asp Thr Leu Met
       50                  55                  60
   Thr Leu
   65
<210> 3
   <211> 67
   <212> PRT
   <213> Artificial
<220>
   <223> JS-TAG
<400> 3
   Val Thr Ala Pro Leu Ala Gly Thr Ile Trp Lys Val Leu Ala Ser Glu
   1               5                   10                  15
   Gly Gln Thr Val Ala Ala Gly Glu Val Leu Leu Ile Leu Glu Ala Met
               20                  25                  30
   Lys Met Glu Thr Glu Ile Arg Ala Ala Gln Ala Gly Thr Val Arg Gly
           35                  40                  45
   Ile Ala Val Lys Ala Gly Asp Ala Val Ala Val Gly Glu Thr Leu Met
       50                  55                  60
   Thr Leu Val
   65
<210> 4
   <211> 68
   <212> PRT
   <213> Artificial
<220>
   <223> JS-TAG
<400> 4
   Val Thr Ala Pro Leu Ala Gly Thr Ile Trp Lys Val Leu Ala Ser Glu
   1               5                   10                  15
   Gly Gln Thr Val Ala Ala Gly Glu Val Leu Leu Ile Leu Glu Ala Met
               20                  25                  30
   Lys Met Glu Thr Glu Ile Arg Ala Ala Gln Ala Gly Thr Val Arg Gly
           35                  40                  45
   Ile Ala Val Lys Ala Gly Asp Ala Val Ala Val Gly Glu Thr Leu Met
       50                  55                  60
   Thr Leu Val Asp
   65
<210> 5
   <211> 67
   <212> PRT
   <213> Artificial
<220>
   <223> JS-TAG
<400> 5
   Met Val Thr Ala Pro Leu Ala Gly Thr Ile Trp Lys Val Leu Ala Ser
   1               5                   10                  15
   Glu Gly Gln Thr Val Ala Ala Gly Glu Val Leu Leu Ile Leu Glu Ala
               20                  25                  30
   Met Lys Met Glu Thr Glu Ile Arg Ala Ala Gln Ala Gly Thr Val Arg
           35                  40                  45
   Gly Ile Ala Val Lys Ala Gly Asp Ala Val Ala Val Gly Glu Thr Leu
       50                  55                  60
   Met Thr Leu
   65
<210> 6
   <211> 68
   <212> PRT
   <213> Artificial
<220>
   <223> JS-TAG
<400> 6
   Met Val Thr Ala Pro Leu Ala Gly Thr Ile Trp Lys Val Leu Ala Ser
   1               5                   10                  15
   Glu Gly Gln Thr Val Ala Ala Gly Glu Val Leu Leu Ile Leu Glu Ala
               20                  25                  30
   Met Lys Met Glu Thr Glu Ile Arg Ala Ala Gln Ala Gly Thr Val Arg
           35                  40                  45
   Gly Ile Ala Val Lys Ala Gly Asp Ala Val Ala Val Gly Glu Thr Leu
       50                  55                  60
   Met Thr Leu Val
   65
<210> 7
   <211> 69
   <212> PRT
   <213> Artificial
<220>
   <223> JS-TAG
<400> 7
   Met Val Thr Ala Pro Leu Ala Gly Thr Ile Trp Lys Val Leu Ala Ser
   1               5                   10                  15
   Glu Gly Gln Thr Val Ala Ala Gly Glu Val Leu Leu Ile Leu Glu Ala
               20                  25                  30
   Met Lys Met Glu Thr Glu Ile Arg Ala Ala Gln Ala Gly Thr Val Arg
           35                  40                  45
   Gly Ile Ala Val Lys Ala Gly Asp Ala Val Ala Val Gly Glu Thr Leu
       50                  55                  60
   Met Thr Leu Val Asp
   65
<210> 8
   <211> 70
   <212> PRT
   <213> Artificial
<220>
   <223> JS-TAG
<400> 8
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu
   65                  70
<210> 9
   <211> 71
   <212> PRT
   <213> Artificial
<220>
   <223> JS-TAG
<400> 9
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Val
   65                  70
<210> 10
   <211> 72
   <212> PRT
   <213> Artificial
<220>
   <223> JS-TAG
<400> 10
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Val Asp
   65                  70
<210> 11
   <211> 67
   <212> PRT
   <213> Artificial
<220>
   <223> JS-TAG
<400> 11
   Val Thr Ala Pro Leu Ala Gly Thr Ile Trp Lys Val Leu Ala Ser Glu
   1               5                   10                  15
   Gly Gln Thr Val Ala Ala Gly Glu Val Leu Leu Ile Leu Glu Ala Met
               20                  25                  30
   Lys Met Glu Thr Glu Ile Arg Ala Ala Gln Ala Gly Thr Val Arg Gly
           35                  40                  45
   Ile Ala Val Lys Ala Gly Asp Ala Val Ala Val Gly Asp Thr Leu Met
       50                  55                  60
   Thr Leu Val
   65
<210> 12
   <211> 68
   <212> PRT
   <213> Artificial
<220>
   <223> JS-TAG
<400> 12
   Val Thr Ala Pro Leu Ala Gly Thr Ile Trp Lys Val Leu Ala Ser Glu
   1               5                   10                  15
   Gly Gln Thr Val Ala Ala Gly Glu Val Leu Leu Ile Leu Glu Ala Met
               20                  25                  30
   Lys Met Glu Thr Glu Ile Arg Ala Ala Gln Ala Gly Thr Val Arg Gly
           35                  40                  45
   Ile Ala Val Lys Ala Gly Asp Ala Val Ala Val Gly Asp Thr Leu Met
       50                  55                  60
   Thr Leu Val Asp
   65
<210> 13
   <211> 67
   <212> PRT
   <213> Artificial
<220>
   <223> JS-TAG
<400> 13
   Met Val Thr Ala Pro Leu Ala Gly Thr Ile Trp Lys Val Leu Ala Ser
   1               5                   10                  15
   Glu Gly Gln Thr Val Ala Ala Gly Glu Val Leu Leu Ile Leu Glu Ala
               20                  25                  30
   Met Lys Met Glu Thr Glu Ile Arg Ala Ala Gln Ala Gly Thr Val Arg
           35                  40                  45
   Gly Ile Ala Val Lys Ala Gly Asp Ala Val Ala Val Gly Asp Thr Leu
       50                  55                  60
   Met Thr Leu
   65
<210> 14
   <211> 68
   <212> PRT
   <213> Artificial
<220>
   <223> JS-TAG
<400> 14
   Met Val Thr Ala Pro Leu Ala Gly Thr Ile Trp Lys Val Leu Ala Ser
   1               5                   10                  15
   Glu Gly Gln Thr Val Ala Ala Gly Glu Val Leu Leu Ile Leu Glu Ala
               20                  25                  30
   Met Lys Met Glu Thr Glu Ile Arg Ala Ala Gln Ala Gly Thr Val Arg
           35                  40                  45
   Gly Ile Ala Val Lys Ala Gly Asp Ala Val Ala Val Gly Asp Thr Leu
       50                  55                  60
   Met Thr Leu Val
   65
<210> 15
   <211> 69
   <212> PRT
   <213> Artificial
<220>
   <223> JS-TAG
<400> 15
   Met Val Thr Ala Pro Leu Ala Gly Thr Ile Trp Lys Val Leu Ala Ser
   1               5                   10                  15
   Glu Gly Gln Thr Val Ala Ala Gly Glu Val Leu Leu Ile Leu Glu Ala
               20                  25                  30
   Met Lys Met Glu Thr Glu Ile Arg Ala Ala Gln Ala Gly Thr Val Arg
           35                  40                  45
   Gly Ile Ala Val Lys Ala Gly Asp Ala Val Ala Val Gly Asp Thr Leu
       50                  55                  60
   Met Thr Leu Val Asp
   65
<210> 16
   <211> 70
   <212> PRT
   <213> Artificial
<220>
   <223> JS-TAG
<400> 16
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Asp Thr Leu Met Thr Leu
   65                  70
<210> 17
   <211> 70
   <212> PRT
   <213> Artificial
<220>
   <223> JS-TAG
<400> 17
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Asp Thr Leu Met Thr Leu
   65                  70
<210> 18
   <211> 70
   <212> PRT
   <213> Artificial
<220>
   <223> JS-TAG
<400> 18
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Asp Thr Leu Met Thr Leu
   65                  70
<210> 19
   <211> 102
   <212> PRT
   <213> Phage TP21
<400> 19
   Pro Pro Thr Pro Ile Pro Gln Pro Glu Pro Gln Leu Pro Ser Gly Gln
   1               5                   10                  15
   Tyr Asp Ser Ser Trp Phe Thr Lys Glu Ser Gly Thr Phe Thr Leu Asn
               20                  25                  30
   Thr Thr Ile Asn Leu Arg Thr Ala Pro Phe Ser Asn Ala Pro Leu Ile
           35                  40                  45
   Ala Thr Leu Ser Lys Gly Gln Gln Val Ser Tyr Asp Gly Tyr Gly Ile
       50                  55                  60
   Glu Leu Asp Gly His Val Trp Ile Arg Gln Pro Arg Ala Asn Gly Thr
   65                  70                  75                  80
   Tyr Gly Tyr Met Ala Thr Gly Glu Ser Ala Asn Gly Lys Arg Val Asp
                   85                  90                  95
   Tyr Trp Gly Ser Phe Lys
               100
<210> 20
   <211> 160
   <212> PRT
   <213> Phage Bastille
<400> 20
   Glu Lys Glu Gly Ala Lys Glu Thr Leu Val Pro Ala Leu Asn Lys Val
   1               5                   10                  15
   Val Thr Tyr Glu Val Gly Thr Asn Leu Ile Pro Glu Ile Gln Asp Lys
               20                  25                  30
   Leu Ala Phe Leu Gly Tyr Glu Ala Arg Ile Asn Phe Thr Gly Leu Gly
           35                  40                  45
   Asp Gly Leu Val Ser Ile Glu Thr Ser His Gln Val Gly Ala Glu Leu
       50                  55                  60
   Asp Lys Leu Thr Ala Trp Leu Asp Glu Arg Gly Trp Ala Tyr Tyr Tyr
   65                  70                  75                  80
   Thr Ser Ser Lys Glu Gly Tyr Asn Gly Lys Ser Lys Val Val Thr Tyr
                   85                  90                  95
   Asp Met Gly Thr Asn Lys Ile Pro Glu Leu Ser Asn Val Leu Ala Tyr
               100                 105                 110
   Gln Gly Met Gln Thr Ala Ile Val Phe Thr Gly Lys Gly Asp Gly Leu
           115                 120                 125
   Ile Arg Leu Glu Ser Thr Pro Leu Asp Glu Ser Arg Leu Gln Asn Phe
       130                 135                 140
   Lys Asn Ile Leu Glu Ala Gln Lys Ile Ala Tyr Tyr Met Tyr Ser Glu
   145                 150                 155                 160
<210> 21
   <211> 101
   <212> PRT
   <213> Staphylococcus aureus subsp. aureus USA300
<400> 21
   Met Pro Pro Val Pro Ala Gly Tyr Thr Leu Asp Lys Asn Asn Val Pro
   1               5                   10                  15
   Tyr Lys Lys Glu Gln Gly Asn Tyr Thr Val Ala Asn Val Lys Gly Asn
               20                  25                  30
   Asn Val Arg Asp Gly Tyr Ser Thr Asn Ser Arg Ile Thr Gly Val Leu
           35                  40                  45
   Pro Asn Asn Thr Thr Ile Thr Tyr Asp Gly Ala Tyr Cys Ile Asn Gly
       50                  55                  60
   Tyr Arg Trp Ile Thr Tyr Ile Ala Asn Ser Gly Gln Arg Arg Tyr Ile
   65                  70                  75                  80
   Ala Thr Gly Glu Val Asp Ile Ala Gly Asn Arg Ile Ser Ser Phe Gly
                   85                  90                  95
   Lys Phe Ser Ala Val
               100
<210> 22
   <211> 226
   <212> PRT
   <213> Phage A511
<400> 22
   Lys Ala Gly Ile Pro Ile Thr Leu Asp Ser Gly Ser Lys Thr Ser Asp
   1               5                   10                  15
   Lys Gly Ile Lys Ser His Lys Trp Val Ala Asp Lys Leu Gly Gly Thr
               20                  25                  30
   Thr His Gln Asp Pro Tyr Ala Tyr Leu Ser Ser Trp Gly Ile Ser Lys
           35                  40                  45
   Ala Gln Phe Ala Ser Asp Leu Ala Lys Val Ser Gly Gly Gly Asn Thr
       50                  55                  60
   Gly Thr Ala Pro Ala Lys Pro Ser Thr Pro Ala Pro Lys Pro Ser Thr
   65                  70                  75                  80
   Pro Ser Thr Asn Leu Asp Lys Leu Gly Leu Val Asp Tyr Met Asn Ala
                   85                  90                  95
   Lys Lys Met Asp Ser Ser Tyr Ser Asn Arg Asp Lys Leu Ala Lys Gln
               100                 105                 110
   Tyr Gly Ile Ala Asn Tyr Ser Gly Thr Ala Ser Gln Asn Thr Thr Leu
           115                 120                 125
   Leu Ser Lys Ile Lys Gly Gly Ala Pro Lys Pro Ser Thr Pro Ala Pro
       130                 135                 140
   Lys Pro Ser Thr Ser Thr Ala Lys Lys Ile Tyr Phe Pro Pro Asn Lys
   145                 150                 155                 160
   Gly Asn Trp Ser Val Tyr Pro Thr Asn Lys Ala Pro Val Lys Ala Asn
                   165                 170                 175
   Ala Ile Gly Ala Ile Asn Pro Thr Lys Phe Gly Gly Leu Thr Tyr Thr
               180                 185                 190
   Ile Gln Lys Asp Arg Gly Asn Gly Val Tyr Glu Ile Gln Thr Asp Gln
           195                 200                 205
   Phe Gly Arg Val Gln Val Tyr Gly Ala Pro Ser Thr Gly Ala Val Ile
       210                 215                 220
   Lys Lys
   225
<210> 23
   <211> 197
   <212> PRT
   <213> Phage A511
<400> 23
   Gly Gly Thr Thr His Gln Asp Pro Tyr Ala Tyr Leu Ser Ser Trp Gly
   1               5                   10                  15
   Ile Ser Lys Ala Gln Phe Ala Ser Asp Leu Ala Lys Val Ser Gly Gly
               20                  25                  30
   Gly Asn Thr Gly Thr Ala Pro Ala Lys Pro Ser Thr Pro Ala Pro Lys
           35                  40                  45
   Pro Ser Thr Pro Ser Thr Asn Leu Asp Lys Leu Gly Leu Val Asp Tyr
       50                  55                  60
   Met Asn Ala Lys Lys Met Asp Ser Ser Tyr Ser Asn Arg Asp Lys Leu
   65                  70                  75                  80
   Ala Lys Gln Tyr Gly Ile Ala Asn Tyr Ser Gly Thr Ala Ser Gln Asn
                   85                  90                  95
   Thr Thr Leu Leu Ser Lys Ile Lys Gly Gly Ala Pro Lys Pro Ser Thr
               100                 105                 110
   Pro Ala Pro Lys Pro Ser Thr Ser Thr Ala Lys Lys Ile Tyr Phe Pro
           115                 120                 125
   Pro Asn Lys Gly Asn Trp Ser Val Tyr Pro Thr Asn Lys Ala Pro Val
       130                 135                 140
   Lys Ala Asn Ala Ile Gly Ala Ile Asn Pro Thr Lys Phe Gly Gly Leu
   145                 150                 155                 160
   Thr Tyr Thr Ile Gln Lys Asp Arg Gly Asn Gly Val Tyr Glu Ile Gln
                   165                 170                 175
   Thr Asp Gln Phe Gly Arg Val Gln Val Tyr Gly Ala Pro Ser Thr Gly
               180                 185                 190
   Ala Val Ile Lys Lys
           195
<210> 24
   <211> 175
   <212> PRT
   <213> Phage A511
<400> 24
   Ala Ser Asp Leu Ala Lys Val Ser Gly Gly Gly Asn Thr Gly Thr Ala
   1               5                   10                  15
   Pro Ala Lys Pro Ser Thr Pro Ala Pro Lys Pro Ser Thr Pro Ser Thr
               20                  25                  30
   Asn Leu Asp Lys Leu Gly Leu Val Asp Tyr Met Asn Ala Lys Lys Met
           35                  40                  45
   Asp Ser Ser Tyr Ser Asn Arg Asp Lys Leu Ala Lys Gln Tyr Gly Ile
       50                  55                  60
   Ala Asn Tyr Ser Gly Thr Ala Ser Gln Asn Thr Thr Leu Leu Ser Lys
   65                  70                  75                  80
   Ile Lys Gly Gly Ala Pro Lys Pro Ser Thr Pro Ala Pro Lys Pro Ser
                   85                  90                  95
   Thr Ser Thr Ala Lys Lys Ile Tyr Phe Pro Pro Asn Lys Gly Asn Trp
               100                 105                 110
   Ser Val Tyr Pro Thr Asn Lys Ala Pro Val Lys Ala Asn Ala Ile Gly
           115                 120                 125
   Ala Ile Asn Pro Thr Lys Phe Gly Gly Leu Thr Tyr Thr Ile Gln Lys
       130                 135                 140
   Asp Arg Gly Asn Gly Val Tyr Glu Ile Gln Thr Asp Gln Phe Gly Arg
   145                 150                 155                 160
   Val Gln Val Tyr Gly Ala Pro Ser Thr Gly Ala Val Ile Lys Lys
                   165                 170                 175
<210> 25
   <211> 158
   <212> PRT
   <213> Phage phi3626
<400> 25
   Glu Tyr Glu Val Gln Glu Ser Lys Pro Val Pro Val Tyr Asp Arg Asn
   1               5                   10                  15
   Lys Phe Lys Thr Asn Ala Arg Ala Leu Val Asn Leu Asp Pro Arg Asp
               20                  25                  30
   Arg Ala Ser Gly Ile Tyr Glu Asp Leu Gly Glu Ile Tyr Lys Asp Glu
           35                  40                  45
   Arg Phe Tyr Val Leu Pro Glu Val Cys Asp Lys Gly Asp Tyr Leu Pro
       50                  55                  60
   Val Leu Tyr Trp Lys Asp Gly Ala Asn Arg Ala Ser Asn Lys Val Trp
   65                  70                  75                  80
   Val Ser Ser Lys Gln Lys Tyr Met Met Ile Asp Thr Tyr His Arg Val
                   85                  90                  95
   Val Asn Val Val Thr Glu Leu Asp Ala Arg Tyr Glu Pro Ser Pro Asn
               100                 105                 110
   Ser Asn Arg Met Gly Tyr Val Cys Asn Ala Glu Arg Val Tyr Val His
           115                 120                 125
   Lys Ile Glu Gly Asn Tyr Ala Leu Cys Thr Tyr Phe Ala Gly Glu Gly
       130                 135                 140
   Tyr Lys Thr Ala Trp Phe Thr Ala Lys Tyr Leu Glu Arg Ile
   145                 150                 155
<210> 26
   <211> 170
   <212> PRT
   <213> bacteriophage phi3626
<400> 26
   Gly Gly Gln Ala Pro Gly Thr Val Ile Glu Asn Val Glu Tyr Glu Val
   1               5                   10                  15
   Gln Glu Ser Lys Pro Val Pro Val Tyr Asp Arg Asn Lys Phe Lys Thr
               20                  25                  30
   Asn Ala Arg Ala Leu Val Asn Leu Asp Pro Arg Asp Arg Ala Ser Gly
           35                  40                  45
   Ile Tyr Glu Asp Leu Gly Glu Ile Tyr Lys Asp Glu Arg Phe Tyr Val
       50                  55                  60
   Leu Pro Glu Val Cys Asp Lys Gly Asp Tyr Leu Pro Val Leu Tyr Trp
   65                  70                  75                  80
   Lys Asp Gly Ala Asn Arg Ala Ser Asn Lys Val Trp Val Ser Ser Lys
                   85                  90                  95
   Gln Lys Tyr Met Met Ile Asp Thr Tyr His Arg Val Val Asn Val Val
               100                 105                 110
   Thr Glu Leu Asp Ala Arg Tyr Glu Pro Ser Pro Asn Ser Asn Arg Met
           115                 120                 125
   Gly Tyr Val Cys Asn Ala Glu Arg Val Tyr Val His Lys Ile Glu Gly
       130                 135                 140
   Asn Tyr Ala Leu Cys Thr Tyr Phe Ala Gly Glu Gly Tyr Lys Thr Ala
   145                 150                 155                 160
   Trp Phe Thr Ala Lys Tyr Leu Glu Arg Ile
                   165                 170
<210> 27
   <211> 96
   <212> PRT
   <213> Phage of Staphylococcus epidermis
<220>
   <221> misc_feature
   <222> (83)..(83)
   <223> Xaa can be any naturally occurring amino acid
<400> 27
   Met Lys Arg Lys Lys Pro Lys Gly Trp Ser Glu Asn Pro Tyr Gly Thr
   1               5                   10                  15
   Tyr Tyr Lys Lys Val Asp Lys Thr Phe Ile Val Gly Ser Glu Lys Ile
               20                  25                  30
   Glu Thr Arg Ile Gly Ser Pro Phe Leu Ser Ala Pro Ser Gly Gly His
           35                  40                  45
   Val Thr Pro Asn Gln Lys Met Thr Phe Asp Tyr Leu Ala Gln Gln Asp
       50                  55                  60
   Gly Tyr Glu Trp Gly Gln Leu Glu Asn Asn Arg Gly Gln Gln Glu Phe
   65                  70                  75                  80
   Val Pro Xaa Arg Pro Leu Ser Gln Lys Glu Tyr Trp Gly Ile Leu Lys
                   85                  90                  95
<210> 28
   <211> 105
   <212> PRT
   <213> Phage of Staphylococcus aureus
<400> 28
   Met Asn Thr Val Lys Pro Val Val Ser Ala Trp Lys Arg Asn Lys Tyr
   1               5                   10                  15
   Gly Thr Tyr Tyr Met Glu Glu Ser Ala Arg Phe Thr Asn Gly Asn Gln
               20                  25                  30
   Pro Ile Thr Val Arg Lys Ile Gly Pro Phe Leu Ser Cys Pro Val Ala
           35                  40                  45
   Tyr Gln Phe Gln Pro Gly Gly Tyr Cys Asp Tyr Thr Glu Val Met Leu
       50                  55                  60
   Gln Asp Gly His Val Trp Val Gly Tyr Thr Trp Glu Gly Gln Arg Tyr
   65                  70                  75                  80
   Tyr Leu Pro Ile Arg Thr Trp Asn Gly Ser Ala Pro Pro Asn Gln Ile
                   85                  90                  95
   Leu Gly Asp Leu Trp Gly Glu Ile Ser
               100                 105
<210> 29
   <211> 124
   <212> PRT
   <213> Staphylococcus simulans
<400> 29
   Met Ser Asn Ser Thr Ala Gln Asp Pro Met Pro Phe Leu Lys Ser Ala
   1 5 10 15
   Gly Tyr Gly Lys Ala Gly Gly Thr Val Thr Pro Thr Pro Asn Thr Gly
   20 25 30
   Trp Lys Thr Asn Lys Tyr Gly Thr Leu Tyr Lys Ser Glu Ser Ala Ser
   35 40 45
   Phe Thr Pro Asn Thr Asp Ile Ile Thr Arg Thr Thr Gly Pro Phe Arg
   50 55 60
   Ser Met Pro Gln Ser Gly Val Leu Lys Ala Gly Gln Thr Ile His Tyr
   65 70 75 80
   Asp Glu Val Met Lys Gln Asp Gly His Val Trp Val Gly Tyr Thr Gly
   85 90 95
   Asn Ser Gly Gln Arg Ile Tyr Leu Pro Val Arg Thr Trp Asn Lys Ser
   100 105 110
   Thr Asn Thr Leu Gly Val Leu Trp Gly Thr Ile Lys
   115 120
<210> 30
   <211> 125
   <212> PRT
   <213> Phage of Stapholcoccus capitis
<400> 30
   Met Asn Ser Phe Ser Asn Asn Thr Ala Gln Asp Pro Met Pro Phe Leu
   1 5 10 15
   Lys Ser Ala Gly Tyr Gly Ser Asn Ser Thr Ser Ser Ser Asn Asn Asn
   20 25 30
   Gly Tyr Lys Thr Asn Lys Tyr Gly Thr Leu Tyr Lys Ser Glu Ser Ala
   35 40 45
   Ser Phe Thr Ala Asn Thr Asp Ile Ile Thr Arg Leu Thr Gly Pro Phe
   50 55 60
   Arg Ser Met Pro Gln Ser Gly Val Leu Arg Lys Gly Leu Thr Ile Lys
   65 70 75 80
   Tyr Asp Glu Val Met Lys Gln Asp Gly His Val Trp Val Gly Tyr Asn
   85 90 95
   Thr Asn Ser Gly Lys Arg Val Tyr Leu Pro Val Arg Thr Trp Asn Glu
   100 105 110
   Ser Thr Gly Glu Leu Gly Pro Leu Trp Gly Thr Ile Lys
   115 120 125
<210> 31
   <211> 101
   <212> PRT
   <213> Prophage of Staphylococcus aureus
<400> 31
   Met Pro Pro Val Pro Ala Gly Tyr Thr Leu Asp Lys Asn Asn Val Pro
   1               5                   10                  15
   Tyr Lys Lys Glu Ala Gly Asn Tyr Thr Val Ala Asn Val Lys Gly Asn
               20                  25                  30
   Asn Val Arg Asp Gly Tyr Ser Thr Asn Ser Arg Ile Thr Gly Val Leu
           35                  40                  45
   Pro Asn Asn Ala Thr Ile Lys Tyr Asp Gly Ala Tyr Cys Ile Asn Gly
       50                  55                  60
   Tyr Arg Trp Ile Thr Tyr Ile Ala Asn Ser Gly Gln Arg Arg Tyr Ile
   65                  70                  75                  80
   Ala Thr Gly Glu Val Asp Lys Ala Gly Asn Arg Ile Ser Ser Phe Gly
                   85                  90                  95
   Lys Phe Ser Ala Val
               100
<210> 32
   <211> 190
   <212> PRT
   <213> Prophage of Enterococcus faecalis
<400> 32
   Thr Asn Lys Pro Glu Thr Asp Thr Pro Ala Thr Asp Ala Gly Glu Glu
   1               5                   10                  15
   Ile Glu Lys Ile Pro Asn Ser Asp Val Lys Val Gly Asp Thr Val Lys
               20                  25                  30
   Val Lys Phe Asn Val Asp Ala Trp Ala Thr Gly Glu Ala Ile Pro Gln
           35                  40                  45
   Trp Val Lys Gly Asn Ser Tyr Lys Val Gln Glu Val Thr Gly Ser Arg
       50                  55                  60
   Val Leu Leu Glu Gly Ile Leu Ser Trp Ile Ser Lys Gly Asp Ile Glu
   65                  70                  75                  80
   Leu Leu Pro Asp Ala Thr Val Val Pro Asp Lys Gln Pro Glu Ala Thr
                   85                  90                  95
   His Val Val Gln Tyr Gly Glu Thr Leu Ser Ser Ile Ala Tyr Gln Tyr
               100                 105                 110
   Gly Thr Asp Tyr Gln Thr Leu Ala Ala Leu Asn Gly Leu Ala Asn Pro
           115                 120                 125
   Asn Leu Ile Tyr Pro Gly Gln Val Leu Lys Val Asn Gly Ser Ala Thr
       130                 135                 140
   Ser Asn Val Tyr Thr Val Lys Tyr Gly Asp Asn Leu Ser Ser Ile Ala
   145                 150                 155                 160
   Ala Lys Leu Gly Thr Thr Tyr Gln Ala Leu Ala Ala Leu Asn Gly Leu
                   165                 170                 175
   Ala Asn Pro Asn Leu Ile Tyr Pro Gly Gln Thr Leu Asn Tyr
               180                 185                 190
<210> 33
   <211> 154
   <212> PRT
   <213> Prophage of Enterococcus faecalis
<400> 33
   Ala Pro Lys Tyr Gln Val Gly Gln Ala Ile Arg Phe Thr Ser Ile Tyr
   1 5 10 15
   Pro Thr Pro Asp Ala Leu Ile Asn Glu His Leu Ser Ala Glu Ala Leu
   20 25 30
   Trp Thr Gln Val Gly Thr Ile Thr Ala Lys Leu Pro Asp Arg Gln Asn
   35 40 45
   Leu Tyr Arg Val Glu Asn Ser Gly His Leu Leu Gly Tyr Val Asn Asp
   50 55 60
   Gly Asp Ile Ala Glu Leu Trp Arg Pro Gln Thr Lys Lys Ser Phe Leu
   65 70 75 80
   Ile Gly Val Asp Glu Gly Ile Val Leu Arg Ala Gly Gln Pro Ser Leu
   85 90 95
   Leu Ala Pro Ile Tyr Gly Ile Trp Pro Lys Asn Thr Arg Phe Tyr Tyr
   100 105 110
   Asp Thr Phe Tyr Ile Ala Asp Gly Tyr Val Phe Ile Gly Gly Thr Asp
   115 120 125
   Thr Thr Gly Ala Arg Ile Tyr Leu Pro Ile Gly Pro Asn Asp Gly Asn
   130 135 140
   Ala Gln Asn Thr Trp Gly Ser Phe Ala Ser
   145 150
<210> 34
   <211> 9
   <212> PRT
   <213> Artificial
<220>
   <223> Polypeptidelinker
<400> 34
   Ala Gly Ala Gly Ala Gly Ala Gly Ser
   1 5
<210> 35
   <211> 11
   <212> PRT
   <213> Artificial
<220>
   <223> Polypeptidelinker
<400> 35
   Ala Gly Ala Gly Ala Gly Ala Gly Ser Glu Leu
   1 5 10
<210> 36
   <211> 14
   <212> PRT
   <213> Artificial
<220>
   <223> Polypeptidelinker
<400> 36
   Thr Pro Thr Pro Pro Asn Pro Gly Pro Lys Asn Phe Thr Thr
   1 5 10
<210> 37
   <211> 6
   <212> PRT
   <213> Artificial
<220>
   <223> Polypeptidelinker
<400> 37
   Ala Ala Lys Asn Pro Asn
   1 5
<210> 38
   <211> 9
   <212> PRT
   <213> Artificial
<220>
   <223> Polypeptidelinker
<400> 38
   Ala Gly Ala Gly Ala Gly Ala Glu Leu
   1 5
<210> 39
   <211> 251
   <212> PRT
   <213> Artificial
<220>
   <223> Polypeptide comprising Js(4a) and CBD fragment of Ply3626
<400> 39
   Met Val Gly Ala Val Thr Ala Pro Leu Ala Gly Thr Ile Trp Lys Val
   1 5 10 15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Glu
   65                  70                  75                  80
   Leu Gly Gly Gln Ala Pro Gly Thr Val Ile Glu Asn Val Glu Tyr Glu
                   85                  90                  95
   Val Gln Glu Ser Lys Pro Val Pro Val Tyr Asp Arg Asn Lys Phe Lys
               100                 105                 110
   Thr Asn Ala Arg Ala Leu Val Asn Leu Asp Pro Arg Asp Arg Ala Ser
           115                 120                 125
   Gly Ile Tyr Glu Asp Leu Gly Glu Ile Tyr Lys Asp Glu Arg Phe Tyr
       130                 135                 140
   Val Leu Pro Glu Val Cys Asp Lys Gly Asp Tyr Leu Pro Val Leu Tyr
   145                 150                 155                 160
   Trp Lys Asp Gly Ala Asn Arg Ala Ser Asn Lys Val Trp Val Ser Ser
                   165                 170                 175
   Lys Gln Lys Tyr Met Met Ile Asp Thr Tyr His Arg Val Val Asn Val
               180                 185                 190
   Val Thr Glu Leu Asp Ala Arg Tyr Glu Pro Ser Pro Asn Ser Asn Arg
           195                 200                 205
   Met Gly Tyr Val Cys Asn Ala Glu Arg Val Tyr Val His Lys Ile Glu
       210                 215                 220
   Gly Asn Tyr Ala Leu Cys Thr Tyr Phe Ala Gly Glu Gly Tyr Lys Thr
   225                 230                 235                 240
   Ala Trp Phe Thr Ala Lys Tyr Leu Glu Arg Ile
                   245                 250
<210> 40
   <211> 477
   <212> PRT
   <213> Artificial
<220>
   <223> Polypeptide comprising Js(4a), GFP and CBD fragment of Ply3626
<400> 40
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Thr
   65                  70                  75                  80
   Ser Lys Gly Glu Glu Leu Phe Thr Gly Val Val Pro Ile Leu Val Glu
                   85                  90                  95
   Leu Asp Gly Asp Val Asn Gly His Lys Phe Ser Val Ser Gly Glu Gly
               100                 105                 110
   Glu Gly Asp Ala Thr Tyr Gly Lys Leu Thr Leu Lys Phe Ile Cys Thr
           115                 120                 125
   Thr Gly Lys Leu Pro Val Pro Trp Pro Thr Leu Val Thr Thr Phe Ala
       130                 135                 140
   Tyr Gly Leu Gln Cys Phe Ala Arg Tyr Pro Asp His Met Lys Gln His
   145                 150                 155                 160
   Asp Phe Phe Lys Ser Ala Met Pro Glu Gly Tyr Val Gln Glu Arg Thr
                   165                 170                 175
   Ile Ser Phe Lys Asp Asp Gly Asn Tyr Lys Thr Arg Ala Glu Val Lys
               180                 185                 190
   Phe Glu Gly Asp Thr Leu Val Asn Arg Ile Glu Leu Lys Gly Ile Asp
           195                 200                 205
   Phe Lys Glu Asp Gly Asn Ile Leu Gly His Lys Leu Glu Tyr Asn Tyr
       210                 215                 220
   Asn Ser His Asn Val Tyr Ile Thr Ala Asp Lys Gln Lys Asn Gly Ile
   225                 230                 235                 240
   Lys Ala Asn Phe Lys Ile Arg His Asn Ile Glu Asp Gly Ser Val Gln
                   245                 250                 255
   Leu Ala Asp His Tyr Gln Gln Asn Thr Pro Ile Gly Asp Gly Pro Val
               260                 265                 270
   Leu Leu Pro Asp Asn His Tyr Leu Ser Thr Gln Ser Ala Leu Ser Lys
           275                 280                 285
   Asp Pro Asn Glu Lys Arg Asp His Met Val Leu Leu Glu Phe Val Thr
       290                 295                 300
   Ala Ala Gly Ile Thr His Gly Met Asp Glu Leu Tyr Lys Glu Leu Glu
   305                 310                 315                 320
   Tyr Glu Val Gln Glu Ser Lys Pro Val Pro Val Tyr Asp Arg Asn Lys
                   325                 330                 335
   Phe Lys Thr Asn Ala Arg Ala Leu Val Asn Leu Asp Pro Arg Asp Arg
               340                 345                 350
   Ala Ser Gly Ile Tyr Glu Asp Leu Gly Glu Ile Tyr Lys Asp Glu Arg
           355                 360                 365
   Phe Tyr Val Leu Pro Glu Val Cys Asp Lys Gly Asp Tyr Leu Pro Val
       370                 375                 380
   Leu Tyr Trp Lys Asp Gly Ala Asn Arg Ala Ser Asn Lys Val Trp Val
   385                 390                 395                 400
   Ser Ser Lys Gln Lys Tyr Met Met Ile Asp Thr Tyr His Arg Val Val
                   405                 410                 415
   Asn Val Val Thr Glu Leu Asp Ala Arg Tyr Glu Pro Ser Pro Asn Ser
               420                 425                 430
   Asn Arg Met Gly Tyr Val Cys Asn Ala Glu Arg Val Tyr Val His Lys
           435                 440                 445
   Ile Glu Gly Asn Tyr Ala Leu Cys Thr Tyr Phe Ala Gly Glu Gly Tyr
       450                 455                 460
   Lys Thr Ala Trp Phe Thr Ala Lys Tyr Leu Glu Arg Ile
   465                 470                 475
<210> 41
   <211> 239
   <212> PRT
   <213> Artificial
<220>
   <223> Polypeptide comprising Js(5b) and CBD fragment of Ply3626
<400> 41
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Glu
   65                  70                  75                  80
   Leu Glu Tyr Glu Val Gln Glu Ser Lys Pro Val Pro Val Tyr Asp Arg
                   85                  90                  95
   Asn Lys Phe Lys Thr Asn Ala Arg Ala Leu Val Asn Leu Asp Pro Arg
               100                 105                 110
   Asp Arg Ala Ser Gly Ile Tyr Glu Asp Leu Gly Glu Ile Tyr Lys Asp
           115                 120                 125
   Glu Arg Phe Tyr Val Leu Pro Glu Val Cys Asp Lys Gly Asp Tyr Leu
       130                 135                 140
   Pro Val Leu Tyr Trp Lys Asp Gly Ala Asn Arg Ala Ser Asn Lys Val
   145                 150                 155                 160
   Trp Val Ser Ser Lys Gln Lys Tyr Met Met Ile Asp Thr Tyr His Arg
                   165                 170                 175
   Val Val Asn Val Val Thr Glu Leu Asp Ala Arg Tyr Glu Pro Ser Pro
               180                 185                 190
   Asn Ser Asn Arg Met Gly Tyr Val Cys Asn Ala Glu Arg Val Tyr Val
           195                 200                 205
   His Lys Ile Glu Gly Asn Tyr Ala Leu Cys Thr Tyr Phe Ala Gly Glu
       210                 215                 220
   Gly Tyr Lys Thr Ala Trp Phe Thr Ala Lys Tyr Leu Glu Arg Ile
   225                 230                 235
<210> 42
   <211> 183
   <212> PRT
   <213> Artificial
<220>
   <223> Polypeptide comprising JS5b and CBD21
<400> 42
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Glu
   65                  70                  75                  80
   Leu Pro Pro Thr Pro Ile Pro Gln Pro Glu Pro Gln Leu Pro Ser Gly
                   85                  90                  95
   Gln Tyr Asp Ser Ser Trp Phe Thr Lys Glu Ser Gly Thr Phe Thr Leu
               100                 105                 110
   Asn Thr Thr Ile Asn Leu Arg Thr Ala Pro Phe Ser Asn Ala Pro Leu
           115                 120                 125
   Ile Ala Thr Leu Ser Lys Gly Gln Gln Val Ser Tyr Asp Gly Tyr Gly
       130                 135                 140
   Ile Glu Leu Asp Gly His Val Trp Ile Arg Gln Pro Arg Ala Asn Gly
   145                 150                 155                 160
   Thr Tyr Gly Tyr Met Ala Thr Gly Glu Ser Ala Asn Gly Lys Arg Val
                   165                 170                 175
   Asp Tyr Trp Gly Ser Phe Lys
               180
<210> 43
   <211> 421
   <212> PRT
   <213> Artificial
<220>
   <223> Polypeptide comprising JS10a, GFP and CBD21
<400> 43
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Thr
   65                  70                  75                  80
   Ser Lys Gly Glu Glu Leu Phe Thr Gly Val Val Pro Ile Leu Val Glu
                   85                  90                  95
   Leu Asp Gly Asp Val Asn Gly His Lys Phe Ser Val Ser Gly Glu Gly
               100                 105                 110
   Glu Gly Asp Ala Thr Tyr Gly Lys Leu Thr Leu Lys Phe Ile Cys Thr
           115                 120                 125
   Thr Gly Lys Leu Pro Val Pro Trp Pro Thr Leu Val Thr Thr Phe Ala
       130                 135                 140
   Tyr Gly Leu Gln Cys Phe Ala Arg Tyr Pro Asp His Met Lys Gln His
   145                 150                 155                 160
   Asp Phe Phe Lys Ser Ala Met Pro Glu Gly Tyr Val Gln Glu Arg Thr
                   165                 170                 175
   Ile Ser Phe Lys Asp Asp Gly Asn Tyr Lys Thr Arg Ala Glu Val Lys
               180                 185                 190
   Phe Glu Gly Asp Thr Leu Val Asn Arg Ile Glu Leu Lys Gly Ile Asp
           195                 200                 205
   Phe Lys Glu Asp Gly Asn Ile Leu Gly His Lys Leu Glu Tyr Asn Tyr
       210                 215                 220
   Asn Ser His Asn Val Tyr Ile Thr Ala Asp Lys Gln Lys Asn Gly Ile
   225                 230                 235                 240
   Lys Ala Asn Phe Lys Ile Arg His Asn Ile Glu Asp Gly Ser Val Gln
                   245                 250                 255
   Leu Ala Asp His Tyr Gln Gln Asn Thr Pro Ile Gly Asp Gly Pro Val
               260                 265                 270
   Leu Leu Pro Asp Asn His Tyr Leu Ser Thr Gln Ser Ala Leu Ser Lys
           275                 280                 285
   Asp Pro Asn Glu Lys Arg Asp His Met Val Leu Leu Glu Phe Val Thr
       290                 295                 300
   Ala Ala Gly Ile Thr His Gly Met Asp Glu Leu Tyr Lys Glu Leu Pro
   305                 310                 315                 320
   Pro Thr Pro Ile Pro Gln Pro Glu Pro Gln Leu Pro Ser Gly Gln Tyr
                   325                 330                 335
   Asp Ser Ser Trp Phe Thr Lys Glu Ser Gly Thr Phe Thr Leu Asn Thr
               340                 345                 350
   Thr Ile Asn Leu Arg Thr Ala Pro Phe Ser Asn Ala Pro Leu Ile Ala
           355                 360                 365
   Thr Leu Ser Lys Gly Gln Gln Val Ser Tyr Asp Gly Tyr Gly Ile Glu
       370                 375                 380
   Leu Asp Gly His Val Trp Ile Arg Gln Pro Arg Ala Asn Gly Thr Tyr
   385                 390                 395                 400
   Gly Tyr Met Ala Thr Gly Glu Ser Ala Asn Gly Lys Arg Val Asp Tyr
                   405                 410                 415
   Trp Gly Ser Phe Lys
               420
<210> 44
   <211> 241
   <212> PRT
   <213> Artificial
<220>
   <223> Polypeptide comprising JS5b and CBDBA
<400> 44
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Glu
   65                  70                  75                  80
   Leu Glu Lys Glu Gly Ala Lys Glu Thr Leu Val Pro Ala Leu Asn Lys
                   85                  90                  95
   Val Val Thr Tyr Glu Val Gly Thr Asn Leu Ile Pro Glu Ile Gln Asp
               100                 105                 110
   Lys Leu Ala Phe Leu Gly Tyr Glu Ala Arg Ile Asn Phe Thr Gly Leu
           115                 120                 125
   Gly Asp Gly Leu Val Ser Ile Glu Thr Ser His Gln Val Gly Ala Glu
       130                 135                 140
   Leu Asp Lys Leu Thr Ala Trp Leu Asp Glu Arg Gly Trp Ala Tyr Tyr
   145                 150                 155                 160
   Tyr Thr Ser Ser Lys Glu Gly Tyr Asn Gly Lys Ser Lys Val Val Thr
                   165                 170                 175
   Tyr Asp Met Gly Thr Asn Lys Ile Pro Glu Leu Ser Asn Val Leu Ala
               180                 185                 190
   Tyr Gln Gly Met Gln Thr Ala Ile Val Phe Thr Gly Lys Gly Asp Gly
           195                 200                 205
   Leu Ile Arg Leu Glu Ser Thr Pro Leu Asp Glu Ser Arg Leu Gln Asn
       210                 215                 220
   Phe Lys Asn Ile Leu Glu Ala Gln Lys Ile Ala Tyr Tyr Met Tyr Ser
   225                 230                 235                 240
   Glu
<210> 45
   <211> 479
   <212> PRT
   <213> Artificial
<220>
   <223> Polypeptide comprising JS10a, GFP and CBDBA
<400> 45
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Thr
   65                  70                  75                  80
   Ser Lys Gly Glu Glu Leu Phe Thr Gly Val Val Pro Ile Leu Val Glu
                   85                  90                  95
   Leu Asp Gly Asp Val Asn Gly His Lys Phe Ser Val Ser Gly Glu Gly
               100                 105                 110
   Glu Gly Asp Ala Thr Tyr Gly Lys Leu Thr Leu Lys Phe Ile Cys Thr
           115                 120                 125
   Thr Gly Lys Leu Pro Val Pro Trp Pro Thr Leu Val Thr Thr Phe Ala
       130                 135                 140
   Tyr Gly Leu Gln Cys Phe Ala Arg Tyr Pro Asp His Met Lys Gln His
   145                 150                 155                 160
   Asp Phe Phe Lys Ser Ala Met Pro Glu Gly Tyr Val Gln Glu Arg Thr
                   165                 170                 175
   Ile Ser Phe Lys Asp Asp Gly Asn Tyr Lys Thr Arg Ala Glu Val Lys
               180                 185                 190
   Phe Glu Gly Asp Thr Leu Val Asn Arg Ile Glu Leu Lys Gly Ile Asp
           195                 200                 205
   Phe Lys Glu Asp Gly Asn Ile Leu Gly His Lys Leu Glu Tyr Asn Tyr
       210                 215                 220
   Asn Ser His Asn Val Tyr Ile Thr Ala Asp Lys Gln Lys Asn Gly Ile
   225                 230                 235                 240
   Lys Ala Asn Phe Lys Ile Arg His Asn Ile Glu Asp Gly Ser Val Gln
                   245                 250                 255
   Leu Ala Asp His Tyr Gln Gln Asn Thr Pro Ile Gly Asp Gly Pro Val
               260                 265                 270
   Leu Leu Pro Asp Asn His Tyr Leu Ser Thr Gln Ser Ala Leu Ser Lys
           275                 280                 285
   Asp Pro Asn Glu Lys Arg Asp His Met Val Leu Leu Glu Phe Val Thr
       290                 295                 300
   Ala Ala Gly Ile Thr His Gly Met Asp Glu Leu Tyr Lys Glu Leu Glu
   305                 310                 315                 320
   Lys Glu Gly Ala Lys Glu Thr Leu Val Pro Ala Leu Asn Lys Val Val
                   325                 330                 335
   Thr Tyr Glu Val Gly Thr Asn Leu Ile Pro Glu Ile Gln Asp Lys Leu
               340                 345                 350
   Ala Phe Leu Gly Tyr Glu Ala Arg Ile Asn Phe Thr Gly Leu Gly Asp
           355                 360                 365
   Gly Leu Val Ser Ile Glu Thr Ser His Gln Val Gly Ala Glu Leu Asp
       370                 375                 380
   Lys Leu Thr Ala Trp Leu Asp Glu Arg Gly Trp Ala Tyr Tyr Tyr Thr
   385                 390                 395                 400
   Ser Ser Lys Glu Gly Tyr Asn Gly Lys Ser Lys Val Val Thr Tyr Asp
                   405                 410                 415
   Met Gly Thr Asn Lys Ile Pro Glu Leu Ser Asn Val Leu Ala Tyr Gln
               420                 425                 430
   Gly Met Gln Thr Ala Ile Val Phe Thr Gly Lys Gly Asp Gly Leu Ile
           435                 440                 445
   Arg Leu Glu Ser Thr Pro Leu Asp Glu Ser Arg Leu Gln Asn Phe Lys
       450                 455                 460
   Asn Ile Leu Glu Ala Gln Lys Ile Ala Tyr Tyr Met Tyr Ser Glu
   465                 470                 475
<210> 46
   <211> 182
   <212> PRT
   <213> Artificial
<220>
   <223> Polypeptide comprising JS5b and CBDUSA
<400> 46
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Glu
   65                  70                  75                  80
   Leu Met Pro Pro Val Pro Ala Gly Tyr Thr Leu Asp Lys Asn Asn Val
                   85                  90                  95
   Pro Tyr Lys Lys Glu Gln Gly Asn Tyr Thr Val Ala Asn Val Lys Gly
               100                 105                 110
   Asn Asn Val Arg Asp Gly Tyr Ser Thr Asn Ser Arg Ile Thr Gly Val
           115                 120                 125
   Leu Pro Asn Asn Thr Thr Ile Thr Tyr Asp Gly Ala Tyr Cys Ile Asn
       130                 135                 140
   Gly Tyr Arg Trp Ile Thr Tyr Ile Ala Asn Ser Gly Gln Arg Arg Tyr
   145                 150                 155                 160
   Ile Ala Thr Gly Glu Val Asp Ile Ala Gly Asn Arg Ile Ser Ser Phe
                   165                 170                 175
   Gly Lys Phe Ser Ala Val
               180
<210> 47
   <211> 177
   <212> PRT
   <213> Artificial
<220>
   <223> Polypeptide comprising JS5b and CBDpitti20
<220>
   <221> misc_feature
   <222> (164)..(164)
   <223> Xaa can be any naturally occurring amino acid
<400> 47
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Glu
   65                  70                  75                  80
   Leu Met Lys Arg Lys Lys Pro Lys Gly Trp Ser Glu Asn Pro Tyr Gly
                   85                  90                  95
   Thr Tyr Tyr Lys Lys Val Asp Lys Thr Phe Ile Val Gly Ser Glu Lys
               100                 105                 110
   Ile Glu Thr Arg Ile Gly Ser Pro Phe Leu Ser Ala Pro Ser Gly Gly
           115                 120                 125
   His Val Thr Pro Asn Gln Lys Met Thr Phe Asp Tyr Leu Ala Gln Gln
       130                 135                 140
   Asp Gly Tyr Glu Trp Gly Gln Leu Glu Asn Asn Arg Gly Gln Gln Glu
   145                 150                 155                 160
   Phe Val Pro Xaa Arg Pro Leu Ser Gln Lys Glu Tyr Trp Gly Ile Leu
                   165                 170                 175
   Lys
<210> 48
   <211> 186
   <212> PRT
   <213> Artificial
<220>
   <223> Polypeptide comprising JS5b and CBDPitti26
<400> 48
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Glu
   65                  70                  75                  80
   Leu Met Asn Thr Val Lys Pro Val Val Ser Ala Trp Lys Arg Asn Lys
                   85                  90                  95
   Tyr Gly Thr Tyr Tyr Met Glu Glu Ser Ala Arg Phe Thr Asn Gly Asn
               100                 105                 110
   Gln Pro Ile Thr Val Arg Lys Ile Gly Pro Phe Leu Ser Cys Pro Val
           115                 120                 125
   Ala Tyr Gln Phe Gln Pro Gly Gly Tyr Cys Asp Tyr Thr Glu Val Met
       130                 135                 140
   Leu Gln Asp Gly His Val Trp Val Gly Tyr Thr Trp Glu Gly Gln Arg
   145                 150                 155                 160
   Tyr Tyr Leu Pro Ile Arg Thr Trp Asn Gly Ser Ala Pro Pro Asn Gln
                   165                 170                 175
   Ile Leu Gly Asp Leu Trp Gly Glu Ile Ser
               180                 185
<210> 49
   <211> 203
   <212> PRT
   <213> Artificial
<220>
   <223> Polypeptide comprising JS5b and CBDLS
<400> 49
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Glu Leu Met
   65                  70                  75                  80
   Ser Asn Ser Thr Ala Gln Asp Pro Met Pro Phe Leu Lys Ser Ala Gly
                   85                  90                  95
   Tyr Gly Lys Ala Gly Gly Thr Val Thr Pro Thr Pro Asn Thr Gly Trp
               100                 105                 110
   Lys Thr Asn Lys Tyr Gly Thr Leu Tyr Lys Ser Glu Ser Ala Ser Phe
           115                 120                 125
   Thr Pro Asn Thr Asp Ile Ile Thr Arg Thr Thr Gly Pro Phe Arg Ser
       130                 135                 140
   Met Pro Gln Ser Gly Val Leu Lys Ala Gly Gln Thr Ile His Tyr Asp
   145                 150                 155                 160
   Glu Val Met Lys Gln Asp Gly His Val Trp Val Gly Tyr Thr Gly Asn
                   165                 170                 175
   Ser Gly Gln Arg Ile Tyr Leu Pro Val Arg Thr Trp Asn Lys Ser Thr
               180                 185                 190
   Asn Thr Leu Gly Val Leu Trp Gly Thr Ile Lys
           195                 200
<210> 50
   <211> 204
   <212> PRT
   <213> Artificial
<220>
   <223> Polypeptide comprising JS5b and CBDALE-1
<400> 50
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Glu Leu Met
   65                  70                  75                  80
   Asn Ser Phe Ser Asn Asn Thr Ala Gln Asp Pro Met Pro Phe Leu Lys
                   85                  90                  95
   Ser Ala Gly Tyr Gly Ser Asn Ser Thr Ser Ser Ser Asn Asn Asn Gly
               100                 105                 110
   Tyr Lys Thr Asn Lys Tyr Gly Thr Leu Tyr Lys Ser Glu Ser Ala Ser
           115                 120                 125
   Phe Thr Ala Asn Thr Asp Ile Ile Thr Arg Leu Thr Gly Pro Phe Arg
       130                 135                 140
   Ser Met Pro Gln Ser Gly Val Leu Arg Lys Gly Leu Thr Ile Lys Tyr
   145                 150                 155                 160
   Asp Glu Val Met Lys Gln Asp Gly His Val Trp Val Gly Tyr Asn Thr
                   165                 170                 175
   Asn Ser Gly Lys Arg Val Tyr Leu Pro Val Arg Thr Trp Asn Glu Ser
               180                 185                 190
   Thr Gly Glu Leu Gly Pro Leu Trp Gly Thr Ile Lys
           195                 200
<210> 51
   <211> 182
   <212> PRT
   <213> Artificial
<220>
   <223> Polypeptide comprising JS5b and CBDOpf
<400> 51
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Glu
   65                  70                  75                  80
   Leu Met Pro Pro Val Pro Ala Gly Tyr Thr Leu Asp Lys Asn Asn Val
                   85                  90                  95
   Pro Tyr Lys Lys Glu Ala Gly Asn Tyr Thr Val Ala Asn Val Lys Gly
               100                 105                 110
   Asn Asn Val Arg Asp Gly Tyr Ser Thr Asn Ser Arg Ile Thr Gly Val
               115                     120                     125
   Leu Pro Asn Asn Ala Thr Ile Lys Tyr Asp Gly Ala Tyr Cys Ile Asn
       130                     135                     140
   Gly Tyr Arg Trp Ile Thr Tyr Ile Ala Asn Ser Gly Gln Arg Arg Tyr
   145                     150                     155                 160
   Ile Ala Thr Gly Glu Val Asp Lys Ala Gly Asn Arg Ile Ser Ser Phe
                   165                     170                     175
   Gly Lys Phe Ser Ala Val
               180
<210> 52
   <211> 271
   <212> PRT
   <213> Artificial
<220>
   <223> Polypeptide comprising JS5b and CBDEF0355
<400> 52
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Glu
   65                  70                  75                  80
   Leu Thr Asn Lys Pro Glu Thr Asp Thr Pro Ala Thr Asp Ala Gly Glu
                   85                  90                  95
   Glu Ile Glu Lys Ile Pro Asn Ser Asp Val Lys Val Gly Asp Thr Val
               100                 105                 110
   Lys Val Lys Phe Asn Val Asp Ala Trp Ala Thr Gly Glu Ala Ile Pro
           115                 120                 125
   Gln Trp Val Lys Gly Asn Ser Tyr Lys Val Gln Glu Val Thr Gly Ser
       130                 135                 140
   Arg Val Leu Leu Glu Gly Ile Leu Ser Trp Ile Ser Lys Gly Asp Ile
   145                 150                 155                 160
   Glu Leu Leu Pro Asp Ala Thr Val Val Pro Asp Lys Gln Pro Glu Ala
                   165                 170                 175
   Thr His Val Val Gln Tyr Gly Glu Thr Leu Ser Ser Ile Ala Tyr Gln
               180                 185                 190
   Tyr Gly Thr Asp Tyr Gln Thr Leu Ala Ala Leu Asn Gly Leu Ala Asn
           195                 200                 205
   Pro Asn Leu Ile Tyr Pro Gly Gln Val Leu Lys Val Asn Gly Ser Ala
       210                 215                 220
   Thr Ser Asn Val Tyr Thr Val Lys Tyr Gly Asp Asn Leu Ser Ser Ile
   225                 230                 235                 240
   Ala Ala Lys Leu Gly Thr Thr Tyr Gln Ala Leu Ala Ala Leu Asn Gly
                   245                 250                 255
   Leu Ala Asn Pro Asn Leu Ile Tyr Pro Gly Gln Thr Leu Asn Tyr
               260                 265                 270
<210> 53
   <211> 235
   <212> PRT
   <213> Artificial
<220>
   <223> Polypeptide comprising JS5b and CBDEF1293
<400> 53
   Met Val Gly Ala Val Thr Ala Pro Leu Ala Gly Thr Ile Trp Lys Val
   1               5                   10                  15
   Leu Ala Ser Glu Gly Gln Thr Val Ala Ala Gly Glu Val Leu Leu Ile
               20                  25                  30
   Leu Glu Ala Met Lys Met Glu Thr Glu Ile Arg Ala Ala Gln Ala Gly
           35                  40                  45
   Thr Val Arg Gly Ile Ala Val Lys Ala Gly Asp Ala Val Ala Val Gly
       50                  55                  60
   Glu Thr Leu Met Thr Leu Ala Gly Ala Gly Ala Gly Ala Gly Ser Glu
   65                  70                  75                  80
   Leu Ala Pro Lys Tyr Gln Val Gly Gln Ala Ile Arg Phe Thr Ser Ile
                   85                  90                  95
   Tyr Pro Thr Pro Asp Ala Leu Ile Asn Glu His Leu Ser Ala Glu Ala
               100                 105                 110
   Leu Trp Thr Gln Val Gly Thr Ile Thr Ala Lys Leu Pro Asp Arg Gln
           115                 120                 125
   Asn Leu Tyr Arg Val Glu Asn Ser Gly His Leu Leu Gly Tyr Val Asn
       130                 135                 140
   Asp Gly Asp Ile Ala Glu Leu Trp Arg Pro Gln Thr Lys Lys Ser Phe
   145                 150                 155                 160
   Leu Ile Gly Val Asp Glu Gly Ile Val Leu Arg Ala Gly Gln Pro Ser
                   165                 170                 175
   Leu Leu Ala Pro Ile Tyr Gly Ile Trp Pro Lys Asn Thr Arg Phe Tyr
               180                 185                 190
   Tyr Asp Thr Phe Tyr Ile Ala Asp Gly Tyr Val Phe Ile Gly Gly Thr
           195                 200                 205
   Asp Thr Thr Gly Ala Arg Ile Tyr Leu Pro Ile Gly Pro Asn Asp Gly
       210                 215                 220
   Asn Ala Gln Asn Thr Trp Gly Ser Phe Ala Ser
   225                 230                 235
<210> 54
   <211> 27
   <212> DNA
   <213> Artificial
<220>
   <223> Variant of 5' coding sequence for JS-TAG
<400> 54
   atggttggtg cagttacagc tccgctg 27
<210> 55
   <211> 27
   <212> DNA
   <213> Artificial
<220>
   <223> Variant of 5' coding sequence for JS-TAG
<400> 55
   atggtaggtg cagttacagc tccgctg 27
<210> 56
   <211> 27
   <212> DNA
   <213> Artificial
<220>
   <223> Variant of 5' coding sequence for JS-TAG
<400> 56
   atggttggtg cagtaacagc tccgctg 27

## Beschreibung

Die vorliegende Erfindung betrifft Polypeptide die eine enzymatisch nicht aktive, Zellwand-bindende Domäne eines Endolysins oder eines anderen Zellwand lysierenden Enzyms, und eine Sequenz gemäß SEQ ID NO: 1 oder Derivate gemäß SEQ ID NO: 2-18 davon umfassen, wobei das Polypeptid neben der Zellwand-bindenden Domäne keine weiteren Domänen eines Endolysins oder eines anderen Zellwand lysierenden Enzyms umfasst, sowie Mittel zu deren Herstellung. Ferner betrifft die vorliegende Erfindung Verfahren, um Bakterien, insbesondere gram-positive Bakterien, zu binden, anzureichern, aus einer Probe zu entfernen, einzufangen und/oder nachzuweisen.

Gram-positive Bakterien sind in der Umwelt weit verbreitet. Sie sind in Proben wie Erde, Wasser, Pflanzenmaterial, Fäkalien, aber auch in Menschen und Tieren zu finden. Eine ganze Reihe pathogener Keime wie z.B. aus den Gattungen Listeria, Bacillus, Clostridium, Staphylococcus, Streptococcus, Enterococcus, Micrococcus oder Mycobacterium ist von besonderer Bedeutung im Lebensmittelsektor sowie in Prävention, Diagnostik und Therapie von Infektionskrankheiten bei Mensch und Tier.

Bakterien der *Bacillus cereus* Gruppe repräsentieren Mikroorganismen von großer ökonomischer und medizinischer Wichtigkeit sowie herausragender Bedeutung im Bioterrorismussektor. Die Bakterien der Gruppe sind untereinander sehr nahe verwandt; eine große Anzahl davon sind sequenziert (Rasko et al, FEMS Microbiol. Reviews, 2005, 29, 303-329). Sie sind in der Natur weit verbreitet und kommen in verschiedenen Nahrungsmitteln, meist pflanzlichen Ursprungs, vor. Es handelt sich um aerob lebende, bewegliche, stäbchenförmige gram-positive Bakterien. Aufgrund ihrer resistenten Endosporen können sie verschiedene Verfahren überleben, die angewandt werden, um Lebensmittel haltbar zu machen, wie z. B. das Trocknen oder Erhitzen. Häufig befallene Lebensmittel sind vor allem stärkehaltige Nahrungsmittel, Getreide, Reis, Gewürze, Gemüse und Fertigprodukte. Fleisch kann durch Kontamination der verwendeten Gewürze befallen werden. Milchprodukte sind häufig belastet, weil Sporen die Pasteurisierung überleben und sich danach ungehindert vermehren können. Die *Bacillus cereus* Gruppe besteht aus 6 nahe miteinander verwandten Arten und beinhaltet neben dem Lebensmittelkeim *Bacillus cereus,* den äußerst gefährlichen humanpathogenen Keim *Bacillus anthracis,* dem eine immense Bedeutung im Bioterrorismussektor zugewiesen wird, den insektenpathogenen *Bacillus thuringiensis* (weite Verbreitung durch mikrobiologische Insektizide auf *B. thuringiensis* Basis), den rhizoidartigen *Bacillus mycoides* sowie *Bacillus pseudomycoides* und *Bacillus weihenstephanensis.*

Listerien sind human- und tierpathogene Bakterien, die häufig in Nahrungsmitteln insbesondere in Fisch, Fleisch und Milchprodukten vorkommen. Die Gattung Listeria umfaßt 6 verschiedene Species mit 16 unterschiedlichen Serotypen. Obwohl nur ein geringer Anteil der nahrungsmittelbedingten Erkrankungen von *Listerien* ausgelöst werden (ca. 1% in den USA), werden nahezu 30 % der jährlich tödlich verlaufenden Erkrankungen, die durch Nahrungsmittelpathogene verursacht werden, diesem Keim zugeschrieben. Betroffen sind vor allem immunsupprimierte Personen, z.B. ältere Menschen, Diabetiker, an Krebs und/oder AIDS erkrankte Personen. Schwangere und das noch ungeborene Kind stellen ca. 25 % aller Fälle der an invasiven Listeriosen Erkrankten dar.

Staphylokokken und Enterokokken sind derzeit mit die problematischsten Erreger bei Infektionskrankheiten, weil sie in zunehmendem Maße multiresistente Keime entwickeln (z.B. MRSA - multi resistant *Staphylococcus aureus* und VRE - vancomycin resistant *Enterococcus*), die zu dramatischen Entwicklungen, d.h. sowohl schlechten Krankheitsprognosen als auch einer Kostenexplosion, vor allem im stationären Gesundheitswesen führen.

Potentielle Pathogene sind im Infektionsstadium und im Lebensmittelbereich häufig in sehr kleinen Zellzahlen vorhanden und zusätzlich von einer störenden Hintergrundflora begleitet. Es werden zum einen Methoden benötigt, um relevante gram-positive Keime effizient zu entfernen, zum anderen Methoden, um sie selektiv anzureichern, um z.B. eine sensitive Detektion zu erreichen.

Kriterien für eine gute Detektionsmethode sind Empfindlichkeit, Schnelligkeit, Verlässlichkeit sowie einfache und kostengünstige Anwendung.

Am Anfang steht praktisch immer die Anreicherung der nachzuweisenden Organismen. Bei konventionellen Methoden wird dies in der Regel in einem mehrstufigen Prozess erreicht. Eine Primäranreicherung erfolgt meist mit nicht oder wenig selektiven flüssigen Nährmedien. Darauf folgt eine selektive sekundäre Anreicherung, gefolgt von einer primären häufig auf Selektivagar erfolgenden Isolation. Einzelkolonien werden in Subkultur erneut angereichert und anschließend mit diversen Nachweismethoden detektiert. Derartige konventionelle Verfahren dauern bis zum positiven Nachweis eines Keims sehr lange. So betragen die Standard Nachweiszeiten für Listerien nach ISO 11290-1:1996/FDAM 1:2004(E) mehr als 4-7 Tage und nach FDA und USDA/FSIS 4-7 Tage. Die Anreicherung von Bakterien der *Bacillus cereus* Gruppe dauert nach Standardmethoden (USFDA- method, chapter 14; ISO 7932:2004) 1,5 bis 2,5 Tage, der anschließende Nachweis 2 Tage und eine genauere Unterscheidung innerhalb der *Bacillus cereus* Gruppe nochmals 2-3 Tage.

In der Nahrungsmittelindustrie stellt die Nachweiszeit einen bedeutenden Faktor im Hinblick auf die kurze Haltbarkeit mancher Lebensmittel und die kostspielige Lagerung dar, die notwendig ist, bis abgesichert ist, dass die Probe nicht kontaminiert ist. Darüber hinaus können immer wieder kostspielige Rückrufaktionen beobachtet werden, wenn kontaminierte Ware vor Erhalt der Kontrollergebnisse vorzeitig ausgeliefert wurde. Im Gesundheitswesen sind lange Nachweiszeiten ebenso problematisch, weil sich geeignete spezifische Behandlungsmethoden auf gesicherter Grundlage ebenfalls erst nach einer Identifizierung der pathogenen Erreger durchführen lassen.

Als schnellere Alternative zu konventionellen Anreicherungs- und Nachweisverfahren wurden häufig Antikörper basierte Methoden eingesetzt (z. B. US 6,699,679, US 2004/0197833, UA 2006/0211061, Fluit et al., 1993, Appl Environ Microbiol., 59, 1289-1293, Jung et al., 2003, J Food Prot., 66, 1283-1287, Hawkes et al., 2004, Biosens. Bioelectron., 19, 1021-1028). Auch der Einsatz von zuckerbindenden Lektinen als Rezeptoren für den Kohlenhydratanteil der Bakterienoberflächen wurde angedacht. Jedoch sind diese meist zu unspezifisch, um aus einer Mischkultur bestimmte Bakterien zu selektieren und weisen aufgrund ihrer inultimeren Bindungseigenschaften häufig Agglutinationsprobleme auf Auch bei den Antikörper basierten Verfahren war die Wiederfindungsrate an Bakterien relativ schlecht, sie lagen im Bereich von 5 % bis 25 %. Weitere Nachteile von immunomagnetischen Separationsmethoden (IMS) sind neben der schlechten Wiederfindungsrate, ungenügende Empfindlichkeit bei niedrigen Kontaminationsraten, Kreuzreaktionen mit anderen Zellen und häufig Agglutinationsprobleme bei Antikörper beschichteten Partikeln. Zudem ist es relativ schwierig, Antikörper gegen intakte Bakterien zu gewinnen. Bei Reinkulturen sind die Methoden zwar vielversprechend, weisen jedoch bei Mischkulturen oder komplexen Matrizes wie Lebensmitteln erhebliche Probleme auf.

Als Alternative zu Antikörpern ist aus dem Stand der Technik die Verwendung Zellwandbindender Domänen (CBDs) von Bakteriophagen Peptidoglykanhydrolasen zur Bindung gram-positiver Bakterien bekannt (Loessner et al., 2002, Mol. Microbiol., 44, 335-349). EP 1147419 und WO2004/088321 verwenden CBDs zum Nachweis von Zellen, wobei die CBDs an eine feste Phase gebunden werden und in der Regel einen Marker tragen.

EP1399551 beschreibt ein Verfahren zur selektiven Aufreinigung von gram negativen Bakterienzellen oder Zellbestandteilen unter Verwendung von Bakteriophagenhüllproteinen oder Bakteriophagenschwanzproteinen. Hierbei werden die Bakterien in einem 2-Schritt-Verfahren, bei dem zuerst eine Bindung der Bindemoleküle an die Zielzellen, dann die Immobilisierung der Komplexe auf feste Träger erfolgt, gebunden. Zur Immobilisierung werden die Bakteriophagenschwanzproteine mit Hilfe von His-Tag, Biotin oder Strep-Tag an funktionalisierte Oberflächen von festen Trägern gekoppelt. Besonders für dieses 2-Schritt-Verfahren ist eine effiziente, schnelle und nicht kovalente Kopplung des Bindeprotein-Zielzellen-Komplexes an einen funktionalisierten Träger von entscheidender Bedeutung, vor allem, wenn die Zielzellen zusammen mit dem festen Träger von der Probe separiert werden sollen.

US 5,252,466 offenbart eine Methode, um Fusionsproteine herzustellen, die einen Tag zur *in vivo* Biotinylierung enthalten und damit leicht zu reinigen sind. Die Biotinylierungsdomänen stellen hierbei beispielsweise die 1.3S Untereinheit der *Propionibacterium shermanii* Transcarboxylase, Tomaten-Biotin-Protein, die α-Untereinheit der *Klebsiella pneumoniae* Oxalacetatdecarboxylase oder das *Escherichia coli* Biotin Carboxyl Carrier Protein dar, die im gleichen Leserahmen zusammen mit der Biotinligase von einem Plasmid exprimiert und so *in vivo* biotinyliert werden. Mit Hilfe eines Phagendisplaysystems wurde eine proteolytisch stabilere Minimalversion der Biotinylierungsdomäne der Klebsiella Oxalacetatdecarboxylase entwickelt, die sich, eignet, um entsprechende Fusionsproteine zu reinigen und zu detektieren (Stolz et al., 1998, FEBS-Lett. 440, 213-217). US 5,874,239 beansprucht ebenfalls eine Methode zur Biotinylierung von Fusionsproteinen, bei denen eine Reihe von Tags vorgeschlagen werden, so genannte "Avi-Tags", die mit einer Länge von 13 bis 50 Aminosäuren mit bevorzugten Formen von ca. 20 Aminosäuren kürzer sind als die Biotinylierungsdomäne der Klebsiella Oxalacetatdecarboxylase.

Daher liegt der Erfindung die Aufgabe zu Grunde, effizientere und produktivere Verfahren und die Mittel zu ihrer Durchführung bereitzustellen, um gram-positive Bakterien schnell, einfach und effizient zu binden, anzureichern, zu entfernen, einzufangen und nachzuweisen.

Die Aufgabe wird durch den in den Patentansprüchen definierten Gegenstand gelöst.

Die nachfolgenden Abbildungen dienen der Erläuterung der Erfindung.

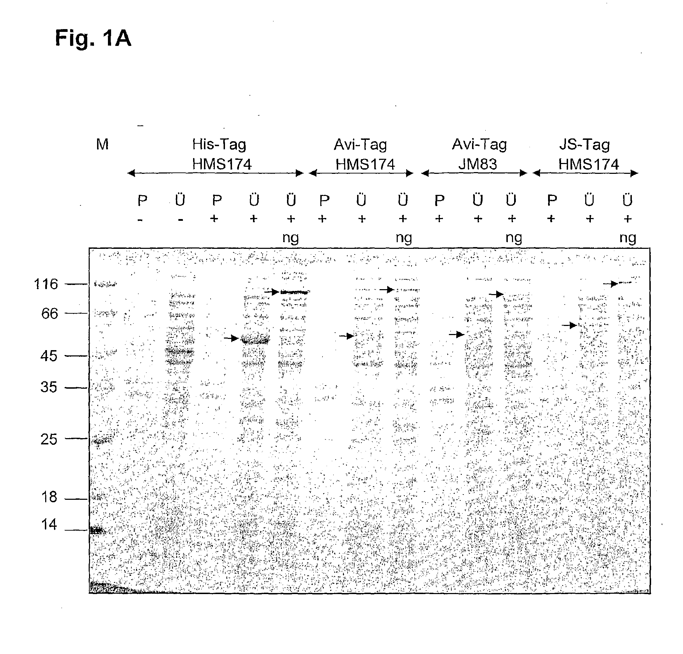

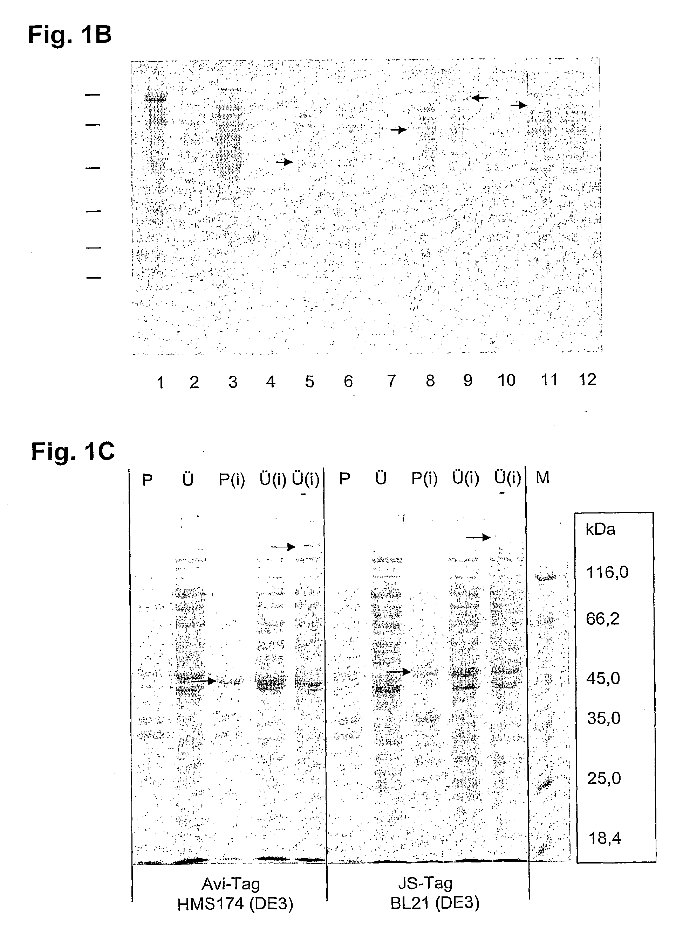

Figur 1: Bakteriophagenschwanzproteine mit JS-Tag
   Fig 1A: Vergleich der Expression und funktionellen Assemblierung von P22 ähnlichen Phagenschwanzproteinen mit verschiedenen Tags.
      Ein P22 ähnliches Phagenschwanzprotein aus *Salmonella* wurde mit Strep-Tag, Avi-Tag und JS-Tag am N-Terminus kloniert und *in E*. coli HMS174 (DE3) bzw. *E*. coli JM83 exprimiert. Die Proben wurden auf ein 12 %iges SDS-Gel aufgetragen und Coomassie gefärbt. Der Versuch ist in Experiment 1 beschrieben. M: Marker; P: Pellet; Ü: Überstand; +: induziert; -: nicht induziert; ng: nicht gekocht. Die Pfeile markieren die Positionen der Phagenschwanzproteinmonomere bzw. -trimere.
   Fig 1B: Klonierung und Expression mit JS-Tag von drei Bakteriophagenschwanzproteinen aus Salmonellaphagen
      Drei verschiedene Bakteriophagenschwanzproteine (Tsp) von Salmonellaphagen wurden als Fusionsproteine mit JS-Tag kloniert und in E. coli HMS174 (DE3) exprimiert. Die Proben aus dem Zellaufschluss wurden auf ein 12 % iges SDS-Gel aufgetragen und mit Coomassie gefärbt. Spur 1: Marker (Molekulargewichte von oben: 118 kDa, 85 kDa, 47 kDa, 36 kDa, 26 kDa, 20 kDa), Spur 2: Pellet (P), nicht induziert, Spur 3: Überstand (Ü), nicht induziert, Spur 4-12 (alle nach Induktion), Spur 4: Felix ähnliches Schwanzprotein (Felix-Tsp, 48 kDa), P, Spur 5: Felix-Tsp, Ü, gekocht, Spur 6: Felix-Tsp, ungekocht, Spur 7: P22 ähnliches Schwanzprotein (P22-Tsp, 67 kDa), P, Spur 8: P22-Tsp, Ü, gekocht, Spur 9: P22-Tsp, Ü, ungekocht, Spur 10: ∈15-ähnliches Schwanzprotein (∈15-Tsp, 93 kDa), P, Spur 11: ∈15-Tsp, Ü, gekocht, Spur 12: ∈15-typ, Ü, ungekocht. Die Pfeile markieren die erwarteten Positionen für die Phagenschwanzproteinmonomere und SDS-resistenten -trimere (bei nicht gekochten Proben).
   Fig 1C: Vergleichende Klonierung und Expression mit Avi-Tag bzw. JS-Tag eines Schwanzproteins eines Campylobacterphagen
      Ein *Campylobacter*-Phagenschwanzprotein (putative tail fiber protein H für *Campylobacter jejuni,* Acc. No: ZP01067412) wurde mit N-terminalem Avi-Tag bzw. JS-Tag (Version 5b) in das Plasmid pET21d kloniert und in *E*. *coli* HMS174(DE3) bzw. *E*. *coli* BL21(DE3) exprimiert. Der Versuch ist in Experiment 1 beschrieben. Dargestellt ist ein 9 %iges, Coomassie-gefärbtes SDS-Gel mit verschiedenen Proben aus dem Expressions- und Löslichkeitstest. P: Pellet; Ü: Überstand; (i): induziert; -: nicht gekocht; M: Marker. Die Pfeile markieren die Positionen der Monomere bzw. Trimere (bei nicht gekochten Proben) der Phagenschwanzproteine nach Induktion.

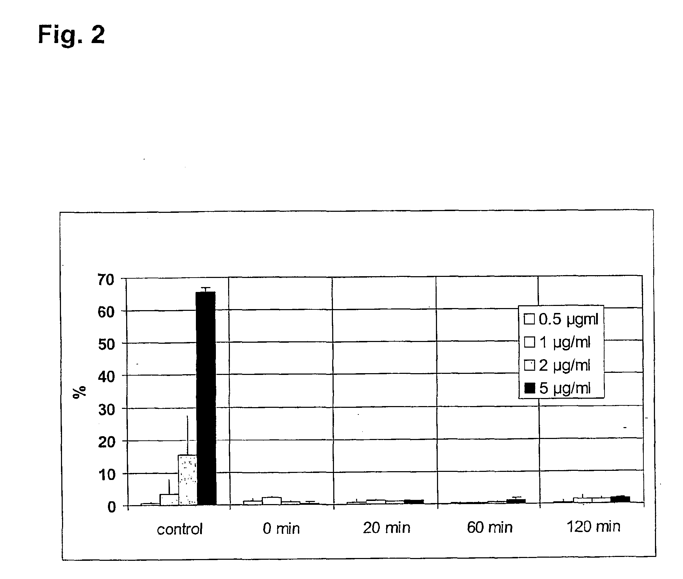

Figur 2: Listerien CBDs werden nach chemischer Biotinylierung komplett inaktiv
   Dargestellt ist, welcher Anteil der eingesetzten Listerien im Bindeassay nach chemischer Biotinylierung noch gebunden werden. Es wurden Proteinkonzentrationen von 0,5 µg/ml, 1 µg/ml, 2 µg/ml und 5 µg/ml im Test eingesetzt. Eine Inkubation mit NHS-Biotin erfolgte für 0 min, 20 min, 60 min und 120 min. Als Kontrolle wurde Avi-CBD eingesetzt.

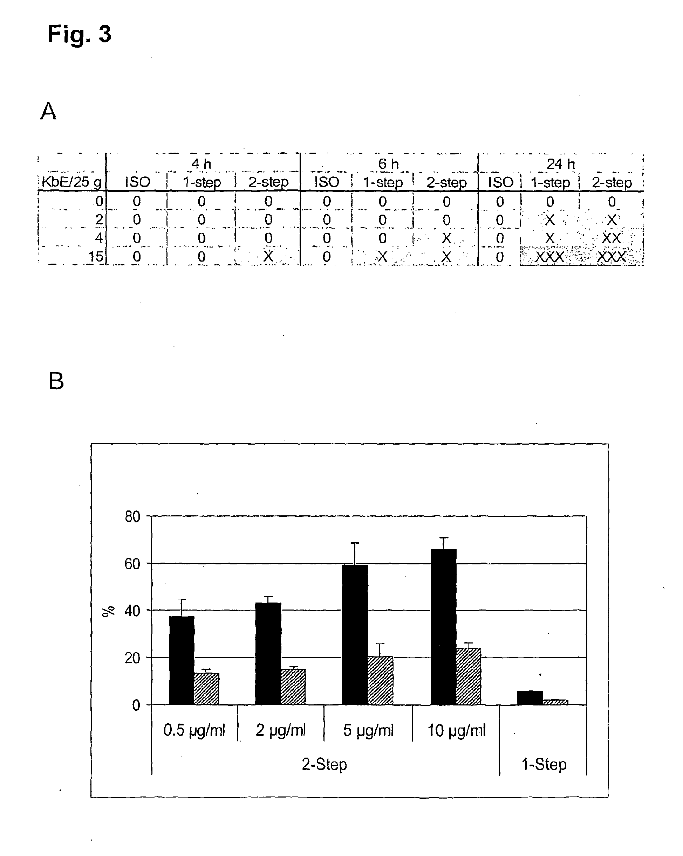

Figur 3: Vergleich von 1-Schritt und 2-Schrittverfahren
   Fig. 3A zeigt einen Vergleich des Listerien-Nachweises aus Camembert nach dem 1-Schritt-und 2-Schritt- sowie ISO-Verfahren.
      Es wird vergleichend die Zeitabhängigkeit untersucht, nach der bei den verschiedenen Verfahren sehr geringe Konzentrationen an *L. monocytogenes* in Camembert nachgewiesen werden können. 5 Portionen Camembert (je 25 g) wurden mit 0, 2, 4, 15 und 46 KbE kontaminiert und nach 4 h, 6 h und 24 h Anreicherung nach dem 1-Schritt-, 2-Schritt- oder ISO (ISO: 11290-1:1996 FDAM1)-Verfahren getestet. Für das 1-Schritt- und 2-Schritt-Verfahren wurde Strep-Tag-GFP-CBD511_f2 als spezifischer Ligand verwendet. Die Werte sind ermittelt aus je 4 Versuchen. (0: keine Kolonien auf Platte, X: < 10 Kolonien, XX: 10 - 30 Kolonien, XXX: > 30 Kolonien).
   Fig. 3B zeigt im 1-Schritt- und 2-Schritt-Verfahren die Konzentrationsabhängigkeit des Nachweises von *L. monocytogenes* (Stamm EGDe, schwarze Balken, und ScottA, schraffierte Balken) aus Mozzarella unter Verwendung des Fusionsproteins JS-GFP-CBD511_f2. Die Versuchsdurchführung ist in Experiment 3b beschrieben. Dargestellt ist jeweils, wie viel % der insgesamt eingesetzten Listeriazellen der jeweiligen Stämme aus 1ml Proben aus Mozzarella wieder gefunden wurden. Die Werte sind Mittelwerte aus jeweils 2 Versuchen.
Figur 4: JS-Tag im Vergleich zu Avi-Tag
   Figur 4A: Vergleich der Zellbindekapazität von JS-Tag und Avi-Tag Konstrukten.
      Die Zellbindekapazität der verschiedenen Konstrukte wurde mit dem *Listeria* Stamm ScottA nach dem 2-Schritt-Verfahren getestet. Es wurden folgende Konstrukte eingesetzt: JS-GFP_CBD511_f3 (Kreis), JS-CBD511_f3 (Quadrat) und Avi-GFP_CBD511_f3 (Dreieck). Angegeben ist der Anteil der an den magnetischen Partikeln haftenden Listerien in Prozent der eingesetzten Zellen. Alle Ansätze wurden 2fach durchgeführt und Mittelwerte gebildet.

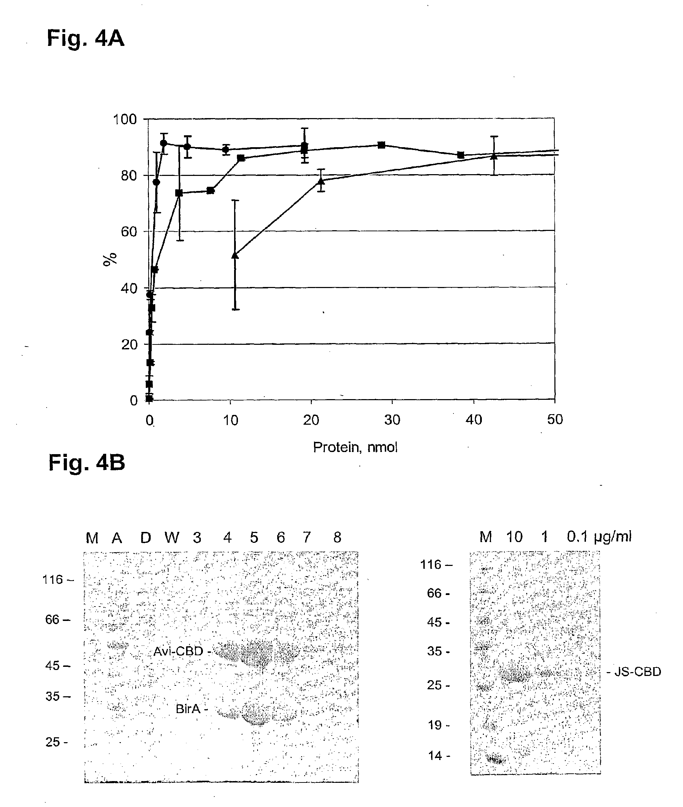

   Figur 4B: Vergleichende Reinigung von Avi-GFP-CBD511_f2 und JS-CBD511_f2.
      Das linke Bild zeigt ein Coomassie gefärbtes Gel der Reinigung von Avi-GFP-CBD511_f2, das rechte das Endprodukt der Reinigung von JS-CBD511_f2. Der Versuch ist in Experiment 4b beschrieben. M: Marker; A: Auftrag auf die Säule; D: Durchlauf; W: Waschfraktion; 3 - 8: Fraktionen, in denen Avi-GFP-CBD511_f2 enthalten ist. 10, 1, 0.1 µg/ml: aufgetragene Proteinkonzentrationen für JS-CBD511_f2. Die Positionen der Banden für Avi-GFP-CBD511_f2 und JS-CBD511_f2 sowie BirA sind markiert.

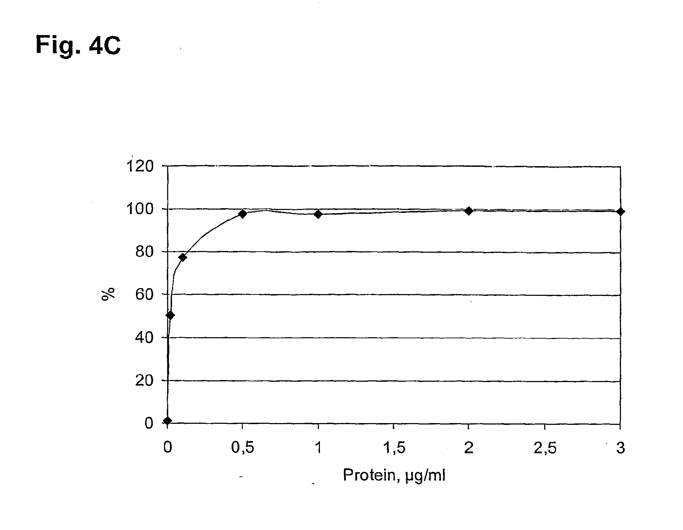

   Figur 4C: Konzentrationsabhängigkeit der Bindung von Listerien
      Es wurde untersucht, ab welcher Konzentration an spezifischem Bindeprotein die maximale Zellbindung erreicht wird. Als Bindeprotein wurde JS-CBD511_f3 in den Konzentrationen 0 µg/ml, 0,02 µg/ml, 0,1 µg/ml, 0,5 µg/ml, 1 µg/ml, 2 µg/ml und 3 µg/ml eingesetzt. Der Versuch ist in Experiment 4b beschrieben.

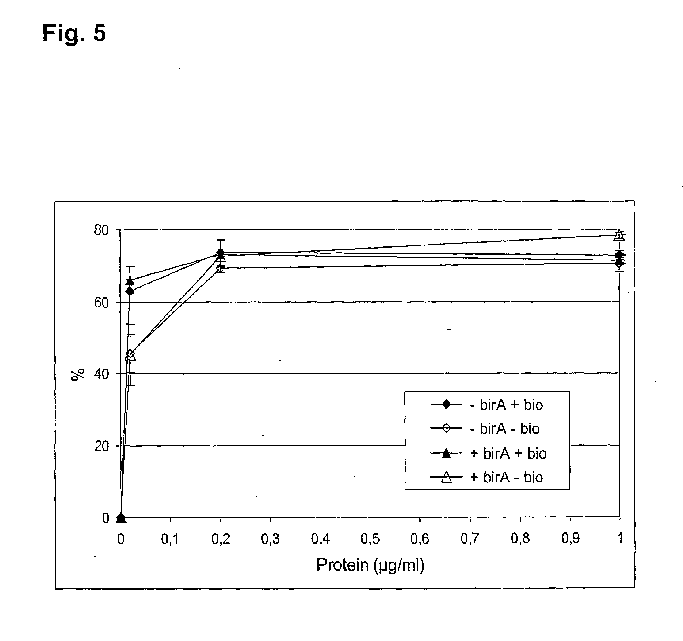

Figur 5: Auswirkung der Koexpression von birA auf die Bindungseffizienz von JS-Tag-CBDs.
   Dargestellt ist der Anteil der gebundenen Bakterienzellen (*Listeria* ScottA) in Abhängigkeit von der eingesetzten Menge an spezifischem Bindeprotein JS5b-CBD511_f2. Bei einem Teil der Proteine wurde birA auf einem Zusatzplasmid koexprimiert (Dreiecke), beim anderen Teil erfolgte keine Koexpression mit birA (Rauten). Außerdem wurde bei 2 Versuchen zusätzlich Biotin zugegeben (gefüllte Symbole), während dies bei 2 Versuchen unterblieb (offene Symbole).

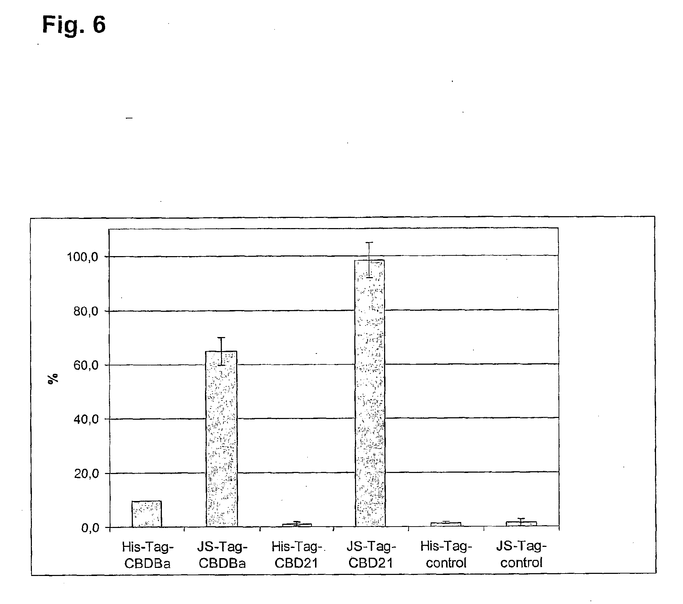

Figur 6: Vergleich der spezifischen Bindung von JS-Tag-CBDs an Streptavidin-Partikel mit der Bindung von His-Tag-CBDs an Nickel-NTA-Partikel.
   *Bacillus cereus* Bakterien wurden im 2-Schritt-Verfahren mit zwei verschiedenen CBDs (CBDBa und CBD21) entweder über Hexa-His-Tag an Nickel-NTA-Magnetpartikel oder über JS-Tag an Streptavidin-Magnetpartikel gebunden. Dargestellt ist der Anteil der spezifisch gebundenen Bakterien an den insgesamt eingesetzten Bakterien (Konzentration 3x10³ KbE/ml).

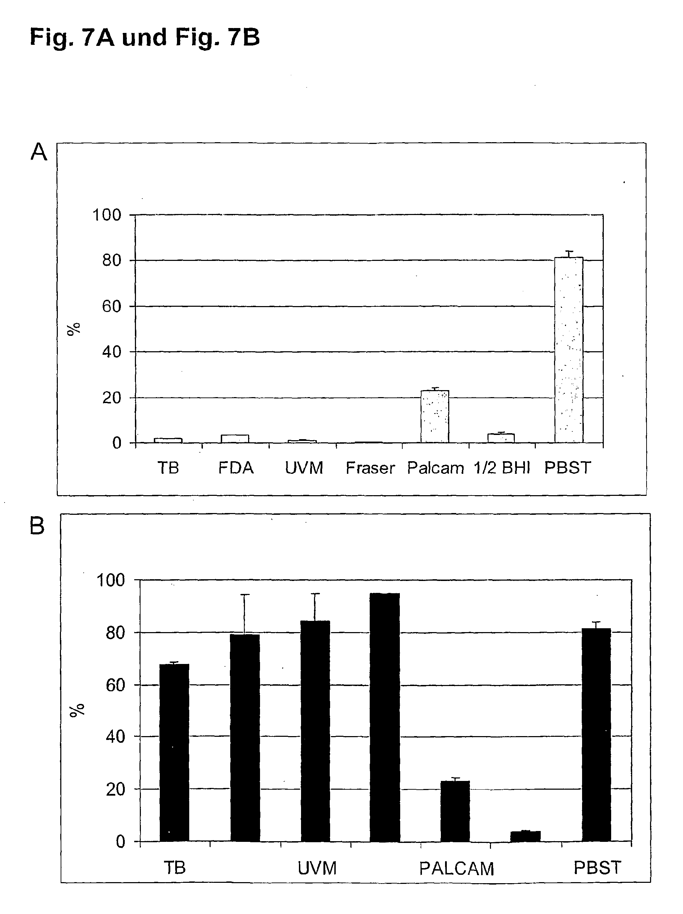

Figur 7: Bakterienbindung mit Strep-Tag- bzw. JS-Tag-CBDs in verschiedenen Medien. *Listeria monocytogenes* EGDe Zellen wurden nach dem erfindungsgemäßen Verfahren aus verschiedenen Medien bzw. PBST-Puffer angereichert. Der Versuch ist in Experiment 8 erläutert.
   Fig. 7A: Avi-CBD511_f2 (5 µg/ml) wurde verwendet, um Listerien anzureichern.
   Fig. 7B: JS-CBD511_f2 (5 µg/ml) wurde verwendet, um Listerien anzureichern.

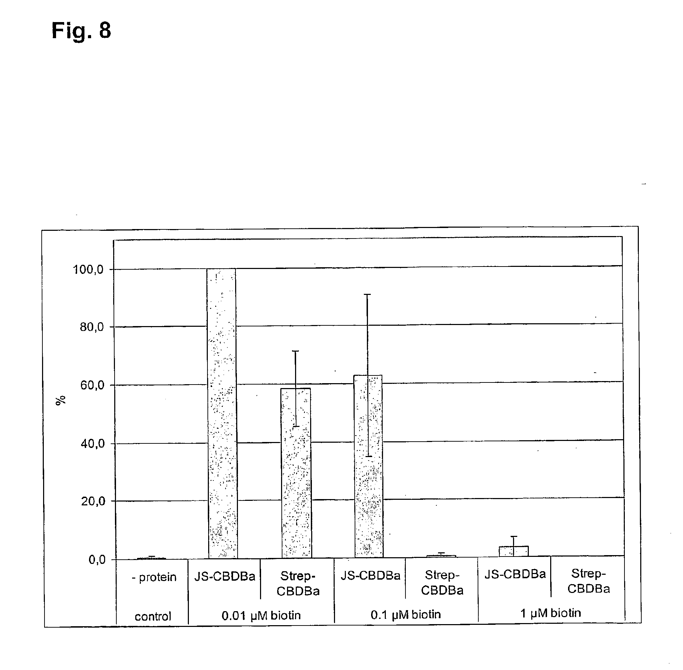

Figur 8: Bakterienbindung in biotinhaltigen Proben
   Die spezifische Anreicherung von *Bacillus cereus* mit den Konstrukten Strep-Tag-CBDBa und JS-Tag-CBDBa wurde bei Biotinkonzentrationen von 0,01 µM, 0,1 µM und 1 µM im Vergleich untersucht.

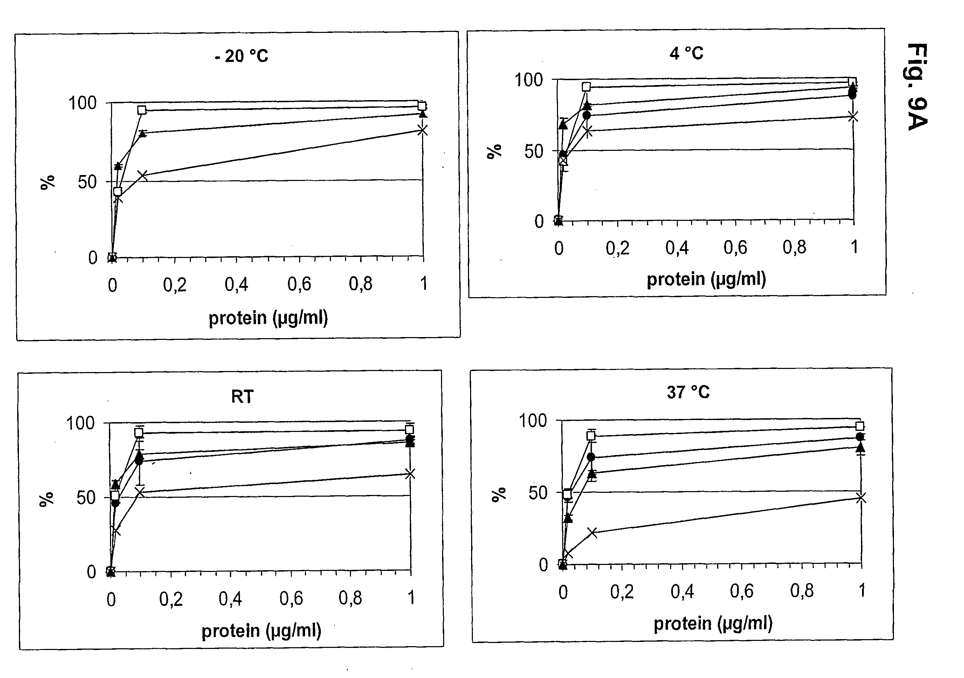

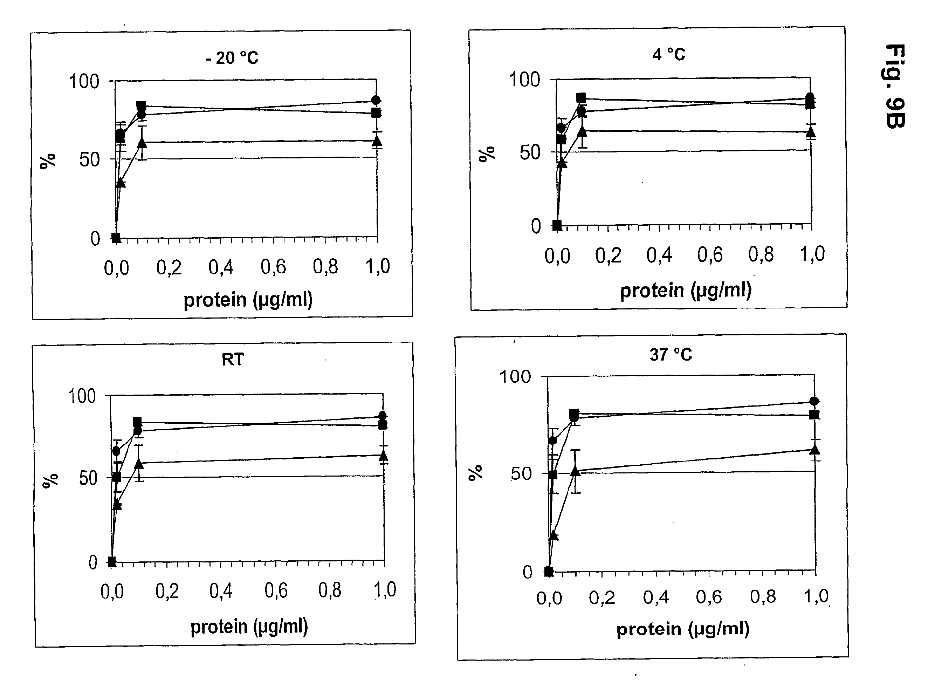

Figur 9: Langzeitstabilität der JS-Tag-CBDs unter verschiedenen Bedingungen
   Sowohl JS-CBD511_f3 als auch magnetische Streptavidin-Partikel wurden im Temperaturbereich - 20 °C bis 37 °C inkubiert und danach in Zellbindetests mit *Listeria monocytogenes* ScottA eingesetzt.
   Fig. 9A: Inkubation in Natriumphosphat, pH 7, 2 mM EDTA. Angegeben ist der Anteil der gebundenen Listerien bei der angegebenen Konzentration an JS-CBD511_f3 nach 1 Tag (geschlossener Kreis), 14 Tagen (offenes Rechteck), 60 Tagen (geschlossenes Dreieck) und 126 Tagen (Kreuz).
   Fig. 9B: Inkubation in Imidazol, 100 mM NaCl, pH 7, + 30 % AS. Angegeben ist der Anteil der gebundenen Listerien bei der angegebenen Konzentration an JS-CBD511_f3 nach 0 Tagen (Kreis), 33 Tagen (Rechteck) une 74 Tagen (Dreieck).

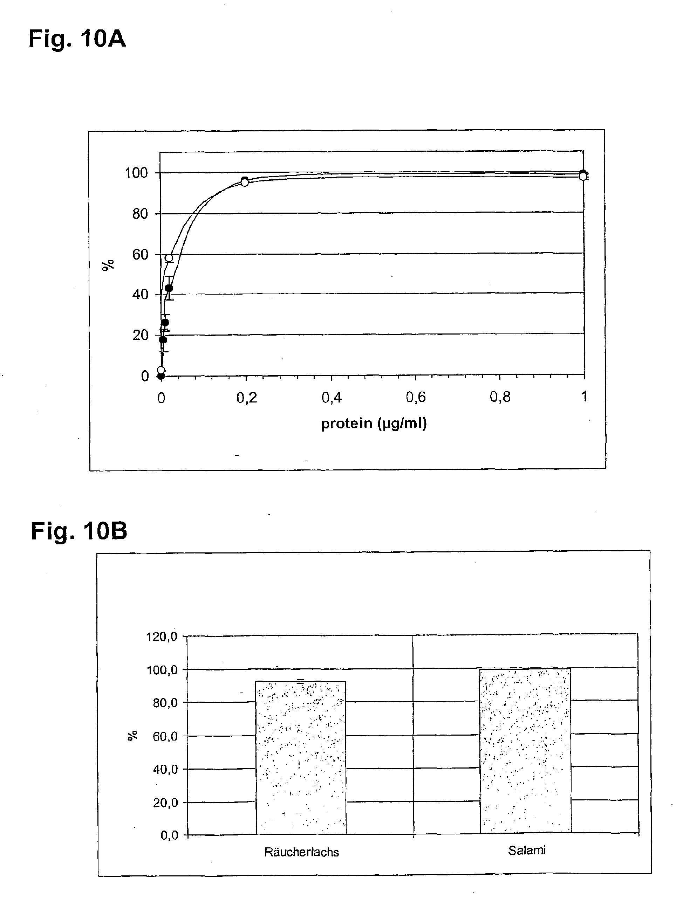

Figur 10: *Listeria* Capture mit JS-CBD511-Konstrukten aus verschiedenen Lebensmitteln
   Figur 10A: Konzentrationsabhängige Entfernung von *Listeria monocytogenes* ScottA aus Milch und Käse. JS4b-CBD511_f2 wurde in verschiedenen Konzentrationen zur Bindung eingesetzt. Angegeben ist, wie viele % der eingesetzten Bakterien bei den jeweiligen Proteinkonzentrationen an die Magnetpartikel gebunden wurden.
   Figur 10B: Entfernung von *Listeria innocua* aus Salami und Räucherlachs.

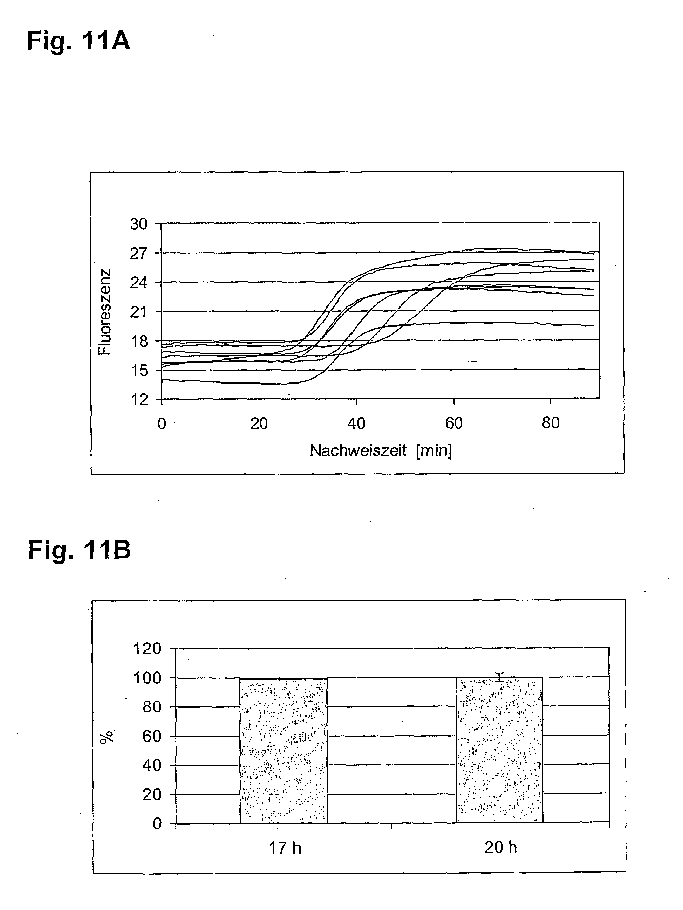

Figur 11: Anreicherung von Listerien aus Lebensmitteln und anschließender Nachweis mit NASBA Technik
   Aus Salami, die in geringen Mengen mit *Listeria monocytogenes* Scott A kontaminiert worden war (5 KbE/25 g), wurden nach 17 h bzw. 20 h Vorinkubation in LEB-FDA Medium unter Verwendung von JS5b-CBD511_f2 die Listerien angereichert. In Figur 11A ist das Fluoreszenzsignal dargestellt, dass sich nach der Enzymreaktion mit Listerien spezifischen Primern ergibt. Figur 11B gibt an, wie viele % der eingesetzten Bakterien mit JS5b-CBD511_f2 aus der Probe gebunden werden können.

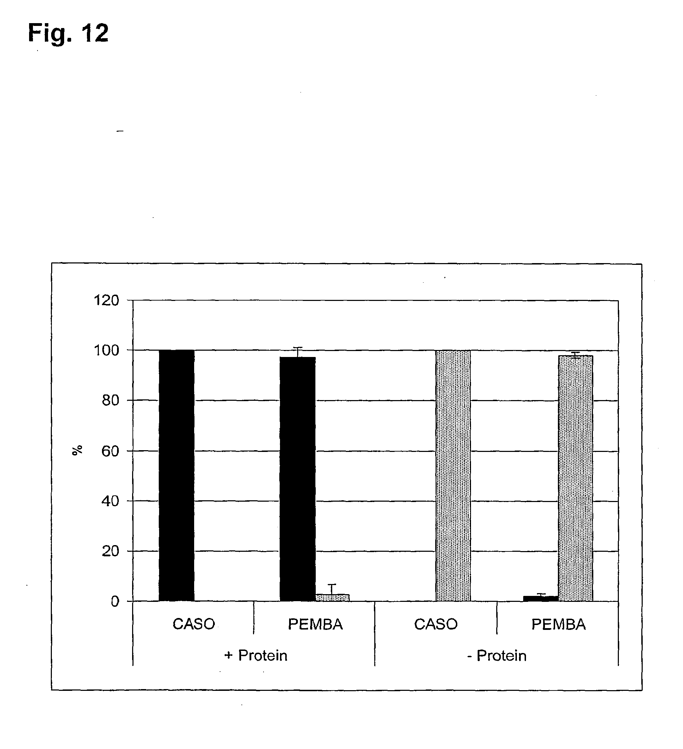

Figur 12: Spezifische Bindung von *Bacillus cereus* aus einer Mischung von Bakterien.
   Unter Verwendung der spezifischen JS-Tag-CBDBa wird *Bacillus cereus* aus einer Mischung von Bakterien (*Bacillus cereus* (DSMZ345), *Salmonella tennessee, Listeria monocytogenes* (ScottA), *Staphylococcus aureus, E. coli* HMS174 (DE3)) angereichert. Angegeben ist die Anzahl der gebundenen *Bacillus cereus* Zellen in % der insgesamt wiedergefundenen Zellen, die an die Magnetpartikel (schwarze Balken) gebunden wurden oder im Überstand bzw. der Waschfraktion (schraffierte Balken) verblieben. Die Zellen wurden zum einen auf Vollmedium ausplattiert (CASO, Merck), um auch die anderen Zellen aus der Mischkultur zu erhalten, zum anderen auf Selektivplatten für *Bacillus cereus* (PEMBA). Als Kontrollen wurden Versuche durchgeführt, bei denen zwar Magnetpartikel, aber kein Bindeprotein zugegeben wurde.

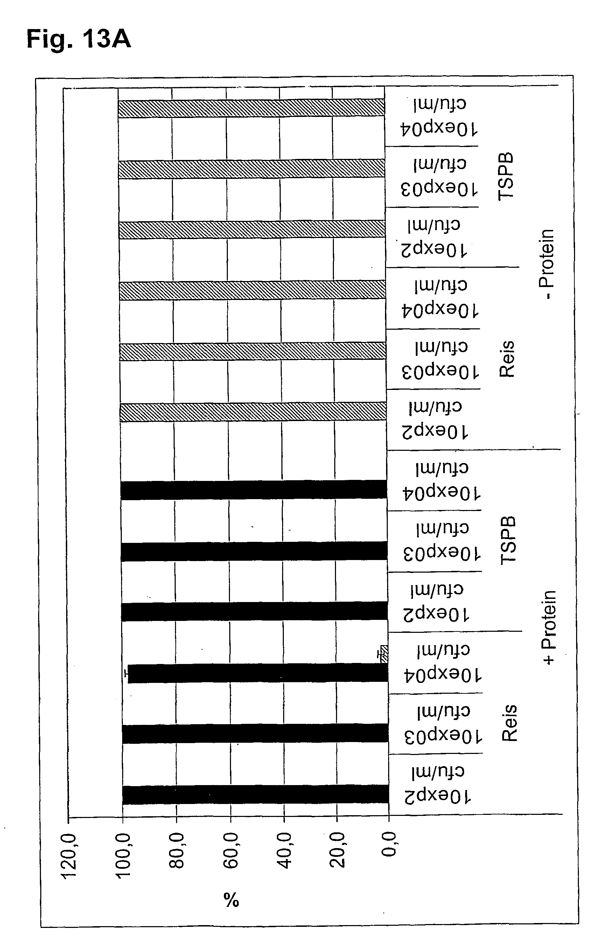

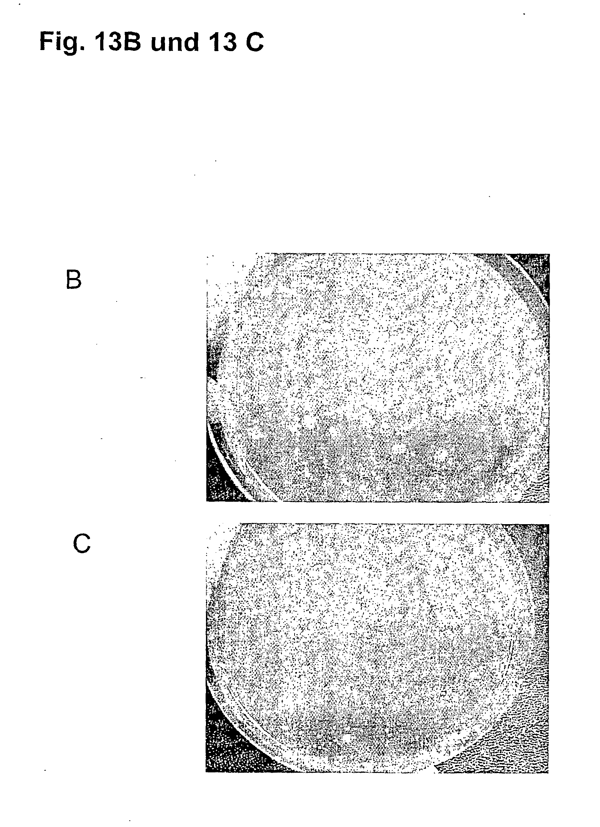

Figur 13: Anreicherung von *Bacillus cereus* aus Lebensmitteln.
   Das Auftreten von pathogenen Keimen aus der *Bacillus cereus*-Gruppe ist ein besonderes Problem bei vorgekochten stark kohlenhydrathaltigen Lebensmitteln wie Fertigprodukten oder wieder aufgewärmtem Reis. Aus diesem Grund wurde als Lebensmittelprobe vorgekochter Reis verwendet, der zum Fertiggaren nur noch 2 min in der Mikrowelle erhitzt werden muss. Die mit Medium verdünnte und homogenisierte Lebensmittelprobe wurde mit *Bacillus cereus* Zellen in Konzentrationen von 10², 10³ oder 10⁴ KbE/ml bespikt. TSPB-Medium wurde als Kontrolle eingesetzt. Figur 13A zeigt die Anzahl der gebundenen Zellen in % der wieder gefundenen Zellen in der Beadsfraktion (schwarze Balken) und in den Überständen (schraffierte Balken). Figur 13B zeigt exemplarisch eine PEMBA-Platte, auf der die Beadsfraktion mit den daran gebundenen Zellen ausplattiert wurde. *Bacillus cereus* Kolonien sind zu erkennen an ihrem blumenförmigen und ausufernden Wachstum. Figur 13C zeigt im Vergleich eine PEMBA-Platte, auf der eine Überstandsfraktion mit den darin enthaltenen Zellen ausplattiert wurde.

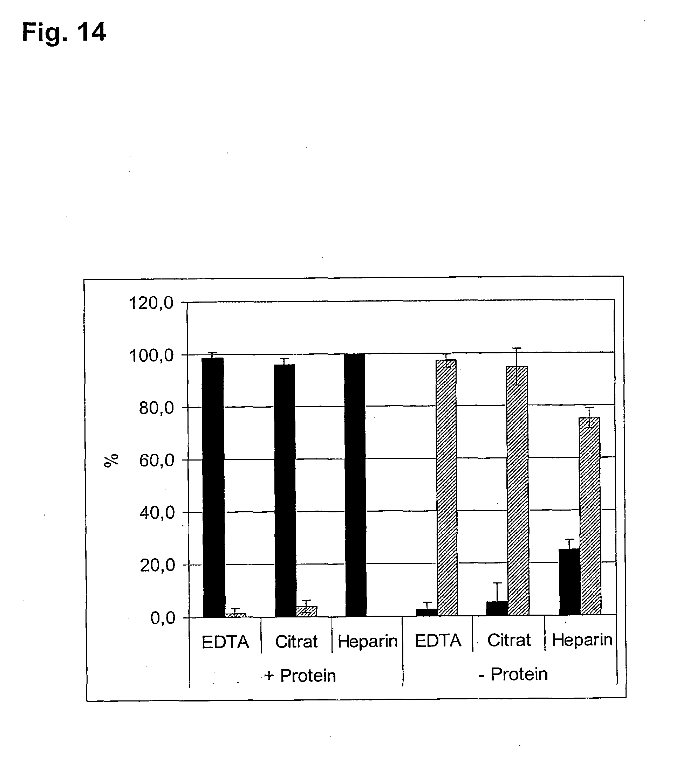

Figur 14: Anreicherung von *Bacillus cereus* aus Blut mit Hilfe von CBD 21 und Magnetbeads Menschliches Blut wurde mit *Bacillus cereus* (DSMZ345) in einer Konzentration von 10³ KbE/ml bespikt. Die Blutproben wurden vorher mit Citrat, EDTA oder Heparin versetzt, um die Blutgerinnung zu hemmen, und 1:1 mit PBST-Puffer verdünnt. JS-Tag-CBD21 (10 µg/ml) (schwarze Balken), Kontrollen ohne Protein (schraffierte Balken).

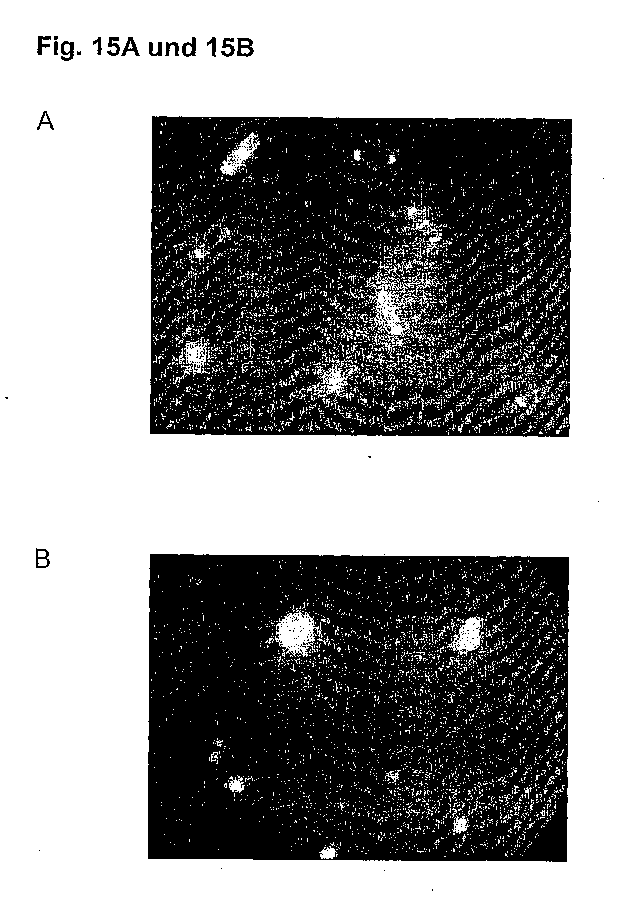

Figur 15: Spezifische Bindung von *Clostridium perfringens* mit JS-Tag-CBD3626
   Die grüne Fluoreszenz des Abstandshalters GFP wurde hier als Marker verwendet, um die Bindung von JS-Tag-GFP-CBD3636 an die Clostridien bzw. an die Magnetpartikel sichtbar zu machen. Figur 15 A zeigt exemplarisch die Bindung von JS-Tag-GFP-CBD3626 an *Clostridium perfringens* Zellen. 15 B zeigt exemplarisch die Bindung von JS-Tag-GFP-CBD3626 an Magnetpartikel.

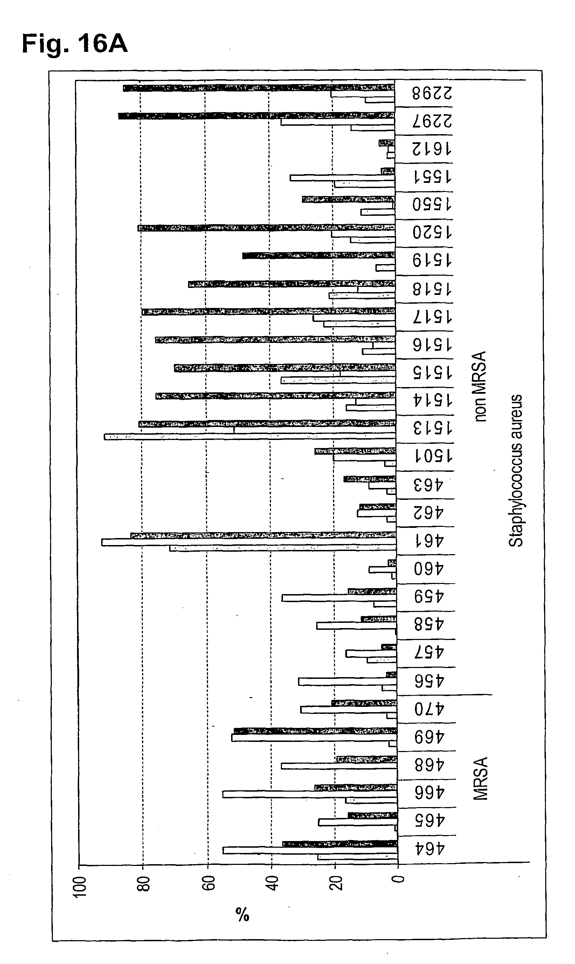

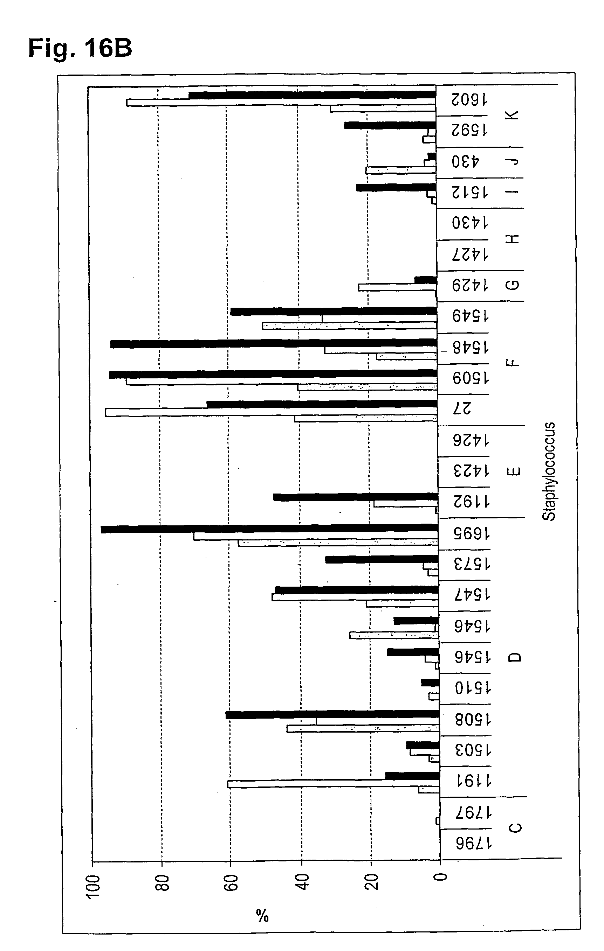

Figur 16: Spezifische Zellbindung verschiedener *Staphylococcus* Stämme durch erfindungsgemäße JS-Tag-CBD-Konstrukte gemessen im Zellbindungstest
   Es wurden die erfindungsgemäßen JS-Tag-CBD-Konstrukte JS-CBDPitti20 (graue Balken), JS-CBDOpf (weiße Balken) und JS-CBDUSA (schwarze Balken) verwendet. Dargestellt ist, wie viele Prozent der eingesetzten Zellen im Zellbindungstest spezifisch gebunden wurden. Die Durchführung des Versuchs ist in Experiment 20 beschrieben. Die angegebenen Zahlen sind Nummern aus der PROFOS Stammsammlung. Die *Staphylococcus* Stämme sind Patientenisolate sowie DSMZ bzw. ATCC Stämme.
   Figur 16A: Zellbindung an verschiedene *Staphylococcus aureus* Stämme, unterteilt in MRSA Stämme und nicht MRSA Stämme.
   Figur 16B: Zellbindung an verschieden nicht *Staphylococcus aureus* Staphylokokken Stämme: C = *Staphylococcus carnosus,* D = *Staphylococcus epidermidis*, E = *Staphylococcus equorum*, F = *Staphylococcus haemolyticus*, G = *Staphylococcus saprophyticus*, H = *Staphylococcus sciuri*, I = *Staphylococcus simulans*, J = *Staphylococcus warneri*, K =
   *Staphylococcus xylosus*

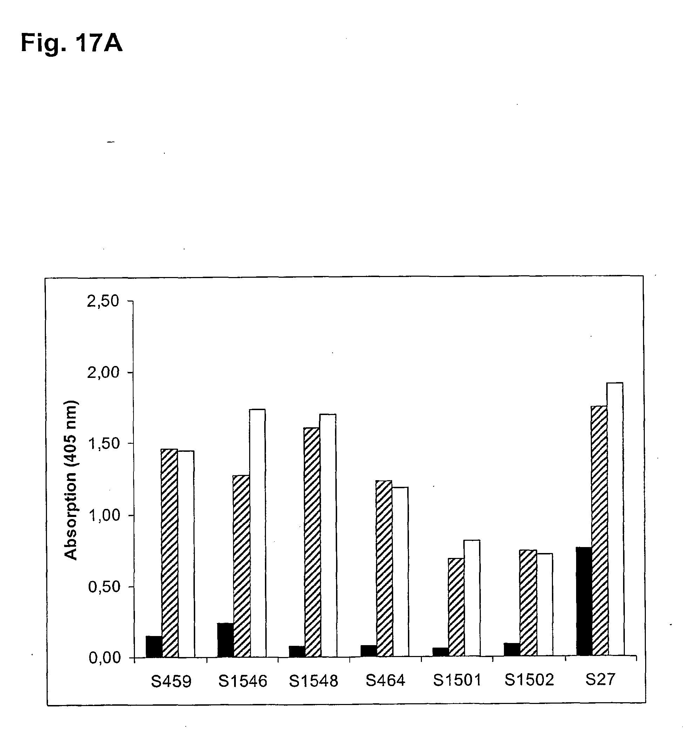

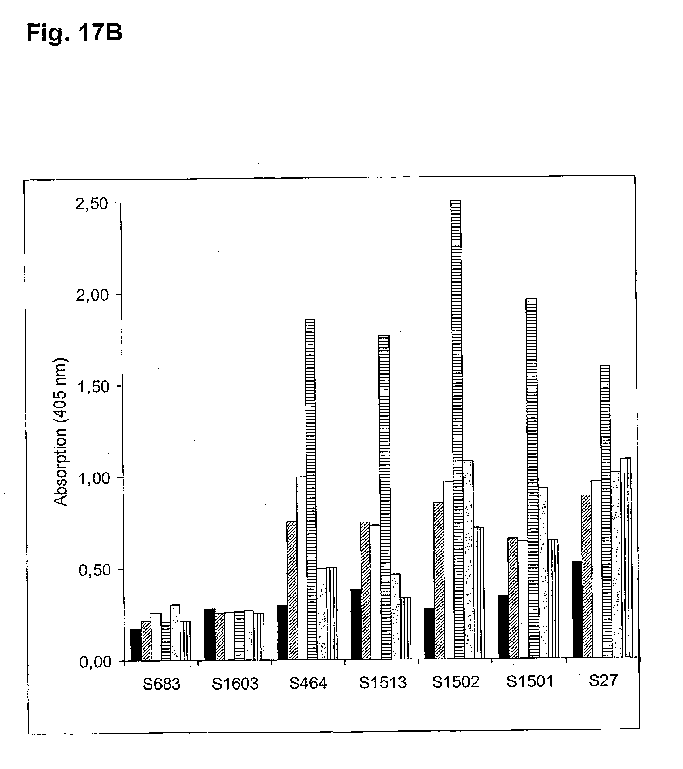

Figur 17: Spezifische Zellbindung verschiedener *Staphylococcus* Stämme durch erfindungsgemäße JS-Tag-CBD-Konstrukte im Peroxidasetest
   Die Durchführung und das Prinzip des Peroxidasetests sind in Experiment 21 beschrieben. Figur 17A zeigt die spezifische Bindung der JS-Tag-Konstrukte JS-CBDALE-1 (schraffierte Balken) und JS-CBDLS (Lysostaphin) (weiße Balken) an verschiedene *Staphylococcus* Stämme. Die Hintergrundabsorption einer Pufferkontrolle ohne Proteinzugabe (schwarze Balken) ist ebenfalls dargestellt. Folgende *Staphylococcus* Stämme wurden im Test eingesetzt: S459 - *S*. *aureus* (Patientenprobe), S1546 *- S*. *epidermidis* (DSMZ 20044), S 1548 *- S. haemolyticus* (DSMZ 20228), 5464 - *S.aureus* (Patientenprobe), S1501 - *S.aureus* MRSA (Patientenprobe), S1502 - *S.aureus* MRSA (Patientenprobe), S27 *S.haemolyticus.* Figur 17B zeigt die spezifische Bindung weiterer *Staphylococcus* spezifischer JS-Tag-Konstrukte an eine Reihe von *Staphylococcus* Spezies sowie an einen E. *coli* Stamm als unspezifische Kontrolle. Pufferkontrolle (schwarze Balken), JS-CBDALE-1 (schraffiert), JS-CBDLS (weiß), JS-CBDPitti20 (horizontal gestreift), JS-CBDOpf (grau), JS-CBDUSA (vertikal gestreift). Folgende Bakterienstämme wurden im Test eingesetzt: S683 - *E*. *coli* (ECOR01), S1603 - *Streptococcus mutans* (DSMZ 20523), S464 - *S*. *aureus* (Patientenisolat), S1513 - *S*. *aureus* (Patientenisolat), S1502 - *S.aureus* MRSA (Patientenprobe), S1501 - *S.aureus* MRSA (Patientenprobe), S27 *S.haemolyticus.*

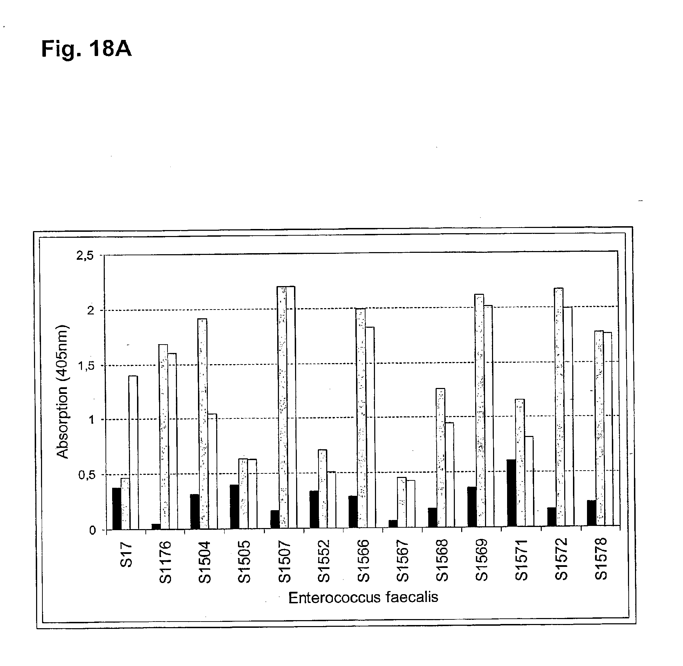

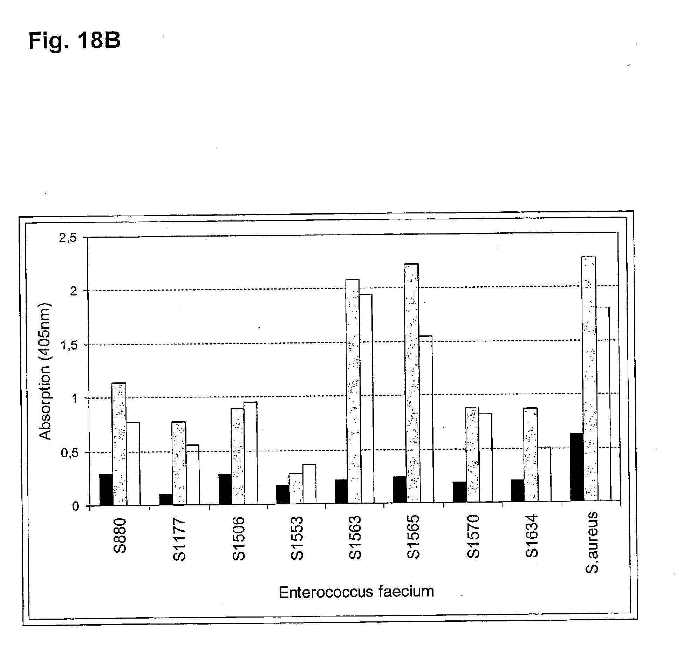

Figur 18: Spezifische Zellbindung im Peroxidasetest durch erfindunsgemäße Polypeptidkonstrukte abgeleitet aus *Enterococcus* Endolysinen
   Die Durchführung des Tests ist in Experiment 22 beschrieben. Es wurden zwei erfindungsgemäße JS-Tag-CBD-Konstrukte eingesetzt: JS-CBDEF0355 (graue Balken) und JS-CBDEF1293 (weiße Balken). Die Pufferkontrolle ohne Proteinzugabe (schwarze Balken) ist ebenfalls dargestellt. Die angegebenen Nummern sind Stammnummern aus der PROFOS Stammsammlung. Es handelt sich um Patientenisolate sowie DSMZ und ATCC Stämme der Gattungen *Enterococcus* und *Staphylococcus.*
   Figur 18A zeigt die spezifische Bindung an verschiedene *Enterococcus faecalis* Stämme.
   Figur 18B zeigt die spezifische Bindung an verschiedene *Enterococcus faecium* Stämme sowie an einen *Staphylococcus aureus* Stamm.

Die Erfindung wird im Folgenden beschrieben.

Der Begriff "Bakterien-Entfernung" wie hier verwendet bedeutet vollständige oder teilweise Entfernung von Bakterien aus Probenmaterial.

Der Begriff "Bakterien-Anreicherung" wie hier verwendet bezeichnet die Konzentrierung der Bakterien ausgehend von der in der Probe vorhandenen Ausgangskonzentration. Die Anreicherung hat in der Regel in dem Maße zu erfolgen, dass ein entsprechender Nachweisschritt eine eindeutig positive oder eindeutig negative Aussage ermöglicht.

Der Begriff "Einfangen" von Bakterien (engl. "capture") bezeichnet den Vorgang des Herausholens und Bindens der Bakterien aus einer Probe mit Hilfe der Fähigkeit der "CBDs", Bakterien aus der Probe spezifisch zu binden.

Der Begriff "Probenmaterial" oder "Probe" wie hier verwendet umfasst sämtliche Lösungen, in denen Bakterien nachgewiesen werden sollen oder aus denen Bakterien entfernt werden sollen. Beispiele für geeignete Proben sind: wässrige Lösungen und Gemische aus Wasser und organischen Lösungsmitteln, Nahrungsmitteln, Medien, Blut, Blutprodukte, Plasma, Serum, Urin, sonstige Körperflüssigkeiten, diagnostische Proben, Proteinlösungen, Wasser-Ethanol Gemische, Prozesslösungen wie z.B. Waschlösungen zur Untersuchung der Kontamination medizinischer Geräte oder in der Lebensmittelverarbeitung. Umfasst sind ferner auch Lösungen, in denen zu untersuchende oder zu isolierende nicht-wässrige feste Substanzen gelöst wurden, beispielsweise Proteine, DNA, RNA, Zucker, Salze, Nahrungsmittel, Nahrungsmittel-Medien-Homogenisate, Arzneimittel, Impfstoffe, Umweltproben, Fäkalien, organische und anorganische Chemikalien, z.B. NaCl, MgCl₂, Purine, Pyrimidine.

Der Begriff "Endolysin" wie hier verwendet bezeichnet ein Enzym, das in seiner ursprünglichen Funktion zur Freisetzung neuer Phagen am Ende eines jeweiligen Phagenreproduktionszyklusses dient. In der Natur kann solch ein Endolysin beispielsweise vom Phagengenom kodiert werden. Diese Endolysine bestehen aus mindestens je einer enzymatisch aktiven Domäne und einer an die Zellwand der jeweiligen Wirtszelle bindenden enzymatisch nicht aktiven Domäne. Zusätzlich sind unter Endolysin auch die ähnlich aufgebauten Autolysine zu verstehen. Diese sind in der Natur bakterienkodiert und bestehen ebenfalls aus mindestens je einer enzymatisch aktiven zellwandhydrolysierenden Domäne und einer an die Zellwand des Zielbakteriums bindenden enzymatisch nicht aktiven Domäne. Phagenkodierte Endolysine und bakterienkodierte Autolysine sind häufig homolog zueinander (Garcia et al., 1988, Proc. Natl. Acad. Sci. USA, 85, 914-918) und die Module sind austauschbar und können sogar in Chimären aus bakterien- und phagenkodierten Sequenzen miteinander kombiniert werden (Diaz et al., 1990, Proc. Natl. Acad. Sci. USA, Vol. 87, 8125-8129). Deshalb können erfindungsgemäß neben den Zellwand bindenden Domänen aus Phagenendolysinen auch die entsprechenden nicht enzymatisch aktiven Zellbindedomänen anderer Zellwand lysierender Enzyme wie z.B. Autolysine, Bakteriocine oder Phagenschwanzproteine eingesetzt werden.

Der Begriff "CBD" wie hier verwendet bezeichnet Polypeptiddomänen bzw. -sequenzen, die von Endolysinen oder anderen Zellwand lysierenden Enzymen stammen und für die spezifische Bindung der Endolysine oder der anderen Zellwand lysierenden Enzyme an die Bakterienzellwand verantwortlich sind. Diese sind enzymatisch nicht aktiv.

Der Begriff "erfindungsgemäßes Polypeptid", wie hier verwendet, bezeichnet ein Polypeptid, das eine enzymatisch nicht aktive, Zellwand-bindende Domäne eines Endolysins oder eines anderen Zellwand lysierenden Enzyms (CBD), und eine Sequenz gemäß SEQ ID NO: 1 oder Derivate davon umfasst, wobei das Polypeptid neben der Zellwand-bindenden Domäne keine weiteren Domänen eines Endolysins oder anderen Zellwand lysierenden Enzyms umfasst, insbesondere keine vollständige enzymatisch aktive Domäne (EAD) eines Endolysins.

Der Begriff "JS-Tag" wie hier verwendet bezeichnet eine Polypeptidsequenz, die eine Sequenz gemäß SEQ ID NO: 1 oder Derivate gemäß SEQ ID NO: 2-18 davon umfasst. Der JS-Tag stammt aus der Biotinakzeptordomäne der α-Untereinheit der *Klebsiella pneumoniae* Oxalacetatdecarboxylase und enthält die Konsensussequenz MKM (K wird biotinyliert), damit das Polypeptid *in vivo* durch das Protein Biotinligase biotinyliert werden kann. Gegenüber der kompletten α-Untereinheit der *Klebsiella pneumoniae* Oxalacetatdecarboxylase ist der JS-Tag verkürzt. Eine mögliche Minimalsequenz für den JS-Tag umfasst 66 Aminosäuren entsprechend Aminosäuren 529 bis 594 der *Klebsiella pneumoniae* Oxalacetatdecarboxylase (SEQ ID NO: 2). Der Begriff "JS-Tag" umfasst auch Derivate gemäß SEQ ID NO. 2-18 der Sequenz gemäß SEQ ID NO:1. Derivate, wie hierin verwendet, umfasst solche Sequenzen, die noch zu mindestens 80% homolog zu SEQ ID NO: 1 sind. Beispiele für solche Derivate sind in den SEQ ID NO: 2-18 angegeben

Der Begriff "gerichtete Immobilisierung" wie hier verwendet bedeutet, dass die CBDs über Biotin als spezifischem Kopplungsagens an geeigneten Oberflächen immobilisiert werden, z.B. über mit Streptavidin- oder Avidin- versehene Magnetpartikel oder andere Träger.

Der Begriff "Oberfläche" oder "Träger" wie hier verwendet umfasst alle Materialien, an die eine Kopplung oder Adhäsion eines CBD-Moleküls bzw. erfindungsgemäßen Polypeptids direkt oder indirekt möglich ist, wie z.B. an Glasoberflächen, an Chromatographiematerialien wie Agarose, Sepharose, Acrylat, an Plastikoberflächen wie Polystyrol, Polyethylen, Polycarbonat, Polypropylen, Polysulfon, Polymethylmetacrylat, an Filtermaterialien oder Membranen wie Cellulose, Celluloseacetat, Nitrocellulose, PVDF, an magnetische oder nicht magnetische Partikel aus Glas, Latex, Plastik, Metall, Metalloxid.

Der Begriff "1-Schritt-Verfahren" wie hier verwendet bezeichnet ein Verfahren, bei dem spezifische Bindeproteine, beispielsweise ein erfindungsgemäßes Polypeptid, bereits vor der Zugabe der Probe entweder gerichtet oder ungerichtet an einen geeigneten Träger oder einer Oberfläche immobilisiert wurden. Nach Inkubation der immobilisierten Bindeproteine mit der Probe wird der Bakterien-Bindeprotein-Träger-Komplex von der Probe abgetrennt und evtl. anschließend gewaschen.

Der Begriff "2-Schritt-Verfahren" wie hier verwendet bedeutet ein Verfahren, bei dem nicht-immobilisierte spezifische Bindeproteine, beispielsweise ein erfindungsgemäßes Polypeptid, mit der Probe in Kontakt gebracht und inkubiert werden. Die gebildeten Bakterien-Bindeprotein-Komplexe werden anschließend mit einem geeigneten Träger oder einer Oberfläche in Kontakt gebracht, so dass die Bakterien-Bindeprotein-Komplexe über die Bindeproteine mit Hilfe des biotinylierten Affinitätstags an die Träger oder Oberflächen gebunden werden. Nachfolgend werden die Bakterien-Bindeprotein-Träger-Komplexe von der Probe abgetrennt und evtl. gewaschen. Die Bindeproteine sind mit einem Polypeptid oder einer chemischen Gruppe derart modifiziert, dass sie an einen Träger oder eine Oberfläche spezifisch binden, die mit dem jeweiligen Bindungspartner des Polypeptids oder der chemischen Gruppe versehen sind.

Der Begriff "Polypeptid", wie hierin verwendet, bezeichnet eine Polypeptidkette von mindestens 5 Aminosäuren.

Der Begriff "Bakterien-Polypeptid-Komplex" oder "Polypeptid-Bakterien-Komplex", wie hierin verwendet, bezeichnet einen Komplex, in dem Bakterien und das erfindungsgemäße Polypeptid (die erfindungsgemäßen Polypeptide) vorliegen.

Der Begriff "Träger-Polypeptid-Bakterien-Komplex", wie hierin verwendet, bezeichnet einen Komplex, in dem Bakterien, das erfindungsgemäße Polypeptid (die erfindungsgemäßen Polypeptide) sowie ein Träger(-material) vorliegen.

Die vorliegende Erfindung betrifft ein Polypeptid umfassend
i) eine enzymatisch nicht aktive, Zellwand-bindende Domäne eines Endolysins oder anderen Zellwand lysierenden Enzyms (CBD), und
ii) eine Sequenz gemäß SEQ ID NO: 1 oder ein Derivat davon,
   wobei das Polypeptid neben der Zellwand-bindenden Domäne keine weiteren Domänen eines Endolysins oder anderen Zellwand lysierenden Enzyms umfasst.

Insbesondere betrifft die vorliegende Erfindung ein erfindungsgemäßes Polypeptid, das biotinyliert ist. In einer besonderen Ausführungsform weist die Zellwand-bindende Domäne eines Endolysins oder anderen Zellwand lysierenden Enzyms (CBD) im erfindungsgemäßen Polypeptid die Fähigkeit auf, gram-positive Bakterien gezielt zu binden. Das erfindungsgemäße Polypeptid ist geeignet, um damit Bakterien zu binden, anzureichern, aus einer Probe zu entfernen, einzufangen und/oder nachzuweisen.

Daher betrifft die vorliegende Erfindung die Verwendung eines erfindungsgemäßen Polypeptids, um Bakterien zu binden, anzureichern, aus einer Probe zu entfernen, einzufangen und/oder nachzuweisen.

Dementsprechend betrifft die vorliegende Erfindung ferner ein Verfahren zum Binden, zur Anreicherung, zur Entfernung, zum Einfangen und/oder dem Nachweis von Bakterien aus einer Probe, umfassend die Schritte:
a) In Kontakt Bringen und/oder Inkubation einer Probe mit einem biotinylierten erfindungsgemäßen Polypeptid,
b) In Kontakt Bringen und/oder Inkubation der durch Schritt a) erhaltenen Polypeptid-Bakterien-Komplexe mit einem Träger, der mit einer Biotin-bindenden Substanz versehen ist.
c) Trennen des durch Schritt b) erhaltenen Träger- Polypeptid-Bakterien-Komplexe von der Probe,
d) gegebenenfalls Abwaschen unspezifisch anhaftender Bestandteile der Probe von dem Träger-Polypeptid-Bakterien-Komplex,
e) gegebenenfalls Abtrennen des Trägers vom Polypeptid-Bakterien Komplex, und
f) gegebenenfalls Nachweis der Bakterien.

In dem erfindungsgemäßen Verfahren muss die Dauer der Inkubation der Probe mit den entsprechenden erfindungsgemäßen Polypeptiden (Schritt a) bzw. die Inkubation der Bakterien-Polypeptid-Komplexe mit dem Trägermaterial (Schritt b) an die jeweilige Probe angepasst werden und kann in einer Ausführungsfonn zwischen wenigen Sekunden und etwa 24 h variieren. Generell ist Schritt a) des erfindungsgemäßen Verfahrens, in der die funktionalisierten, d.h. biotinylierten erfindungsgemäßen Polypeptide an die Bakterien binden, schneller als Schritt b), in dem die biotinylierten Polypeptid-Bakterien-Komplexe an dem biotinbindenden Träger immobilisiert werden. Geeignete Inkubationszeiten für Schritt a) des erfindungsgemäßen Verfahrens sind insbesondere etwa 0.1 min bis etwa 10 min, für Schritt b) insbesondere etwa 10 min bis etwa 60 min oder bei Bedarf auch über Nacht. Während es bei Schritt a) des erfindungsgemäßen Verfahrens in der Regel genügt, die zugegebenen erfindungsgemäßen Polypeptide mit der Probe gründlich zu mischen, kann es bei der Inkubation mit dem Trägermaterial (Schritt b) notwendig sein, z.B. nach Zugabe von Trägermaterial, das Probengefäß liegend zu rollieren, um eine möglichst effiziente Bindung an den Träger zu erreichen.

Bei sehr geringen Bakterienkonzentrationen im Ausgangsmaterial ist unter Umständen eine Vorinkubationsphase in einem geeigneten Nährmedium notwendig, um eine effiziente Anreicherung zu erreichen und somit eine geeignete Probe für das Verfahren gemäß der vorliegenden Erfindung zu erhalten. Proben, die feste Bestandteile beinhalten, wie z.B. Lebensmittelproben, können vor Verwendung in dem erfindungsgemäßen Verfahren homogenisiert und in geeigneten Lösungen wieder aufgenommen werden, bevor sie für das erfindungsgemäße Verfahren verwendet werden.

Im erfindungsgemäßen Verfahren wird ein mit einer Biotin-bindenden Substanz versehener Träger verwendet, d.h. der Träger wurde funktionalisiert. Die Biotin-bindende Substanz sollte in der Lage sein, Biotin mit hoher Affinität zu binden. Besonders geeignete Bindungspartner, d.h. Biotin-bindende Substanzen, auf der Oberfläche des Trägers sind beispielsweise Streptavidin, Avidin und biotinbindende Varianten davon wie z.B. monomeres Avidin, Avidin mit teilweise acetylierten Aminogruppen oder teilweise esterifizierten Carboxylgruppen davon. Hydrophile Oberflächen sind gegenüber hydrophoben Oberflächen bevorzugt, da sie in der Regel besser geeignet sind, Proteine zu binden und weniger zur Agglutination neigen.

Die Anreicherung der Bakterien erfolgt durch das Abtrennen der Bakterien von der restlichen Probe. Die Anreicherung der Bakterien kann in dem beschriebenen Verfahren beispielsweise auf magnetischer Basis, über chromatographische Verfahren oder im "Batch"-Verfahren erfolgen. Bevorzugt ist eine magnetische Anreicherung, da diese Methode im Vergleich zu anderen Verfahren sehr schnell ist, miniaturisiert und auch automatisiert werden kann. Aber auch das chromatographische Verfahren oder das "Batch"-Verfahren können verwendet werden, insbesondere, wenn das Verfahren nicht in erster Linie für einen nachfolgenden Bakteriennachweis genutzt wird, sondern darauf abzielt, z.B. eine größere Menge an Bakterien zu isolieren und mit den so isolierten Bakterien weiterzuarbeiten.

Bei der Anreicherung auf magnetischer Basis werden die Polypeptid-Bakterien-Komplexe mit geeigneten Magnetpartikeln als Träger in Schritt b) des erfindungsgemäßen Verfahrens inkubiert. Die Magnetpartikel können in bestimmten Ausführungsformen Durchmesser im Bereich von etwa 0,1 µm bis etwa 100 µm aufweisen. Bevorzugt sind jedoch kleinere Partikel im Durchmesser zwischen etwa 0,5 µm bis etwa 5 µm, besonders bevorzugt Partikel mit dem Durchmesser von etwa 0,8 µm bis etwa 2 µm, da kleinere Partikel weniger schnell sedimentieren und deshalb eine bessere Durchmischung ermöglichen und im Vergleich zu größeren Partikeln relativ eine größere Oberfläche aufweisen sowie hohe Wiederfindungsraten zeigen. Beispiele für geeignete Magnetpartikel sind MagPrep-Streptavidin Partikel (Merck), Streptavidin-Magnetic-Particles (Roche), Streptavidin-Beads (Dynal), Streptavidin gekoppelte Silika-Beads (MicroCoat), Streptavidin gekoppelte Polyvinylalkohol-Beads (PA-Streptavidin-Beads, Microcoat). Nach Schritt b) des erfindungsgemäßen Verfahrens wird der Träger-Polypeptid-Bakterien-Komplex durch Anlegen eines Magnetfeldes magnetisch von der Probe separiert. Geeignete Magnetseparatoren gibt es beispielsweise von den Firmen Ambion, Ademtech, Bilatec, BioLabs, Dynal, Polysciences und Promega.

Bei der Anreicherung mit Hilfe des Batch-Verfahrens wird zuerst die Bakterien haltige Probe mit dem erfindungsgemäßen Polypeptid inkubiert, anschließend wird Trägermaterial zugegeben, das geeignet ist, hochaffin Biotin zu binden, gemischt und nochmals gemeinsam inkubiert. Anschließend kann der Träger-Polypeptid-Bakterien-Komplex von der Probe abzentrifugiert, sedimentiert oder filtriert werden. Bevorzugt ist die Anreicherung über das Batch-Verfahren insbesondere bei sehr niedrigen Bakterienkonzentrationen.

Alternativ können die Polypeptid-Bakterien-Komplexe von der Probe abgetrennt werden und damit angereichert werden, indem sie auf eine Chromatographiesäule aufgetragen werden, die biotinbindendes Säulenmaterial enthält.

Eine Trennung der Polypeptid-Bakterien-Komplexe vom funktionalisierten Träger kann durch eine Verdrängung, wie z.B. mittels Zugabe einer entsprechenden Menge an Biotin, erfolgen oder durch die Einstellung Proteine stark denaturierender Bedingungen, wie z.B. etwa 8 M Guanidiniumchlorid oder etwa pH 1,5, bzw. durch die Zugabe von Biotinidase, die Biotin von Peptiden abspaltet. Es können auch nur die Bakterien von den biotinylierten erfindungsgemäßen Polypeptiden abgetrennt werden, indem Bedingungen gewählt werden, bei denen die erfindungsgemäßen Polypeptide nicht mehr an ihren Rezeptor auf der Bakterienoberfläche binden. Da für die Erfindung unterschiedliche erfindungsgemäße Polypeptide eingesetzt werden, müssen die genauen Bedingungen dafür im Einzelfall getestet werden. Dies kann beispielsweise dadurch erfolgen, dass man einen fluoreszierenden Marker einführt und im Fluoreszenzmikroskop überprüft, ob die zuvor gebundenen Bakterien noch fluoreszieren, da ihre Oberfläche von erfindungsgemäßen Polypeptiden bedeckt ist. Generell ist jedoch eine Veränderung der Ionenstärke hin zu sehr hoher oder sehr geringer Ionenstärke, eine Änderung des pH-Wertes hin zu stark sauer oder alkalisch, wie z.B. 50 mM Natriumphosphat und pH 11 für 5 min, die Zugabe von Detergenzien oder chemischen Denaturierungsmitteln wie Harnstoff oder Guanidiniumchlorid, oder eine Kombination aus den angeführten Möglichkeiten geeignet, um die CBD-Bakterienbindung zu unterbinden, da es sich hierbei um eine spezifische Protein-Protein-Interaktion handelt. Für viele Anwendungen wie z.B. bei anschließendem Ausplattieren und Zählen der von gebundenen Bakterien abstammenden Kolonien ist jedoch eine Abtrennung der Magnetpartikel von den Bakterien gar nicht notwendig.

Die vorliegende Erfindung betrifft ferner Verfahren zum Nachweis von Bakterien in einer Probe. Der Bakterien-Nachweis umfasst im Anschluss an die oben beschriebenen Verfahrensschritte zur Anreicherung weitere Schritte. Abhängig von der Art der nachzuweisenden Bakterien ist dem Fachmann eine Reihe von Techniken bekannt, die zu dem gewünschten Ergebnis führen. Eine Auswahl geeigneter Nachweisverfahren ist nachfolgend genannt. Beispielsweise können die Bakterien im Komplex mit dem Träger und dem biotinylierten erfindungsgemäßen Polypeptid oder nach Ablösung vom Trägermaterial mittels selektiven Wachstumsbedingungen z.B. Ausplattieren und Inkubieren auf Selektivmedienplatten nachgewiesen werden. Weiterhin ist ein Nachweis der Bakterien mittels nukleinsäurebasierten Methoden möglich, d.h. Nachweis der Nukleinsäuren der Bakterien, z.B. PCR, RT-PCR, PCR-RFLP, rep-PCR-fingerprinting, NASBA, DNA-Hybridierungsmethoden beispielsweise für spezielle Toxine oder andere Pathogenizitätsfaktoren, Multilocus Sequenz Typisierung (MLST), rRNA-Vergleiche. Ferner ist ein Nachweis der Bakterien-Zellwand bzw. ihrer Komponenten z.B. über Zellbindedomänen von Endolysinen oder Antikörper oder per FTIR, bzw. der Nachweis von Bakterien-Bestandteilen, z.B. Proteinen über ELISA oder Enzymen über ihre Aktivität oder Multilocus Enzymelektrophorese (MEE), möglich. Der Bakteriennachweis ist auch über in den Bakterien enthaltenes ATP z.B. in einem Biolumineszenzassay mittels Nachweis über einen Bakterien-spezifischen Bakteriophagen, z.B. für Listerien A511-luxA, möglich (siehe US 5,824,468). Ferner kann der Nachweis der Bakterien im Träger-Polypeptid-Bakterien-Komplex oder nach Ablösung vom Trägermaterial mittels einer weiteren spezifischen CBD erfolgen, die mit einem Marker gekoppelt ist. Eine Reihe Beispiele hierfür sind in EP1147419 aufgezeigt. Möglich ist auch ein konventioneller Nachweis aus einer Kombination mikrobiologischer, morphologischer und/oder biochemischer Nachweismethoden.

Vorzugsweise wird der Nachweis von Bakterien-Bestandteilen, z.B. von Proteinen mittels ELISA oder ähnlichen Techniken (z.B. VIDAS) durchgeführt. Für die Durchführung dieser Methoden ist es notwendig, die Bakterien vor dem eigentlichen Nachweis aufzubrechen. Dies kann beispielsweise mit einem Lyseprotein wie Lysozym oder einem Bakterien-spezifischen Endolysin durchgeführt werden. Lyseproteine können beipsielsweise aus folgender Gruppe ausgewählt werden (Quellenangaben in Klammern entweder als Datenbankzugangscodes für die NCBI-Datenbank (Zahlen-Buchstabenkombinationen) oder als Publikationszitate):
- Ply511 (Q38653), Ply500 (Q37979), Ply118 (Q37976), PlyPSA (1XOV_A), dem Autolysin von Stamm EGDe (NP_466213) für *Listeria,*
- PlyL (1YBO_A, B und C), PlyG (YP_891193), PlyPH (Yoong et al., J.Bac. 2006, 188, 2711-2714), PlyB (2NW0_A und B) für *Bacillus anthracis,*
- PlyBa (CAA72266), Ply21 (CAA72267), Ply12 (CAA72264) für *Bacillus cereus,*
- Ply3626 (WO 03/066845) und die Lysine aus Cl. perfringens Stamm 13 (BAB81921) und Stamm SM101 (YP_699489) für *Clostridium perfringens*
- ΦP1 Lysin (EP1300082) für *Clostridium* tyrobutyricum,
- PlyV12 (NP_049942) für *Enterococcus,*
- PlyC (NP_852017), PlyGBS (AAR99416), Cpl-1 (P15057), Cpl-7 (P19385), Cpl-9 (P19386), Pal Amidase aus dem Phage Dp1 (P19386), B30 Endolysin (AAN28166) und LytA (CAJ34420) für *Streptococcus,*
- Twort Amidase (CAA69021), Staphylococcus Phage P68 Amidase (NP_817332), LysK (O'Flaherty et al., J. Bac., 2005, 187,_7161-7164), ΦSA2usa Lysin (YP_494080), Phill Amidase (NP_803306) und Zellwandhydrolase (NP_803302) oder Phi12 Endolysin (NP_803355), sowie die Autolysine Atl (BAA04185) aus *Staphylococcus aureus ,* AtlE (CAI59555) aus *Staphylococcus epidermidis,* ALE-1 (BAA13069) aus *Staphylococcus capitis* sowie die aus dem *Staphylococcus aureus* Stamm PS47 stammende Peptidoglykanhydrolase (AAA26662) oder Lysostaphin (AAB53783) aus *Staphylococcus simulans* für *Staphylococcus.*

Unterstützt werden kann die Zelllyse durch verschiedene weitere Zusätze wie die Zugabe von Proteasen wie z.B. Proteinase K und die Anwendung von Hitze, vorzugsweise 5 min bei etwa 56 °C, danach 5 min bei etwa 94 °C, oder wie den Zusatz von Detergenzien, vorzugsweise Triton, SDS, Tween, Na-Desoxycholat oder Lösungsmitteln wie z.B. DMSO, Isopropanol, Ethanol, Butanol, Chloroform.

Die erfindungsgemäßen Polypeptide umfassen Polypeptiddomänen/-sequenzen von Endolysinen bzw. Autolysinen, die so genannten CBDs, wobei die erfindungsgemäßen Polypeptide keine enzymatisch aktiven Zellwand hydrolysierenden Bereiche mehr aufweisen. Das Fehlen der Enzymaktivität ist wichtig, damit die Bakterien im Komplex mit dem Träger funktionell separiert werden können. Eine Lyse der gebundenen Bakterien und damit eine Freisetzung der Zellinhalte erfolgt vorzugsweise nur gezielt nach der Separation, wenn dies z.B. für die anschließende Nachweisreaktion erforderlich ist. Daher umfassen in einer besonderen Ausführungsform die erfindungsgemäßen Polypeptide neben der Zellwand-bindenden Domäne keine weiteren Domänen eines Endolysins, insbesondere keine enzymatisch aktive Domäne (EAD) eines Endolysins, und in bestimmten Ausführungsformen auch keine weiteren Sequenzen eines Endolysins. Es kann jedoch auch eine geringe hydrolytische Restaktivität der eingesetzten Polypeptidfragmente unter Umständen tolerierbar sein. Dies hängt jedoch von der jeweiligen Anwendung ab, d.h. es muss überprüft werden, inwieweit sich die Geschwindigkeit der Zellhydrolyse mit der Gesamtdauer der Anwendung vereinbaren lässt, so dass genügend intakte Zellen eingefangen werden. Wie sich eine potentielle Restaktivität der CBDs in einem Hydrolyseassay nachweisen lässt, ist beispielsweise in Loessner et al., 1996, Appl. Environ. Microbiol. 62, 3057-3060 beschrieben.

In einer besonderen Ausfübrungsform weist das erfindungsgemäße Polypeptid Derivate von SEQ ID NO:1 auf, gemäß SEQ ID NOs: 2 bis 18, die als beispielhafte Abwandlung von SEQ ID NO: 1 folgendes aufweisen:
i) im Vergleich zu SEQ ID NO: 1 an Position 60 Asp statt Glu,
ii) am C-Terminus ein zusätzliches Val oder Val-Asp, und/oder
iii) am N-Terminus ein zusätzliches M oder MVGA.

Die oben angegeben Derivate von SEQ ID NO:1 haben sich als vorteilhaft bei der Verwendung in den erfindungsgemäßen Verfahren erwiesen.

Die modulare Organisation von Endolysinen in C-terminale Domänen (CBDs), die für die spezifische Zellbindung zuständig sind und N-terminale Domänen (EADs), die das enzymatisch aktive Zentrum beinhalten, wurde bereits 1990 von Garcia et al. beschrieben (Garcia et al., 1990, Gene, 86, 81-88). Das Konzept der CBDs wurde in Loessner et al., 2002, Mol. Microbiol., 44, 335-349 und Loessner, 2005, Curr. Opin. Microbiol, 8, 480-487 weiter fortgeführt. Im Stand der Technik sind bereits eine Vielzahl von CBDs beschrieben. Häufig ist die enzymatisch aktive Domäne (EAD) am N-Terminus lokalisiert und die CBD am C-Terminus - es gibt jedoch auch Ausnahmen (z. B. Garcia et al., 1988, Proc. Natl. Acad. Sci. USA, 85, 914-918; Loessner, 2005, Curr. Op. Microbiol., 8, 480-487). Die EAD ist in der Regel gut definiert und über Sequenz- und Homologievergleiche mit anderen Hydrolasen, wie z.B. Amidasen, Endopeptidasen, Glycosidasen, Transglycosylasen, Muramidasen, relativ leicht über im Stand der Technik bekannte Sequenzanalyseprogramme und entsprechende Datenbanken mit konservierten Sequenzmotiven zu finden und einzugrenzen (z.B. CDD (Marchler-Bauer et al., 2005; Nucleic Acids Research, 33, D192-D196); Pfam (Finn et al., 2006, Nucleic Acids Research 34, D247-D251) or SMART (Schultz et al., 1998, Proc. Natl. Acad. Sci. USA 95, 5857-5864, Letunic et al., 2006, Nucleic Acids Res 34, D257-D260). Bei der Bestimmnung des CBD-Anteils von Endolysinen finden sich in vielen Fällen ebenfalls bekannte Motive für Zellbindedomänen, die einen guten Anhaltspunkt für die Gewinnung der CBD-Sequenz für die erfindungsgemäßen Polypeptide bilden. Bei der Gruppe der Endolysine, die an Streptococcen binden, ist die CBD relativ einfach zu finden, da meist etwa 20 Aminosäuren lange Cholinbindemotive mit konservierten aromatischen Resten (CW_binding_1, pfam01473) auftreten, die häufig in mehreren Wiederholungen vorkommen (siehe Garcia et al., 1990, Gene, 86, 81-88). Auch die etwa 40 Aminosäuren lange LysM Domäne (pfam01476) findet sich teilweise in CBDs. Es handelt sich hierbei um ein weit verbreitetes Peptidoglykanbindemodul mit konservierter Sekundärstruktur (Bateman & Bycroft, 2000, J. Mol. Biol., 299, 1113-1119). SH3b-Domänen bzw. SH3_3, SH3_4 oder SH3_5 Domänen (smart00287, pfam08239, pfam06347, pfam08460), die prokaryontischen Gegenstücke zu den eukaryontischen und viralen Scr Homologiedomänen, SH3, (Ponting et al, 1999, J. Mol. Biol., 289, 729-745) finden sich ebenfalls oft als Zellbindemotive in CBDs, vor allem bei Staphylococcen und Enterococcen. Aus 3 Helices aufgebaut ist die Peptidoglykanbindedomäne (PG_binding_1, pfam01471; PG binding 2, pfam08823), die häufig auch N-terminal von der EAD zu finden ist. Manchmal findet sich jedoch keinerlei unmittelbare Verwandtschaft für die CBD-Anteile zu anderen dennoch verwandten Bakteriophagenendolysine oder auch anderen kohlenhydratbindender Proteinen und es lassen sich auch kaum einheitlichen Sequenzmotive oder Strukturmodule definieren, die eine CBD kennzeichnen. Die Verwandtschaft kann in solchen Fällen über die EAD bestimmt werden. Als Basis für das erfindungsgemäße Polypeptid dient in diesen Fällen neben den aus dem Stand der Technik bekannten CBDs der Anteil eines Endolysins, der nicht durch die EAD eingenommen wird. Der Rest dieses Endolysins (d.h. Endolysin minus EAD) kann direkt als CBD verstanden und eingesetzt werden, sofern er Zellbindefunktion aufweist. In einigen Ausführungsformen der vorliegenden Erfindung kann es jedoch sinnvoll sein, kürzere Fragmente einzusetzen, (z. B. weil diese eine höhere Stabilität aufweisen) soweit diese auch noch die Zellbindefunktion aufweisen. Der funktionelle Test für eine CBD ist der Nachweis der Zellbindung an die entsprechenden Bakterien. Verschiedene dafür geeignete beispielhafte Assays sind in den Experimenten und Abbildungen beschrieben. Neben einer effizienten Zellbindung sind Expressionsrate, Löslichkeit, Stabilität und einfache Reinigung weitere Eigenschaften, die bei der Definition des als CBD fungierenden Polypeptidanteils berücksichtigt werden sollten. Dem Fachmann sind Methoden aus dem Stand der Technik bekannt, wie er diese Eigenschaften testen kann. Einige Beispiele hierfür sind auch weiter unten in den Experimenten beschrieben. Gezielt geplante CBD-Anteile orientieren sich beispielsweise an strukturellen Vorgaben, die der Fachmann aufgrund von Sekundärstrukturvorhersagen, potentiellen Domänenlinkern und 3D-Modellen beurteilen kann. Geeignete beispielhafte Methoden dazu sind beispielsweise in folgenden Veröffentlichungen beschrieben: Garnier et al., 1996, Methods in Enzymology 266, 540-553; Miyazaki et al., 2002, J. Struct. Funct. Genomics, 15, 37-51; Altschul et al., 1997, Nucleic Acids Res. 17, 3389-3402; Schwede et al., 2003, Nucleic Acids Research 31, 3381-3385. Lund et al, CPHmodels 2.0: X3M a Computer Program to Extract 3D Models. Abstract at the CASP5 conferenceA102, 2002.
Prinzipiell können alle aus dem Stand der Technik bekannten CBDs und alle nach dem oben beschriebenen Verfahren aus Endolysinen abgeleiteten CBDs für die erfindungsgemäßen Polypeptide verwendet und im erfindungsgemäßen Verfahren eingesetzt werden.

In einer besonderen Ausführungsform der Erfindung ist die Zellwand-bindende Domäne des erfindungsgemäßen Polypeptids ausgewählt aus der Gruppe der Zellwand-bindende Domänen der folgenden Endolysine bzw. anderen Zellwand lysierenden Enzyms bestehend aus Ply511 (Q38653), Ply 500 (Q37979), Ply 118 (Q37976), PlyPSA (1XOV_A), EGDe (NP_466213), PLyL (1YB0_A, B und C), PlyG (YP_891193), PlyPH (Yoong et al., J.Bac. 2006, 188, 2711-2714), PlyB (2NW0_A und B), PlyBa (CAA72266), Ply21 (CAA72267), Ply12 (CAA72264), der *Enterococcus faecalis* V583 Prophagen Endolysine, Ply3626 (WO 03/066845), Lysine aus C1. perfringens Stamm 13 (BAB81921) und Stamm SM101 (YP_699489), ΦP1 Lysin (EP1300082), PlyV12 (NP_049942), PlyC (NP_852017), PlyGBS (AAR99416), Cpl-1 (P15057), Cpl-7 (P19385), Cpl-9 (P19386), Pal Amidase (P19386), Twort Amidase (CAA69021), *S. aureus* phage PVL amidase (UniProt 080064), P68 lys16 (NP_817332), ΦSA2usa endolysin (YP_494080), Phill (NP_803306) und Phi12 Endolysin (NP_803355), Zellwandhydrolyse des Staphylococcus aureus Phagen Phi 11 (NP_803302), Phage B30 Endolysin (AAN28166), Phage 168 Endolysin (M J Loessner et al., J Bacteriol. 1997 May; 179(9): 2845-2851), LysK (O'Flaherty et al., J. Bac., 2005, 187, 7161-7164), *S*. *simulans* Lysostaphin (AAB53783), *S*. *capitis* ALE-1 Endopeptidase (BAA13069), Phage PhiNIH1.1 Zellwandhydrolase (NP_438163), LytM (AAB62278), Atl (BAA04185), LytA aus Streptococcus pneumoniae (CAJ34420), aus *Staphylococcus aureus* Stamm PS47 stammende Peptidoglykanhydrolase (AAA26662), Enterolysin A aus Enterococcus faecalis (Q9F8B0), Ami Autolysin von *L. monocytogenes* (Milohanic et al.; Infection and Immunity, August 2004, p. 4401-4409, Vol. 72, No. 8)), Lactobacillus Lysine, z.B. Lysin des Phagen A2 (AJ251788.2 oder Q9MCC8), Phage PL-1 Amidase (Q9MCC6) (beide L. casei) etc.

Als besonders geeignet stellten sich zur Bindung von Listerien Fragmente des Endolysine Ply511, zur Bindung von Bacillen Fragmente der Endolysine PlyBa, Ply21 und Ply12, zur Bindung von Clostridien Fragmente von Ply2636, zur Bindung von Staphylokokken Fragmente des ΦSA2usa Endolysins, des *Staphylococcus* Bacteriocins Lysostaphin, des Lysostaphin ähnlichen ALE-1, der *Staphylococcus* Eigenisolate plyOpf, plyPitti20, plyPitti26 und ähnlicher Proteine homologer Prophagen und zur Bindung von Enterokokken Fragmente aus Endolysinen des Prophagen aus *Enterococcus faecalis* V583 sowie CBDEF0355 und CBDEF1293 heraus.

Beispiele für CBD-Sequenzen, die in den erfindungsgemäßen Polypeptiden auftreten können, sind:
SEQ ID NO: 19: CBD21;
SEQ ID NO: 20: CBDBA
SEQ IN NO: 21: CBDUSA
SEQ ID NO: 22: Ply511 Version 1
SEQ ID NO: 23: Ply511 Version 2
SEQ ID NO: 24: Ply511 Version 3
SEQ ID NO: 25: Ply3626 Version 1
SEQ ID NO: 26: Ply3626 Version 2
SEQ ID NO:27: CBDPitti20
SEQ ID NO:28: CBDPitti26
SEQ ID NO:29: CBDLS
SEQ ID NO:30: CBDALE-1
SEQ ID NO:31: CBDOpf
SEQ ID NO:32: CBDEF0355
SEQ ID NO:33: CBDEF1293

In einer besonderen Ausführungsform umfasst das erfindungsgemäße Polypeptid neben der Zellwand-bindenden Domäne keine weiteren Sequenzen eines Endolysins. Vorzugsweise besteht das erfindungsgemäße Polypeptid dann nur aus einer CBD und einer zusätzlichen Polypeptidsequenz gemäß SEQ ID NO: 1 oder Derivaten davon, gegebenenfalls gekoppelt durch einen Linker oder Abstandshalter.

Der Fachmann besitzt Kenntnisse über die Sequenzen und Strukturen von Domänenlinkersequenzen bzw. über deren Vorhersage (z.B. George und Heringa, 2003, Protein Eng. 15, 871-879; Bae et al., 2005; Bioinformatics, 21, 2264-2270). Domänenlinkersequenzen sind häufig durch einen relativ hohen Anteil hydrophiler Aminosäuren gekennzeichnet, da sie in der Regel wenig strukturiert und lösungsmittelexponiert sind, wie beispielsweise die kurze Linkersequenz AAKNPN (SEQ ID No 37) aus dem Listeria Endolysin PlyPSA (Komdörfer et al., 2006, J. Mol. Biol., 364, 678-689). Auch Polyglycin-Linker werden traditionell verwendet, sind jedoch häufig proteaseempfindlich. Eine spezielle Form der hydrophilen, unstrukturierten Linker sind Prolin und Threonin reiche Sequenzen, die auch als natürliche Linker vorkommen, wie z.B. TPTPPNPGPKNFTT aus Enterolysin A (SEQ ID No 36). Prolin und Threonin reiche Linkersequenzen lassen sich vereinfacht durch das Konsensumotiv (PT)ₓP oder (PT)ₓT beschreiben, wobei x eine ganze Zahl im Bereich 1 bis 10 darstellt. Croux et al. (1993, Molec. Microbiol., 9, 1019-1025) beschreiben sogenannte "junction zones" zwischen den N- und C-terminalen Domänen, also den EADs und CBDs der Endolysine. Diese meist relativ kurzen Bereiche sind "natürliche Linkersequenzen" und ebenfalls geeignet, um die CBD-Module mit Hilfe geeigneter Schnittstellen mit den JS-Tags rekombinant zu einem erfindungsgemäßen Polypeptid zu verbinden. Besonders geeignete Beispiele für spezifische Linker sind z.B. in SEQ ID NOs: 34 bis 38, angegeben.

Beispielsweise kann sich die Sequenz eines erfindungsgemäßen Polypeptids wie folgt zusammensetzen:
i) eine Sequenz für einen JS-Tag ausgewählt aus SEQ ID NO: 1-18,
   ii) eine Sequenz für eine CBD ausgewählt aus SEQ ID NO: 19-33,
   iii) eine Linker-Sequenz ausgewählt aus SEQ ID NO: 34 bis 38
      , und gegebenenfalls
   iv) als Abstandshalter eine Sequenz für GFP, GST oder MBP.

Einige beispielhafte Sequenzen für erfindungsgemäße Polypeptide sind in SEQ ID NO: 39 bis 53 aufgeführt.
Ferner betrifft die Erfindung eine nicht enzymatisch aktive Zellwand-Binde-Domäne, nämlich die CBD von ply21. Überraschenderweise weist die hydrolytisch nicht aktive CBD21 in der Bakterienbindung ein Wirtsspektrum auf, das neben fast allen Bakterien aus der Bacillus-Gruppe (bis auf *B.* polymyxa und *B. sphaericus*) auch weitere Vertreter aus wichtigen Gruppen gram-positiver Bakterien wie Staphylokokken, Enterokokken, Streptokokken und sogar Listerien umfasst. Da CBDs normalerweise ein relativ enges Wirtsspektrum aufweisen (Loessner2005, Curr. Opin. Microbiol, 8, 480-487), ist diese Eigenschaft einer breiten Wirtsspezifität für eine CBD sehr ungewöhlich. Daher kann die CBD21 zusätzlich zur Bindung von allen getesteten Bakterien aus der *Bacillus cereus* Gruppe die Aufgabe lösen, gram-positive Bakterien ganz allgemein anzureichern, besonders jene, welche aus Gruppen stammen, in denen viele pathogene Keime zu finden sind wie z.B. Staphylokokken, Streptokokken, Enterokokken, Mikrokokken, Bazillen und Listerien. Daher betrifft die vorliegende Erfindung in einem weiteren Aspekt ein Polypeptid, welches die Sequenz wie in SEQ ID NO: 19 gezeigt, umfasst, aber neben dieser Zellwand-bindenden Domäne keine weiteren Domäne eines Endolysins umfasst. Vorzugsweise umfasst das Polypeptid keine vollständige enzymatisch aktive Domäne eines Endolysins. Noch bevorzugter umfasst das Polypeptid, welches die Sequenz wie in SEQ ID NO: 19 gezeigt umfasst, keine weiteren Sequenzen eines Endolysins.

Aufgrund der breiten Anwendbarkeit der CBD21 betrifft die vorliegende Erfindung auch die Verwendung der erfindungsgemäßen CBD21, die eine Sequenz gemäß SEQ ID NO: 19 umfasst, aber neben dieser Zellwand-bindenden Domäne keine weiteren Domäne eines Endolysins umfasst, um Bakterien ausgewählt aus der Gruppe bestehend aus Staphylococcus, Streptococcus, Enterococcus, Micrococcus, Bacillus und/oder Listeria (siehe Tabelle 3) zu binden, anzureichern, aus einer Probe zu entfernen, einzufangen und/oder nachzuweisen..

Der Polypeptidanteil der erfindungsgemäßen Polypeptide, der eine geeignete CBD definiert, kann zusätzlich mit einen Polypeptidanteil verknüpft werden, der als Abstandshalter (Spacer) oder gegebenenfalls gleichzeitig als Marker dient. Da CBDs Bereiche aus größeren Proteinen, den Endolysinen, darstellen, sind sie in der Regel mit etwa 100 bis 300 Aminosäuren oder für bestimmte Zellbindemotive noch weniger, relativ klein. Deshalb kann es in einer bevorzugten Ausführungsform sinnvoll sein, zwischen der CBD-Domäne und der Gruppe, die für die Immobilisierung an den Träger zuständig ist, einen Abstandshalter einzuführen. Dies kann verhindern, dass die CBD durch die Immobilisierung denaturiert wird und damit ihre Bindungsfähigkeit an die Zellen verliert. Für die Bindung des Polypeptid-Bakterien-Komplexes im 2-Schritt-Verfahren kann wichtig sein, dass die Gruppen, die für die Immobilisierung an die Oberflächen verantwortlich sind, besser zugänglich werden, wenn sie nicht direkt an die CBD gekoppelt sind. Der Spacer ist vorzugsweise ein gut definiertes, gut exprimierbares, stabiles Proteinmodul, das möglichst wenig mit anderen Proteinen und Oberflächen wechselwirkt (z.B. GFP (green fluorescent Protein), MBP (Maltose Bindungsprotein), GST (Glutathion S-Transferase)). Ein besonders geeignetes Beispiel ist GFP und Varianten davon. Da GFP stark fluoresziert, ist es außerdem geeignet als Marker. Damit kann das erfindungsgemäße Polypeptid z.B. während des Verfahrens verfolgt werden. Im Funktionstest, d.h. Bindungstest kann eine Bindung der CBDs bzw. der erfindungsgemäßen Polypeptide an die Bakterien sowie eine Bindung an den Träger dann leicht nachgewiesen werden. Als Marker können auch weitere Modifikationen dienen, wie sie z.B. in EP1147419 vorgeschlagen werden.

Damit die CBD zum erfindungsgemäßen Polypeptid wird, muss sie mit einem Tag, dem so genannten JS-Tag in einem Fusionsprotein vorliegen. Die extrem gute Bindung zwischen Biotin und seinen Bindungspartnern Streptavidin bzw. Avidin (10⁻¹⁵ M; Gonzales et al., 1997, J. Biol. Chem., 272, 11288-11294) ist vorteilhaft für das Funktionieren des oben beschriebenen 2-Schritt-Verfahrens, das sich gegenüber dem aus dem Stand der Technik (z.B. EP1147419) bekannten 1-Schritt-Verfahren als noch besser erweist. Andere Tags, die ebenfalls geeignet sind, um Proteine an funktionalisierte Oberflächen zu binden sind z.B. der His-Tag oder der Strep-Tag (His-Tag 10⁻⁶ - 10⁻⁸ M; Nieba et al., 1997, Anal. Biochem., 252, 217-228; Strep-Tag ∼ 10⁻⁶ M, Voss & Skerra, 1997, Protein Eng., 10, 975-982). Die Bindung des Bakterien-Polypeptid-Komplexes an den Träger ist mit dem 2-Schritt-Verfahren effizienter, was sich sowohl in der Bindezeit, in einem geringeren möglichen Verlust von Bakterien unter schwierigen Bedingungen wie z.B. in Lebensmittelproben als auch in einer größeren Sensitivität bemerkbar machen kann. Betrachtet man eine Auswahl von möglichen biotinylierten Kopplungsgruppen, erweist sich der JS-Tag als bevorzugt. Eine chemische Biotinylierung führt zum einen nicht zu einer definierten Biotinylierung an einer bestimmten Stelle, mit der eine gerichtete Immobilisierung der CBDs auf dem Träger möglich wäre, was für die Funktionalität der Bindeproteine wünschenswert ist, zum anderen werden die Proteine dadurch oft inaktiviert. Dies kann insbesondere bei den recht kleinen Proteindomänen der CBDs zutreffen. Der Avi-Tag, der so etwas wie eine Minimalsequenz darstellt, die in Fusionsproteinen *in vivo* noch biotinyliert wird, erwies sich ebenfalls gegenüber dem JS-Tag als weniger geeignet, da hier höhere Proteinmengen eingesetzt werden mussten, um zu einer effektiven Bindung der Batterien an Magnetbeads zu gelangen. Die in US 5,252,466 zur Fusion mit Proteinen vorgeschlagenen Biotinylierungsdomänen sind im Vergleich zu den Avi-Tags relativ groß. So umfasst beispielsweise die Biotinylierungsdomäne der Klebsiella pneumoniae Oxalacetatdecarboxylase 595 Aminosäuren, was sowohl hinsichtlich der Expressionsausbeuten unvorteilhaft ist als auch insofern, dass der große Fusionsanteil relativ Protease empfindlich und somit wenig stabil ist.

In Kombination mit CBDs erwies sich der JS-Tag und Derivate davon als sehr guter Biotinylierungstag, um Bakterien aus Proben zu binden, anzureichern, zu entfernen, einzufangen und nachzuweisen. Es handelt sich hierbei um Abschnitte aus der α-Untereinheit der *Klebsiella pneumoniae* Oxalacetetdecarboxylase und Derivate davon, die das Konsensusmotiv (MKM) zur *in vivo* Biotinylierung enthalten. Gegenüber der kompletten Biotinylierungsdomäne wurde das Polypeptid stabiler, z.B. gegenüber Proteolyse, und ist als Affinitätstag z.B. bei Klonierung, Expression und Reinigung leichter zu handhaben. Die Minimalsequenz für den JS-Tag beträgt 66 Aminosäuren, was den Aminosäuren 529 bis 594 der *Klebsiella pneumoniae* Oxalacetatdecarboxylase entspricht plus Methionin als Start. Besonders geeignet sind Sequenzen die unter SEQ ID NO:1 bis 18 beschrieben sind. In Cronan, 1990, J. Biol. Chem., 265, 10327-10333 wird hervorgehoben, dass konservierte Prolin- und Alanin-reiche Regionen N-terminal von dem MKM-Motiv gelegen, eine wichtige strukturelle Funktion für die Biotinylierung des Lysins durch die Biotinligase übernehmen sollen. Bei der α-Untereinheit der *Klebsiella pneumoniae* Oxalacetatdecarboxylase ist dieser Bereich mit einer 22 Aminosäuren langen Region aus P und A sogar besonders ausgeprägt. Es stellte sich jedoch heraus, dass dieser Bereich für die Biotinylierung in dem hier beschriebenen System nicht notwendig ist, da die oben beschriebene Minimalsequenz diesen Bereich nicht enthält und dennoch sehr effizient biotinyliert wird. Zusätzlich zu den Aminosäuren 529 bis 594 der *Klebsiella pneumoniae* Oxalacetatdecarboxylase stellten sich sehr kurze Peptide (MVGA) als sehr gute N-terminale Startsequenz heraus (siehe SEQ ID NOs: 8-10 und 16-18).

Die Fusion zwischen dem JS-Tag und der CBD bzw. einem zusätzlich dazwischen eingeführten Spacermodul kann sowohl N-terminal als auch C-terminal von der CBD erfolgen. Bei den erfindungsgemäßen Polypeptiden ist die N-terminale Fusion bevorzugt, da der CBD-Anteil im Endolysin normalerweise C-terminal gelegen ist und der JS-Tag (plus gegebenenfalls Spacermodul) strukturell die fehlende EAD des Endolysins ersetzen kann. Da die Biotinylierungsdomänen in Proteinen, die *in vivo* biotinyliert werden, fast ausschliesslich am C-Terminus zu finden sind, ist es nicht nahe liegend, dass diese auch N-terminal eingesetzt gut funktionieren. In Cronan, 1990, J. Biol. Chem., 265, 10327-10333 wurden aus diesem Grund nur C-terminale Fusionen von Proteinen mit einer Biotinylierungsdomäne der 1.3 S Untereinheit der *Propionibacterium shermanii* Transcarboxylase verwendet. Der JS-Tag kann, falls kein Spacermolekül eingesetzt wird, über einen Linker (Ausführungsformen siehe oben) mit der CBD verbunden werden oder auch ohne Linker, so dass lediglich 1 bis 3 Aminosäuren eingeführt werden, um eine Restriktionsschnittstelle für die Klonierung zu erhalten. Als beispielhaftes geeignetes Linkerpeptid stellte sich die Sequenz AGAGAGAGS oder AGAGAGAGSEL (SEQ ID NO:34 oder 35) heraus. Es können jedoch auch andere Linkersequenzen aus Proteinen, bei denen die Struktur bekannt ist, oder Linkersequenzen für relativ unstrukturierte Peptide (z. B. Prolin und Threonin reiche Linker wie (PT)₃T(PT)₃T(PT)₃), verwendet werden. Ein Beispiele für einen PT-reichen Linker ist TPTPPNPGPKNFTT (SEQ ID NO:36). Ein Beispiel für einen (kurzen) hydrophile Linker ist AAKNPN (SEQ ID NO:37). Ein weiteres Beispiel für einen Linker, der in der vorliegenden Erfindung Verwendung finden kann, ist AGAGAGAEL (SEQ ID NO:38).

Das erfindungsgemäße Polypeptid für die Verwendung in den erfindungsgemäßen Verfahren kann dadurch biotinyliert werden, dass es *in vitro* unter dem Fachmann bekannten Bedingungen mit Hilfe einer Biotinligase biotinyliert wird. Überraschenderweise stellte sich aber auch heraus, dass im Gegensatz zu der in US5252466 beschriebenen Erfindung, bei der Herstellung der erfindungsgemäßen biotinylierten Polypeptide in Bakterienzellen eine Koexpression des biotinylierten Fusionsproteins mit der Biotinligase (*birA*) nicht notwendig ist, da die Biotinylierung auch in Abwesenheit von externer Biotinligase funktioniert. Erstaunlicherweise ist in Vollmedium wie z.B. LB nicht einmal der Zusatz von Biotin zum Medium notwendig. Fusionsproteine aus JS-Tag und CBD werden in kommerziell erhältlichen *E*. *coli* Expressionsstämmen, wie z.B. BL21 (DE3), HMS174 (DE3), JM83, ohne zusätzliche Koexpression von *birA* und sogar ohne Zusatz von Biotin effizient biotinyliert. Im Gegensatz zu der in US 5,252,466 vorgeschlagenen Verwendung des Biotinylierungstags als Mittel zur leichteren Reinigung von Proteinen, müssen die hier beschriebenen Fusionen aus JS-Tag und CBD nicht über Affinitätssäulen für Biotin gereinigt werden, sondern können auch auf auf konventionelle Art, wie z.B. über Kationen- oder Anionenaustauschchromatographie, hydrophobe Chromatographie, fraktionierte Ammoniumsulfatfällung, etc., gereinigt werden. Dies hat zum einen den Vorteil, dass das entsprechende Affinitätsmaterial für Biotin sehr teuer ist und oft nur eine geringe Kapazität besitzt, wie z.B. Chromatographiematerial, das Streptavidin oder Streptactin gekoppelt trägt, zum anderen sind die Fusionsproteine nur recht schwer wieder vom Affinitätsmaterial zu lösen, weil die Bindung zwischen Biotin und seinem Bindungspartner sehr effizient ist. Dies führt zu Problemen bei der beschriebenen Reinigungsmethode. So wird das Zielprotein verzögert von der Säule eluiert ("schmiert") und unter Umständen bei der Elution denaturiert.

Die Stabilität der erfindungsgemäßen Konstrukte hängt zu einem gewissen Grad von den speziellen Eigenschaften der verwendeten CBDs ab. Es lassen sich sowohl die Fusionskonstrukte aus JS-Tag, CBD und gegebenenfalls Linker, Spacer bzw. Marker als auch die funktionalisierten Träger wie z.B. Magnetbeads gut über längere Zeit lagern ohne dass ihre Bindungsfähigkeit verloren geht. Eine Lagerung ist in einem Temperaturbereich von ca. - 20 °C bis etwa 37 °C möglich. Bevorzugt ist eine Lagerung bei Temperaturen von etwa - 20 °C bis etwa 10 °C. In der Regel sollte die Lagerung bei annähernd neutralen pH-Werten (pH 6-7) erfolgen, eine Lagerung bei pH-Werten bis hin zu pH 10 ist aber beispielsweise ebenfalls möglich, wenn der CBD-Anteil dies zulässt. Geeignete Puffersysteme für die Lagerung sind z.B. 100 mM Natriumphosphatpuffer, pH 6 bis pH 10, 2 mM EDTA oder 10 mM Imidazol, 100 mM NaCl, pH 7. Der Zusatz von allgemein stabilisierenden Agenzien wie z. B. Glyzerin oder Ammoniumsulfat mit z.B. 30 % Sättigung wirkt sich positiv auf die Lagerungsfähigkeit aus.

Mit dem erfindungsgemäßen Verfahren unter Verwendung der erfindungsgemäßen Polypeptid-Konstrukte lassen sich prinzipiell alle gram-positiven Bakterien wie Clostridien, Bazillen, Listerien, Staphylokokken, Lactobazillen, Enterokokken, Aerokokken, Pediokokken, Streptokokken, Mycoplasmen, Leuconostoc binden und damit anreichern, einfangen, immobilisieren und gegebenenfalls nachweisen. Die Art der Anwendung richtet sich nach der Art der eingesetzten Proben, die weiter oben definiert sind. Besonders geeignet ist die Methode, um potentiell pathogene Bakterien aus Lebensmittelproben anzureichern und nachzuweisen. Das Verfahren ist jedoch ebenso geeignet, um pathogene Bakterien aus medizinischen oder diagnostischen Proben anzureichern und nachzuweisen. Weiterhin ist es geeignet, um gram-positive Bakterien aus Proben zu entfernen oder nachzuweisen, in denen diese unerwünscht sind wie z.B. in pharmazeutischen oder kosmetischen Präparaten und Prozesslösungen. Das erfindungsgemäße Verfahren bringt in der Anreicherung eine erhebliche Zeitersparnis gegenüber konventionellen Anreicherungsverfahren wie z.B. ISO-Methoden. In Kombination mit einer magnetischen Separation bei einem Einsatz von funktionalisierten Magnetpartikeln zum Einfangen der Bakterien-Poylpeptid-Komplexe können aufwändige und schwer zu automatisierende Separationsmethoden wie z.B. Zentrifugationsschritte ersetzt werden.

Die Erfindung betrifft ferner Nukleinsäuren sowie Vektoren, die für die erfindungsgemäßen Polypeptide kodieren sowie Zellen, die die Nukleinsäuren bzw. Vektoren exprimieren. Der Fachmann ist in der Lage geeingte Nukleinsäuren und Vektoren, die für die erfindungsgemäßen Polypeptide-kodieren mit im Stand der Technik beknanten Maßnahmen herzustellen. Beispielsweise lassen sich ausgehend genetischen Code aus Aminosäuresequenz der erfindungsgemäßen Polypeptide geeingte Nukleinsäuresequenzen ableiten. Dabei kann gegebenenfalls eine optimierte Codonverwednung je nach gewähltem Expressionssystem berücksichtigt werden. Ebenos ist der Fachmann in der Lage, geeignete Vektoren auszuwählen, z.B. um die Expression des erfindungsgemäße Polypeptids über die oben genannte Nukleinsäure zu gewährleisten.

Überraschenderweise stellte sich heraus, dass die Nukleotidsequenz im N-terminalen Teil des JS-Tags der translatierten Polypeptidsequenz wichtig für eine effektive Expression ist. ATreiche Sequenzen zeigten gegenüber GC-reichen Sequenzen unter Beibehaltung der Aminosäuresequenz eine wesentlich effizientere Expression. Es wird vermutet, dass die Ausbildung von Sekundärstrukturelementen am Anfang der transkribierten RNA die Effizienz der Translation beeinflusst. Unter diesen AT-reichen Sequenzen stellten sich drei Varianten (SEQ ID NO: 54-56) als besonders gut geeignet für Fusionen zwischen JS-Tag und anschließender CBD heraus (siehe Tabelle 1). Folglich beginnt in einer bevorzugten Ausführungsform die Teilsequenz der Nukleinsäure, die für den JS-Tag in den erfindungsgemäßen Polypeptiden kodiert, mit einer Sequenz, die ausgewählt ist aus SEQ ID NO: 54-56.

Das erfindungsgemäße Verfahren bzw. die erfindungsgemäßen Polypeptidfragmente zeichnen - sich durch die folgenden Vorteile aus:
- Eine Fusion mit dem JS-Tag ist generell als Kombination mit CBDs aus Endolysinen geeignet, um gram-positive Bakterien schnell und effizient zu binden.
- Eine Fusion aus CBD und JS-Tag ist sehr gut geeignet, um in einem effizienten 2-Schritt-Verfahren Bakterien anzureichern, weil Biotin als Kopplungsgruppe eine extrem gute Immobilisierung der CBD-Bakterien-Komplexe ermöglicht.
- Das 2-Schritt-Verfahren in Kombination mit der Fusion aus CBD und JS-Tag ermöglicht einen geringen Einsatz an Trägermaterial und vor allem an spezifischem Bindeprotein, was ökonomische Vorteile bringt. Die Biotinylierung in diesem Verfahren ist sehr effizient und genau definiert, so dass im Vergleich zu anderen Methoden ein extrem hoher Anteil an funktionellem Bindeprotein zur Verfügung steht. Zudem ist die Bindung von Bakterien an freies Bindeprotein viel effizienter als an Bindeprotein, das vorher auf Oberflächen immobilisiert wurde, wo häufig sterische Probleme, unspezifische Bindung als Nebenreaktion und nicht funktionelle Immobilisierung auftreten.
- Das erfindungsgemäße Verfahren ist ebenfalls geeignet, um mit ein und demselben Träger verschiedene CBD-Bakterien-Komplexe zu immobilisieren.
- Die Fusion aus CBD und JS-Tag erweist sich als Konstruktion von hoher Stabilität, die sich insbesondere durch Langzeitstabilität bei Temperaturen bis zu ca. 30 °C, und Proteasestabilität auszeichnet.
- Der JS-Tag hat eine Länge, die gut zu handhaben ist, weil er nicht zu lang ist, andererseits aber lange genug, um auf einen Spacer verzichten zu können.
- Das erfindungsgemäße Verfahren zur Herstellung eines Polypeptids gemäß der vorliegenden Erfindung funktioniert auch ohne die Koexpression von *birA.*

Die vorliegende Erfindung betrifft ferner einen Kit, umfassend einen Träger, der mit einer Biotin-bindenden Substanz versehen ist wie z.B. Streptavidin oder Avidin als funktionelle Gruppen, ferner umfassend mindestens eine Variante der erfindungsgemäßen Polypeptidfragmente, bei denen eine CBD mit JS-Tag fusioniert ist sowie die für die Anreicherung und gegebenenfalls den Nachweis der gram-positiven Bakterien erforderlichen Pufferlösungen, z.B. Waschpuffer, Ablösepuffer und/oder Zellaufbruchspuffer.

Die folgenden Beispiele erläutern die Erfindung und sind nicht als einschränkend aufzufassen. Sofern nicht anders angegeben, wurden molekularbiologische Standardmethoden verwendet, wie z.B. von Sambrook et al., 1989, Molecular cloning: A Laboratory Manual 2. Auflage, Cold Spring Harbor Laboratory Press, Cold Spring Harbor, New York, beschrieben.

### Experiment 1: Expression, Löslichkeit und funktionelle Assemblierung von Phagenschwanzproteinen mit Biotinylierungs-Tags

Das Ergebnis des Experiments ist in Figur 1 darstellt. Die dort erwähnten Phagenschwanzproteine aus Salmonellen- oder Campylobacterphagen wurden nach Standardmethoden entweder in pET21a oder pET21d kloniert und bei 30 °C in den angegebenen Expressionsstämmen nach IPTG-Induktion exprimiert (jeweils 1 ml Ansätze). Nach 3 bis 4 h wurden die Zellen abzentrifugiert (Tischzentrifuge, 5 min, 13000 rpm) und die Pellets in Puffer (z.B. 20 mM Tris, 5 mM EDTA, pH 8) gelöst Die Zellen wurden mit Ultraschall aufgeschlossen und nochmals zentrifugiert (20 min, 13000 rpm, 4 °C). Die Überstände, die das lösliche Protein enthalten, wurden abgenommen und entweder für 5 min gekocht oder nicht gekocht. In nicht gekochten Proben sollten sich SDS-resistente native Trimere ausbilden, die bei höheren Molekulargewichten zu finden sind. Die Pellets, die das unlösliche Protein enthalten, wurden in gleichem Volumen Puffer resuspendiert (z. B. 20 mM Tris, pH 9, 50 mM NaCl, 5 mM EDTA). Zu allen Proben wurde Lämmli-Probenpuffer gegeben und die Proben auf 12 % ige bzw. 9 % ige SDS-Polyacrylamidgele aufgetragen und mit Coomassie gefärbt.

In allen drei Teilversuchen zeigt sich, dass mit spezifischen Biotinylierungs-Tags bei Phagenschwanzproteinen keine ausreichende Menge an funktionellem Protein erhalten werden kann. Teilweise ist die Expressionsausbeute sehr schlecht, teilweise liegt ein großer Teil des Proteins in unlöslicher Form vor und in keinem Fall erhält man einen hohen Anteil an nativem Protein, das sich durch SDS-resistente oligomere Formen ausweist. Lediglich bei dem P22 ähnlichen Phagenschwanzprotein konnten auf SDS-Gelen Banden von Monomer und nativem Trimer detektiert werden. Von den anderen beiden Proteinen konnten auf Western-Blots nur sehr schwache Monomerbanden detektiert werden, so dass die Position lokalisiert werden konnte und das prinzipielle Funktionieren von Induktion und Expression nachgewiesen werden konnte.

### Experiment 2: Chemische Biotinylierung von CBDs

Es wurden Stammlösungen von jeweils 1 mg/ml von CBD511 (in PBS; 20 mM Natriumphosphat pH 7,4, 120 mM Natriumchlorid) und NHS-Biotin (in DMSO) hergestellt. Zu 1200 µl Proteinlösung wurden 120 µl NHS-Biotin-Lösung hinzugegeben und gut gemischt. Jeweils 300 µl Probenansatz wurden sofort (0 min Wert) oder nach 20 min, 60 min oder 120 min entnommen und auf Eis zu 30 µl 1 M Tris, pH 8 gegeben, um die Reaktion zu stoppen. Alle Ansätze wurden gegen PBS-Puffer dialysiert. Von allen Ansätzen wurde per Absorptionsmessung die Proteinkonzentration bestimmt und im HABA-Test der Biotinylierungsgrad. Dieser lag bei 1,5 bis 2,5 Biotinmolekülen pro CBD-Molekül. Der Zellbindungstest erfolgte mit dem Stamm *Listeria monocytogenes* Scott A, der im 500 µl Testansatz (Puffer PBST; 20 mM Natriumphosphat pH 7,4, 120 mM Natriumchlorid, 0.1 % Tween 20) in einer Konzentration von 10⁴ KbE/ml eingesetzt wurde. Anschliessend wurde biotinylierte CBD511 in den Konzentrationen 0,5 µg/ml, 1 µg/ml, 2µg/ml bzw. 5 µg/ml zugegeben und für 1 min inkubiert. Als Kontrolle diente JS-Tag-CBD511. Es wurden MagPrep-Streptavidin Partikel (Merck) zu 50 µg/ml zugegeben und die Ansätze für 20 min im Über-Kopf-Rolator bei RT (Raumtemperatur) inkubuiert. Nach 5 min Magnetseparation wurde der Überstand abgenommen und die Magnetpartikel 1 Mal mit 500 µl PBST-Puffer gewaschen (10 min). Nach einer 2. Magnetseparation wurden die Magnetpartikel in 1 Puffervolumen aufgenommen und auf Oxford-Platten ausplattiert (unverdünnt und 1:10 verdünnt). Als Kontrolle wurden die vereinigten Überstände nach 1. und 2. Magnetseparation ausplattiert und nach einer Nacht gezählt. Das Experiment ist in Figur 2 abgebildet. Man sieht, dass alle CBDs durch die chemische Biotinylierung inaktiv werden, während der Zellbindungstest mit der mit JS-Tag spezifisch biotinylierten CBD ordnungsgemäß funktioniert.

### Experiment 3a: Nachweis von Listerien in Camembert mit dem 1-Schritt-, 2-Schritt- und dem ISO-Verfahren

300 g Camembert aus einem Supermarkt wurden steril in 25 g Portionseinheiten aufgeteilt und in Stomacherbeuteln bei -80 °C gelagert. Eine Portionseinheit wurde auf Anwesenheit von Listeria gemäß der Vorschrift ISO: 11290-1:1996 FDAM 1 hin untersucht. Bei Abwesenheit einer Listerienkontamination wurden 5 Portionseinheiten bei RT aufgetaut und mit unterschiedlichen Mengen *L. monocytogenes* ScottA infiziert. Hierfür wurde eine üN-Kultur 1/5 verdünnt und bis zu einer OD₆₀₀ von ca. 1 bei 37 °C inkubiert. Anschließend wurden in sterilem PBST (20 mM Natriumphosphat pH 7,4, 120 mM Natriumchlorid, 0,05 % Tween) serielle Verdünnungen erstellt. Es wurden die Portionseinheiten mit 0, 1-10, 11-50, 50-100 und 100-500 KbE /.25 g Camembert kontaminiert und üN bei 4 °C gelagert. Zur exakten Bestimmung der Zellzahlen wurden Duplikate der Verdünnungen auf Oxford Agar (Profos AG) ausplattiert, die Platten 24 h bei 37°C inkubiert und ausgezählt. Zu den Portionseinheiten wurden dann 225 ml Fraser ½ Medium (Profos AG) steril zugegeben, für 1 min im Stomacher homogenisiert und bei 30 °C inkubiert. Nach einer Inkubationszeit von 4 h, 6 h und 24 h wurde je Ansatz 1 ml entnommen.

1-Schritt Verfahren: Zu 1 ml Homogenisat wurden 300 µg/ml der mit StrepTag-CBD511-f2 beschichteten magnetischen Partikel (Dynabeads Epoxy) gegeben und der Ansatz für 20 min im Über-Kopf-Rolator bei RT inkubuiert.

2-Schritt Verfahren: Zu 1 ml Homogenisat wurden 5 µg des StrepTag-GFP-CBD511-f2 Fusionsproteins gegeben und kurz gemischt. Dann wurden MagPrep-Streptavidin Partikel (Merck) zu 50 µg/ml zugegeben und die Ansätze für 20 min im Über-Kopf-Rolator bei RT inkubuiert.

Anschließend wurden die Partikel-Listerien-Komplexe im Magnetfeld an der Gefäßwandung gesammelt und der Überstand entfernt. Der Partikel-Listerien-Komplex wurde 3 x 1 ml PBST (20 mM Natriumphosphat pH 7,4, 120 mM Natriumchlorid, 0,05 % Tween) 10 min im Über-Kopf-Rolator gewaschen, im Magnetfeld an der Gefäßwandung gesammelt und der Überstand jeweils verworfen. Die Partikel-Listerien-Komplexe wurden in 100 µl PBST resuspendiert und auf Oxford-Agar (Profos AG) ausplattiert. Nach 24 h und 48 h bei 37 °C. wurden die Platten ausgezählt und der Anteil der an den magnetischen Partikeln haftenden Listerien in Prozent der eingesetzten Zellen berechnet. Parallel wurden die kontaminierten Ansätze in Anleitung an die Norm ISO: 11290-1:1996 FDAM 1 auf Listerien hin untersucht. Hierzu wurde zu den angegebnen Zeiten 100 µl auf 10 ml Fraser-Medium (Profos AG) gegeben, 24 h bei 37 °C im Roller inkubiert und dann auf Oxford-Agar (Profos AG) ausgestrichen. Alle Ansätze wurden in Quadruplets durchgeführt.
Es hat sich gezeigt, dass sowohl mit dem 1-Schritt-Verfahren, wie auch mit dem 2-Schritt-Verfahren die notwendigen Anreicherungszeiten signifikant kürzer sind als mit dem Verfahren nach ISO: 11290-1:1996, um geringe Listerienkontaminationen in Camembert nachzuweisen. Hinsichtlich der Verkürzung der Anreicherungszeit sind die Ergebnisse für das 2-Schritt-Verfahren besser als für das 1-Schritt-Verfahren.

### Experiment 3b: Nachweis von Listerien aus Mozzarella

Zu jeweils 25 g Mozzarella wurden je 225 ml FDA-Medium hinzugegeben und die Portionen steril in Stomacherbeuteln homogenisiert. Die Proben wurden über Nacht bei 30 °C inkubiert. Es wurden Listerien der Stämme EGDe (Serotyp 1/2a) und ScottA (Serotyp 4b) zugesetzt in einer Konzentration von 500 KbE/ml.Vor dem Listeriennachweis wurden die Proben jeweils mit 1/10 Volumen PBST gepuffert.

1-Schritt Verfahren: Zu 1 ml Homogenisat wurden 300 µg/ml der mit JS-Tag-GFP-CBD511_f3 beschichteten magnetischen Partikel (Dynabeads M270 Epoxy) gegeben und der Ansatz für 20 min im Über-Kopf-Rolator bei RT inkubuiert.

2-Schritt Verfahren: Zu 1 ml Homogenisat wurden je 0.5, 2, 5 oder 10 µg des JS-Tag-GFP-CBD511_f3 Fusionsproteins gegeben und kurz gemischt. Dann wurden MagPrep-Streptavidin Partikel (Merck) zu 50 µg/ml zugegeben und die Ansätze für 20 min im Über-Kopf-Rolator bei RT inkubuiert.

Anschließend wurden die Partikel-Listerien-Komplexe im Magnetfeld an der Gefäßwandung gesammelt und der Überstand entfernt. Der Partikel-Listerien-Komplex wurde 1 x 1 ml PBST 10 min im Über-Kopf-Rolator gewaschen, im Magnetfeld an der Gefäßwandung gesammelt und der Überstand jeweils verworfen. Die Partikel-Listerien-Komplexe wurden in 100 µl PBST resuspendiert und auf Oxford-Agar (Profos AG) ausplattiert. Nach 24 h bei 37 °C. wurden die Platten ausgezählt und der Anteil der an den magnetischen Partikeln haftenden Listerien in Prozent der eingesetzten Zellen berechnet. Alle Ansätze wurden 2fach durchgeführt.
Es hat sich gezeigt, dass aus Lebensmitteln mit Hilfe des JS-Tag-GFP-CBD511_f3 Fusionsproteins Listerien isoliert werden können. Bei Mozzarella gelingt dies mit dem Stamm EGDe wesentlich besser als mit ScottA. Bei den Lebensmitteln sollten etwas höhere Konzentrationen an Protein eingesetzt werden, um eine hohe Bindungseffizienz zu erreichen.

### Experiment 4: Zellbindekapazität von JS-Tag-CBDs

### Experiment 4a: Vergleich der Zellbindekapazität von JS-Tag und Avi-Tag Konstrukten.

Die Zellbindekapazität der verschiedenen Konstrukte wurde mit dem *Listeria* Stamm ScottA nach dem 2-Schritt-Verfahren getestet. Es wurden folgende Konstrukte eingesetzt: JS-GFP_CBD511_f3, JS-CBD511_f3 und Avi-Tag-GFP_CBD511_f3. Da die Konstrukte unterschiedlich lang sind, wurden gleiche molare Mengen an Bindeprotein eingesetzt. Zu 1 ml Testansatz (Listerien aus frischer Vorkultur in einer Konzentration von 10⁴ Kbe/ml, PBST-Puffer) wurden die angegebenen Mengen der Fusionsproteine zugegeben und kurz gemischt. Dann wurden MagPrep-Streptavidin Partikel (Merck) zu 50 µg/ml zugegeben und die Ansätze für 20 min im Über-Kopf-Rolator bei RT inkubuiert. Anschließend wurden die Partikel-Listerien-Komplexe im Magnetfeld an der Gefäßwandung gesammelt und der Überstand entfernt. Der Partikel-Listerien-Komplex wurde 1 x 1 ml PBST 10 min im Über-Kopf-Rolator gewaschen, im Magnetfeld an der Gefäßwandung gesammelt und der Überstand jeweils verworfen. Die Partikel-Listerien-Komplexe wurden in 100 µl PBST resuspendiert und auf Oxford-Agar (Profos AG) ausplattiert. Nach 24 h bei 37 °C. wurden die Platten ausgezählt und der Anteil der an den magnetischen Partikeln haftenden Listerien in Prozent der eingesetzten Zellen berechnet. Alle Ansätze wurden 2fach durchgeführt und Mittelwerte gebildet. Das Experiment ist in Figur 4A dargestellt. Man sieht, dass die JS-Tag Konstrukte besser binden als das Avi-Tag Konstrukt. Hier muss wesentlich mehr Protein eingesetzt werden, um die maximale Zellbindung zu erreichen.

### Experiment 4b: Reinigung von Avi-GFP-CBD511_f2 und JS-CBD511_f2

Beide Proteine wurden nach Expression in *E*. *coli* HMS174, Zellernte, Aufschluss und Ammoniumsulfatfällung über Kationenaustauschchromatographie gereinigt. Avi-GFP-CBD511_f2 wurde mit birA koexprimiert.

### Experiment 4c: Konzentrationsabhängigkeit der Zellbindung an die Magnetpartikel.

Es wurde untersucht, ab welcher Konzentration an spezifischem Bindeprotein die maximale Zellbindung erreicht wird. Als Bindeprotein wurde JS-CBD511_f3 in den Konzentrationen 0 µg/ml, 0,02 µg/ml, 0,1 µg/ml, 0,5 µg/ml, 1 µg/ml, 2 µg/ml und 3 µg/ml eingesetzt. Der Versuch wurde analog Experiment 4a durchgeführt. Das Ergebnis ist in Figur 4B abgebildet. Es zeigt sich, dass die maximale Zellbindung von praktisch 100 % aller eingesetzten Zellen bereits bei der sehr geringen Proteinkonzentration von 0,5 µg/ml erreicht wird.

### Experiment 5: Optimierung der Nukleotidsequenz im N-terminalen Bereich des JS-Tags

Es hatte sich gezeigt, dass JS-Tag-CBD-Konstrukte, die die Nukleotidsequenz aus dem C-terminalen Teil der *Klebsiella pneumoniae* Oxalacetatdecarboxylase enthielten, zwar *in vivo* biotinyliert wurden, aber eine sehr schlechte Expressionsausbeute für die Proteine aufwiesen (siehe auch Tabelle 2). Daraufhin wurde die Nukleotidsequenz kodierend für die ersten Aminosäuren der *Klebsiella*-Sequenz und das von uns zusätzlich eingeführte Starterpeptid (MVGA) auf Expressionsausbeute hin optimiert ohne dass die Aminosäuresequenz geändert wurde. Unter einer ganzen Reihe von verschiedenen Sequenzvorschlägen stellten sich 5 Varianten, die für 3 verschiedene Nukleotidsequenzen kodierten, als besonders geeignet heraus. Allen Varianten gemeinsam ist, dass sie im Vergleich zur Originalsequenz AT-reich sind, wenn die Kodonauswahl für die jeweilige Aminosäure dies zulässt. Die Varianten (Variantionen sind grau hinterlegt) sind in Tabelle 1 zusammengefaßt.

**Tabelle 1: Nukleotidvarianten für den N-terminalen Sequenzbereich des JS-Tags**

| **Variante auf Nukleinsäureebene** | **Sequenz** | **SEQ ID NO:** |
|---|---|---|
| | Nukleotidsequenz | |
| Kleb._Oxalacetatdecarboxylase | GTG ACC GCC CCG CTG | |
| | | |
| JS4a | ATG GTT GGT GCA GTT ACA GCT CCG CTG | 54 |
| JS5b, JS5d | ATG GTA GGT GCA GTT ACA GCT CCG CTG | 55 |
| JS10a, JS10c | ATG GTT GGT GCA GTA ACA GCT CCG CTG | 56 |
| | | |
| | Aminosäuresequenz | |
| | M V G A V T A P L | |

Die Varianten JS4a, JS5b, JS5d, JS10a und JS10c stellten sich als besonders gut geeignet für die Expression von Fusionskonstrukten aus JS-Tag und CBD heraus. Abhängig vom verwendeten CBD-Anteil gab es leichte Unterschiede in der Expression zwischen den einzelnen Varianten. Es konnten aber prinzipiell alle eingesetzt werden.

### Experiment 6: Koexpression von JS-Tag-CBD-Konstrukten mit birA

Es wurde getestet, ob eine Koexpression von birA (Biotinligase) notwendig ist, damit die JS-Tag-Konstrukte effizient *in vivo* biotinyliert werden.
Das Konstrukt JS5b-CBD511_f2 wurde im Expressionsstamm *E*. *coli* BL21 (DE3) im Vektor pet21a exprimiert. Falls birA koexprimiert wurde, war zusätzlich das Plasmid pACYC184-birA vorhanden. Aus üN-Kulturen des Expressionsstammes wurde frisches LB-Medium (in 2 1 Kolben, Gesamtmenge 4 1 bis 101) angeimpft, die Zellen bei einer OD₆₀₀ von ca. 0,4 bis 0,6 mit 1 mM IPTG induziert und nach 4 h geerntet. Zu einem Teil der Ansätze wurde bei der Induktion zusätzlich 50 µM Biotin zugegeben. Jedes Konstrukt wurde zwei Mal getestet. Die Zellen wurden nach der Ernte zentrifugiert und aufgeschlossen. Die Reinigung erfolgte durch fraktionierte Ammoniumsulfatfällung und anschliessende Kationenaustauschchromatographie. Die Reinheit der Proteine wurde in SDS-Gelen dokumentiert.

**Tabelle 2: Expressions- und Reinigungsausbeuten verschiedener JS-Tag-CBD-Konstrukte.**

| Konstrukt | BirA Koexpression | Expressions-Temperatur | Protein mg/g Zellen |
|---|---|---|---|
| JS-(original) | + | 37 °C | 1,1 |
| JS-(original) | + | 37 °C | 0,4 |
| JS-(5b-Variante) | + | 30 °C | 5,0 |
| JS-(5b-Variante) | + | 37 °C | 7,0 |
| JS-(5b-Variante) | - | 37 °C | 3,8 |
| JS-(5b-Varinate) | - | 37 °C | 6,9 |

Die Expressionsausbeute ist bei dem in der Nukleotidsequenz optimierten Konstrukt JS-5b (siehe Experiment 5) wesentlich besser als bei der Originalsequenz aus *Klebsiella pneumoniae.* Die Koexpression von birA bringt praktisch keine Erhöhung der Ausbeute an Protein bezogen auf die eingesetzte Menge an Bakterienzellen.

Zellbindetests mit den JS-Tag-Konstrukten mit und ohne Koexpression von birA.

JS5b_CBD511_f2 wurde in Zellbindetests mit dem *Listeria* Stamm Scott A eingesetzt. Es wurden 1 ml Ansätze mit einer Listerienkonzentration von 10³ KbE/ml verwendet. Als Magnetpartikel wurden Streptavidin-Magnetic-Particles (Roche) in einer Konzentration von 0,05 mg/ml eingesetzt. Ansonsten erfolgte die Versuchsdurchführung nach dem 2-Schritt-Verfahren wie in Experiment 3b beschrieben. Es wurden sowohl Konstrukte aus Expression ohne und mit birA als auch ohne und mit Zugabe von zusätzlichem Biotin (50 µM) untersucht. Das Ergebnis ist in Figur 5 abgebildet.

### Experiment 7: Vergleich der Bakterienbindung mit His-Tag-CBDs und JS-Tag-CBDs

*Bacillus cereus* (DSM345) wurde aus einer Vorkultur frisch überimpft und bei 30 °C in TS-Medium bis zu einer OD₆₀₀ von ca. 1 angezogen. Im Testansatz (1ml) wurden die Bakterien in einer Konzentration von 3x10³ KbE/ml eingesetzt. Für die His-Tag-Konstrukte war der Puffer Nickel-Puffer A (20 mM Na-Phosphat, 500 mM NaCl, 20 mM Imidazol, 0,1 % Tween 20, pH 7,4), für JS-Tag-Konstrukte PBST. His-Tag-CBDBa und JS-Tag-CBDBa wurden in einer Konzentration von 1 µg/ml, His-Tag-CBD21 und JS-Tag-CBD21 in einer Konzentration von 0,12 µg/ml zugegeben und für 5 min bei Raumtemperatur inkubiert. Danach wurden Ni-NTA-Agarose-Beads (Qiagen) bzw. MagPrep-Streptavidin-Beads in einer Konzentration von ca. 8x10⁶ Partikel/ml zugegeben, für 20 min rollierend inkubiert und für 5 min mit dem Magnetseparator separiert. Die Magnetpartikel wurden mit einem Ansatzvolumen Puffer gewaschen. Die Waschlösung und der Überstand mit nicht gebundenen Bakterien wurden auf CASO-Vollmediumplatten ausplattiert, ebenso wie die wieder aufgenommenen Magnetpartikel mit gebundenen Bakterien. Nach ca. 18 h bei 27 °C wurden die Kolonien ausgezählt. Es wurden jeweils 2 Versuche durchgeführt. Als Kontrollen dienten Ansätze ohne Proteinzugabe. Der Versuch ist in Figur 6 abgebildet.
Während unter den gegebenen Bedingungen mit den JS-Tag-Konstrukten die Bakterien sehr gut spezifisch gebunden wurden, war die Ausbeute an gebundenen Zellen mit den His-Tag-Konstrukten völlig unzureichend. Es gab bei beiden Sorten von Magnetpartikeln praktisch keine unspezifische Bindung der Bakterien.

### Experiment 8: Anreicherung von Listerien aus verschiedenen Medien bzw. Puffer

*Listeria monocytogenes* EGDe wurde mit Avi-CBD511_f2 bzw. JS-CBD511_f2 (jeweils 5 µg/ml) aus verschiedenen Medien bzw. PBST-Puffer angereichert. Die Listerien wurden in TB-Medium bis zu einer OD₆₀₀ ∼ 1 inkubiert und daraus in den angegebenen Medien oder Puffer Vorverdünnungen hergestellt. Im Test wurden die Listerien in einer Konzentration von 10⁴ KbE/ml eingesetzt. Der Zellbindetest erfolgte nach dem 2-Schritt-Verfahren mit 20 min rollierender Inkubation mit 50 µg/ml MagPrep-Streptavidin Partikel (Merck) und 1 Mal Waschen mit PBST. Die gebundenen und nicht gebundenen Zellen wurden auf Oxford-Agar ausplattiert und nach einer Nacht ausgezählt. Angegeben ist jeweils der Mittelwert aus 2 Versuchen. Das Ergebnis des Experiments ist in Figur 7 abgebildet.

### Experiment 9: Vergleich der Bakterienbindung in biotinhaltigen Proben mit Strep-Tag-CBDs und JS-Tag-CBDs

Biotin ist häufig in Lebensmittelproben enthalten. Da sowohl die Bindung von JS-Tag als auch Strep-Tag an Streptavidin durch Biotin kompetitiert wird, wurde untersucht, welches System in biotinhaltigen Proben besser geeignet ist, um Bakterien spezifisch anzureichern. *Bacillus cereus* (DSM345) wurde aus einer Vorkultur frisch überimpft und bei 30 °C in TS-Medium bis zu einer OD₆₀₀ von ca. 1 angezogen. Im Testansatz (1ml) wurden die Bakterien in einer Konzentration von 1x10³ KbE/ml eingesetzt. Als Testlösung wurden PBST mit den angegebenen Konzentrationen an Biotin (0,01 µM, 0,1 µM, 1 µM) verwendet. JS-Tag- bzw. Strep-Tag-CBDBa wurde in einer Konzentration von 20 µg/ml zugegeben und für ca. 2 min bei RT mit den Bakterien inkubiert. Streptavidin-PA-Beads wurden in einer Konzentration von 50 µg/ml zugegeben, für 20 min rollierend inkubiert und für 5 min mit dem Magnetseparator separiert. Die Magnetpartikel wurden mit einem Ansatzvolumen Puffer gewaschen. Die Waschlösung und der Überstand mit nicht gebundenen Bakterien wurden auf CASO-Vollmediumplatten ausplattiert, ebenso wie die wieder aufgenommenen Magnetpartikel mit gebundenen Bakterien und über Nacht bei RT inkubiert. Nach weiteren 4 h bei 30 °C wurden die Kolonien ausgezählt. Es wurden jeweils 2 Versuche durchgeführt. Als Kontrollen dienten Ansätze ohne Proteinzugabe. Das Ergebnis ist in Figur 8 dargestellt. Es zeigt sich, dass die Zellbindung in biotinhaltigen Proben mit dem JS-Tag-System wesentlich besser funktioniert als mit dem schlechter bindenden Strep-Tag-System. Bei einer Biotinkonzentration von 1 µM kann mit JS-Tag noch spezifische Bindung nachgewiesen werden, während dies bei Strep-Tag unter den gleichen Bedingungen nicht möglich ist.

### Experiment 10: Langzeitstabilität der JS-Tag-CBD-Konstrukte unter verschiedenen Bedingungen

Stammlösungen von JS-CBD-511_f3 (ca. 1 mg/ml) und Streptavidin-Magnetic-Particles (ca. 1 mg/ml) wurden unter den angebebenen Bedingungen inkubiert und in Zellbindetests mit Listerien eingesetzt. Die Konzentration der Magnetpartikel im 1 ml Test (PBST-Puffer) war 50 µg/ml, die Proteinkonzentration wie angegeben und die eingesetzte Bakterienzahl 10⁴ KbE/ml.

Experiment 10a: Bindeprotein und Magnetpartikel wurden bei - 20 °C, 4 °C, RT (ca. 23 °C) und 37 °C in 100 mM Natriumphosphat, pH 6-7, 2 mM EDTA bis zu 126 Tage gelagert und in den angegebenen Konzentrationen in Listerienbindetests eingesetzt. Man sieht, dass lediglich nach 126 Tagen und hier vor allem bei einer Inkubation bei 37 °C die Bindungseffizienz deutlich nach unten geht.
Experiment 10b: Bindeprotein und Magnetpartikel wurden bei - 20 °C, 4 °C, RT (ca. 23 °C) und 37 °C in 10 mM Imidazol, pH 7, 100 mM NaCl plus 30 % Ammoniumsulfat bis zu 74 Tage gelagert und in den angegebenen Konzentrationen in Listerienbindetests eingesetzt. Man sieht, dass bei allen Temperaturen die Bindungseffizienz nach 74 Tagen Inkubation etwas nach unten geht, aber nach wie vor bei über 50 % liegt.
Beide Puffersysteme erscheinen für die Langzeitinkubation von JS-CBD-Konstrukten und geeignete Streptavidin-Magnetpartikel geeignet.
Die Ergebnisse sind in Figur 9 abgebildet.

### Experiment 11: Anreicherung von Listerien aus verschiedenen Lebensmitteln

Der Versuch ist in Figur 10 dargestellt.
Experiment 11a: Eine üN-Kultur *Listeria monocytogenes* Scott A wurde 1:5 in FDA-Oxoid Medium verdünnt und bei 37 °C bis OD₆₀₀ ∼ 1 angezogen. Milch bzw. homogenisierter Käse wurden 1 : 10 in PBST verdünnt und mit Listerien in einer Konzentration von 10⁴ KbE/ml inokuliert. JS4a-CBD511_f2 wurde in den angegebenen Konzentrationen in Eppendorf-Cups vorgelegt und mit jeweils 1 ml Probenlösung gemischt. Als Magnetpartikel wurden Streptavidin-Magnetic-Particles (Roche) in einer Konzentration von 50 µg/ml eingesetzt und die Proben für 10 min im Über-Kopf-Rollator inkubiert. Die Magnetpartikel wurden für 5 min im Magnetseparator separiert und anschliessend 1 Mal mit 1 ml PBST-Puffer gewaschen und wieder in 1 ml Puffer aufgenommen. Von der Beadsfraktion und den vereinigten Überständen (nach 1. Magnetseparation und Waschschritt) wurden je 100 µl unverdünnt und 1:10 verdünnt auf Oxford Agar ausplattiert und über Nacht bei 37 °C inkubiert. Am nächsten Tag wurden die Listerien ausgezählt und berechnet, wie viele Prozent der gefundenen Bakterien jeweils über die JS4a-CBD511_f2 and die Magnetpartikel gebunden wurden. Es zeigte sich, dass sowohl aus Milch als auch aus Käse bereits bei einer Konzentration an spezifischem Bindeproteine von 0,2 µg/ml JS4b-CBD511_f2, die eingesetzten *Listeria* Zellen praktisch vollständig entfernt wurden. Bereits bei einer sehr geringen Proteinkonzentration von 0,02 µg/ml konnten 50 % der Bakterien entfernt werden.
Experiment 11b: Je 25 g Räucherlachs und Salami wurden homogenisiert, 1 : 10 mit LEB-FDA-Medium verdünnt, mit *Listeria innocua* in einer Konzentration von 10⁴ KbE/ml inokuliert und im Stomacherbeutel für 1 h bei RT inkubiert. Die eingesetzte Bakterienverdünnung wurde als Kontrolle ausplattiert. Es wurden jeweils 1 ml Proben aus dem Filtrat der Stomacherbeutel entnommen und mit JS5b-CBD511_f3 (1 µg/ml) vermischt. Bei den Kontrollen wurde keine Protein zugegeben. Direkt anschliessend wurden 400 µg/ml PA-Streptavidin-Beads zugegeben und für 20 min im Über-Kopf-Rollator inkubiert. Die Magnetpartikel wurden für 5 min im Magnetseparator separiert und anschliessend 1 Mal mit 1 ml PBST-Puffer gewaschen und wieder in 1 ml Puffer aufgenommen. Von der Beadsfraktion und den vereinigten Überständen (nach 1. Magnetseparation und Waschschritt) wurden je 100 µl unverdünnt und 1:10 verdünnt auf Oxford Agar ausplattiert und über Nacht bei 37 °C inkubiert. Am nächsten Tag wurden die Listerien ausgezählt und berechnet, wie viele Prozent der gefundenen Bakterien jeweils über die JS5b-CBD511_f3 and die Magnetpartikel gebunden wurden. Es zeigte sich, dass sowohl aus Räucherlachs als auch aus Salami mehr als 90 % der Bakterien gebunden werden konnten.

### Experiment 12: Nachweis von Listerien aus Lebensmitteln mit der NASBA-Technik

Eine üN-Kultur von *Listeria monocytogenes* Scott A wurde 1:5 in LEB-FDA-Medium verdünnt und bei 37 °C bis OD₆₀₀ ∼ 1 angezogen. Davon wurden serielle Verdünnungen bis zu 10² KbE/ml erstellt. Es wurden 2 Mal je 60 g Salami abgewogen und mit Listerien in einer Konzentration von 5 KbE/25 g Lebensmittel kontaminiert. Zu den 60 g Proben wurde je 540 ml LX-Medium (BioMerieux) hinzugegeben, die Proben homogenisiert und in Stomacherbeuteln bei 17 h bzw. 20 h bei 37 °C inkubiert. Anschliessend wurden die Bakterien mit JS5b-CBD511_f2 im 1 ml Ansatz aus dem Überstand des Stomacherbeutels eingefangen. Zusätzlich wurde Überstand als Kontrolle ausplattiert und gezählt. Das Bindeprotein wurde in einer Konzentration von 1 µg/ml zugegeben und für 1 Minute mit der Probe inkubiert. Danach wurde PA-Streptavidin-Magnetpartikel in einer Konzentration von 400 µg/ml zugegeben und für 20 min im Über-Kopf-Rollator inkubiert. Die Magnetpartikel wurden für 5 min im Magnetseparator separiert und anschliessend 3 Mal mit je 1 ml TT-Puffer (50 mM Tris, pH 8,0, 0,1 % Tween 20) gewaschen. Ein Teil der Magnetpartikel mit daran gebundenen Bakterien wurde auf Oxford-Agar ausplattiert und nach 15 bis 20 h Inkubation ausgezählt, um die Bindungseffizienz zu erhalten. Das Ergebnis ist in Teilabbildung A dargestellt. Ein Teil der Magnetpartikel mit über JS-Tag-CBD gebundenen Listerien wurde in die NASBA zum Nachweis eingesetzt. Die Zellen an den Beads werden mit 5 µg/ml Listerien spezifischem Endolysin in 100 µl Lysepuffer A (21 % DMSO, 57 mM Tris, 0,4 % Triton X100) für 15 min bei RT lysiert. Anschliessend werden die Magnetbeads für 5 min im Magnetseparator abgetrennt und je 14 µl des Lysates pro NASBA-Reaktion eingesetzt. Es werden Teststreifen für je 8 Proben verwendet, die jeweils bereits vorgefertigte Listeria-spezifische Primerkügelchen tragen. Zu jeder Probe werden noch 5 µl einer Enzymlösung für NASBA hinzugegeben und die Reaktion nach Herstellerangaben durchgeführt. Als NASBA-System wurde der Nuclisens EasyQ Analyzer (BioMerieux) zusammen mit dem entsprechenden Thermoblock verwendet. Eine Auswertung der Daten erfolgt durch ein zeitabhängiges Fluoreszenzsignal. Bei erfolgreicher Nachweisreaktion steigt das Fluoreszenzsignal nach ca. 30 min Detektionszeit auf ein höheres Niveau an und verbleibt dort. Pro Versuchsansatz wurden 7 NASBA-Reaktionen durchgeführt. Die NASBA-Detektion (nach 17 h Inkubation) wird in Figur 11B dargestellt. Man sieht, dass in allen 7 Reaktionen ein positives Fluoreszenzsignal detektiert wird, der Listeriennachweis über spezifische RNAprimer also geklappt hat.
Es zeigt sich, dass in dem beschriebenen System bereits nach 17 h Inkubation des Lebensmittels mit einer Listerien-Konzentration von 5 KbE/25 g mehr als 99 % der Bakterien gebunden und nachgewiesen werden können. Der Nachweis funktioniert sowohl konventionell über Selektivplatten (siehe Fig. 11A) als auch über nukleinsäurebasierte Methoden wie NASBA, die nach dem Einfangen der Bakterien nur ca. 2,5 h dauern (siehe Fig. 11B).
Vergleichbare Experimente wurden auch mit Räucherlachs, Shrimps, Brie, Puten- und Schweinewurst und Ziegenfrischkäse erfolgreich durchgeführt.

### Experiment 13: Spezifische Zellbindung von JS-Tag-CBDs, die aus Bacillus-Endolysinen abgeleitet wurden.

Für alle Bakterienstämme wurden üN-Kulturen in Vollmedium angesetzt. Die üN-Kulturen wurden 1:20 bis 1:5 in Vollmedium (z.B. CASO, LB, TS, TY) überimpft und zu einer OD₆₀₀ von ca. 1 weiter wachsen gelassen. Wachstumstemperatur für alle Bazillen war 30 °C, für alle anderen Stämme 37 °C. Aus den Vorkulturen wurden Verdünnungsreihen hergestellt. Im Testansatz (500µl Volumen) wurden die Bakterien in einer Konzentration von 10³ bis 10⁴ KbE eingesetzt. Zur Ermittlung der genauen Zellzahlen wurden jeweils Kontrollen der eingesetzten Verdünnungen ausplattiert und ausgezählt. Zum Testansatz (Puffer PBST) wurde jeweils JS-Tag-CBDBa bzw. JS-Tag-CBD21 in Konzentrationen von 10 µg/ml bzw. 1 µg/ml zugegeben und ca. 1 min mit den Zellen inkubiert. Anschliessend wurden MagPrep-Streptavidin Partikel (Merck) in einer Konzentration von 50 µg/ml zugegeben, die vorher mit CASO-Tween (0,1 %)-Lösung blockiert worden waren. Der Ansatz mit Bakterien, Bindeprotein und Magnetpartikeln wurde 20 min im Über-Kopf-Rollator bei RT inkubiert. Die Magnetpartikel wurden für 5 min im Magnetseparator abgetrennt, danach 1x mit 1 ml PBST gewaschen und anschliessend in Puffer resuspendiert. Von der resuspendierten BeadsFraktion mit gebundenen Zellen und von der vereinigten Fraktion aus Überstand nach Magnetseparation und Waschlösung wurden je 100 µl ausplattiert. Nach dem Trocknen wurden die Platten über Nacht bei der jeweiligen Wachstumstemperatur inkubiert und am nächsten Morgen ausgezählt und um entsprechende Verdünnungsfaktoren korrigiert. Angegeben ist jeweils, wie viele Prozent der insgesamt gefundenen Bakterien spezifisch über die JS-Tag-CBD an die Magnetpartikel gebunden und von der Probe abgetrennt wurden. Als Kontrolle für potentiell unspezifische Bindung der Bakterien an die Magnetpartikel dienten Ansätze, in denen kein spezifisches Bindeprotein zugesetzt wurde. Als weitere Kontrolle für die zu erwartenden Zellzahlen dienten die Bakterienvorverdünnungen, die ebenfalls ausplattiert und gezählt wurden. Ausgewertet wurden nur Versuche, in denen die Gesamtzahl der wieder gefundenen Zellen im Bereich 80 % bis 120 % der insgesamt eingesetzten Zellen lag. Es wurden jeweils 2 bis 4 Versuche pro Test durchgeführt.
Eine Übersicht der entsprechenden Bindedaten mit verschiedenen *Bacillus*-Stämmen und anderen gram-positiven wie gram-negativen Bakterien ist in Tabelle 3 dargestellt.

**Tabelle 3: Bindungsfähigkeit von JS-CBDBa und JS-CBD21 für verschiedene Bakterienstämme im Zellbindeassay**

| **ProCC** | **Alternative Bezeichnung** | **Spezies** | **JS-CBDBa** | **JS-CBD21** |
|---|---|---|---|---|
| Bacillus cereus Gruppe | | | | |
| S1579 | DSM345; ATCC 11778, ATCC 9634 | B. cereus | +++ | +++ |
| S1791 | HER1399; ATCC13472 | B. cereus | O | +++ |
| S1792 | WS2453; ATCC12826 | B. cereus | ++ | ++ |
| S2332 | DSM31; ATCC14579; SBC 10528 | B. cereus | - | ++ |
| S2333 | DSM4312; F4810/72; WSBC 10530 | B. cereus | - | ++ |
| S2334 | NHV391/98; WSBC 10559 | B. cereus | +++ | +++ |
| S2335 | F4370/43; WSBC 10602 | B. cereus | - | + |
| S2336 | DSM 4222; F837/76; SBC 10566 | B. cereus | - | + |
| S2337 | ATCC 10987; WSBC 10865 | B. cereus | + | + |
| S332 | | B. cereus | +++ | +++ |
| S2344 | WSBC 10204; type strain | B. weihenstephanensis | - | + |
| S2345 | WSBC 10210 | B. weihenstephanensis | - | + |
| S2346 | WSBC 10295 | B. weihenstephanensis | - | + |
| S2347 | WSBC 10363 | B. weihenstephanensis | - | + |
| S471 | | B. thuringiensis | - | + |
| S2338 | WS2734; ATCC10792; DSM 2046 | B. thuringiensis | - | + |
| S2339 | WSBC 20822 | B. thuringiensis tenebrionis | +++ | +++ |
| S1586 | DSMZ 299 | B. mycoides | | + |
| S1587 | DSMZ 2048; type strain; ATCC 6462 | B. mycoides | + | ++ |
| S2340 | WS2641; ATCC6462; DSM2048 | B. mycoides | - | + |
| S2341 | WS 3118; NRRLB-617; type strain | B. pseudomycoides | - | + |
| S2342 | WS 3119 | B. pseudomycoides | - | + |

| Andere Bacillen | | | | |
|---|---|---|---|---|
| S2355 | WS3125; ATCC14574; DSM23 | B. badius | - | ++ |
| S2350 | WS1526; DSM11; ATCC4513 | B. circulans | - | +++ |
| S2349 | WS3009; ATCC7050; DSM1 | B. coagulans | - | +++ |
| S2348 | WS1527 | B. firmus | - | +++ |
| S2353 | WS1528; ATCC14580; DSM13 | B. licheniformis | - | ++ |
| S1170 | DSM90 | B. megaterium | O | + |
| S2356 | WS1538; DSM36; ATCC842 | B. polymyxa | - | - |
| S2354 | WS1533; ATCC7061; DSM27 | B. pumilus | - | + |
| S2351 | WS1534; ATCC14577; DSM28 | B. sphaericus | - | - |
| S1795 | DSMZ1970 | B. subtilis | - | +++ |
| S0020 | | B. vallismortis | - | +++ |

| Gram-positive Nicht-Bacillen | | | | |
|---|---|---|---|---|
| S776 | ScottA (4b) | Listeria monocytogenes | - | ○ |
| S1095 | EGD (1/2a) | Listeria monocytogenes | - | + |
| S459 | | Staphyloccocus aureus | - | ++ |
| S1513 | | Staphyloccocus aureus | - | ++ |
| S1514 | | Staphyloccocus aureus | - | ++ |
| S1520 | | Staphyloccocus aureus | - | +++ |
| S1546 | | Staphyloccocus epidermides | - | + |
| S1503 | | Staphyloccocus epidermides | - | ++ |
| S1510 | | Staphyloccocus epidermides | - | +++ |
| S1508 | | Staphyloccocus epidermides | - | ++ |
| S1509 | | Staphyloccocus hämolyticus | - | ++ |
| S1511 | | Staphyloccocus hämolyticus | - | ++ |
| S1549 | | Staphyloccocus hämolyticus | hämolyticus - | + |
| S1548 | | Staphyloccocus hämolyticus | - | ○ |
| S1176 | | Enteroccocus faecalis | - | ++ |
| S1187 | | Micrococcus luteus | - | + |
| S1798 | | Streptococcus equi spp equi | ++ | +++ |
| S1603 | | Streptococcus mutans | ++ | +++ |

| Gram-negative Bakterien | | | | |
|---|---|---|---|---|
| S484 | | Salmonella tenessee | | - |
| S169 | | E.coli HMS | - | - |
| | | | | |
| ProCC: PROFOS Culture Collection; | | | | |
| Bindung: 0%: -; < 10 %: o; 10 % - 30 %: +; 30 % - 60%: ++; 60 % -100 %: +++ | | | | |

Beide CBDs zeigen wie erwartet keinerlei Bindung an gram-negative Bakterien. Unerwarteterweise sind jedoch die Bindungsspezifitäten der CBDs, die beide aus *Bacillus cereus* Phagen isoliert wurden, völlig unterschiedlich. Es zeigte sich, dass CBDBa sehr spezifisch ist für Bazillen aus der *Bacillus cereus* Gruppe. Außerhalb dieser Gruppe werden lediglich 2 Streptococcus Stämme erkannt. CBD21 weißt jedoch eine ungewöhnlich breite Bindungsspezifität für gram-positive Bakterien auf. Es werden alle Vertreter aus der *Bacillus cereus* Gruppe gebunden, dazu alle weiteren getesteten Bazillen bis auf *B. sphaericus* und *B. polymyxa.* Außergewöhnlich ist, dass auch gram-positive Bakterien aus anderen Familien gebunden wurden. Die 6 getesteten Familien, die sich dadurch auszeichnen, dass sie ein hohes pathogenes Potential aufweisen, wurden alle erkannt. CBD21 ist daher in besonderer Weise geeignet, um pathogene Bakterien in verschiedenen Bereichen, wo diese ein Problem darstellen, anzureichern, zu entfernen und nachzuweisen. Gegenüber weiteren getesteten Fragmenten, die ebenfalls aus dem Endolysin plyB21 abgeleitet wurden, zeichnete sich das hier dargestellte Fragment (SEQ ID No 19:) durch erhöhte Stabilität und verringerte Aggregationsanfälligkeit aus.

### Experiment 14: Spezifische Bindung von Bacillus cereus aus einer Mischung von Bakterien.

Es wurden über Nachtkulturen folgender Bakterien in Vollmedium angesetzt: *Bacillus cereus* (DSM345), *Salmonella tennessee, Listeria monocytogenes* (ScottA), *Staphylococcus aureus, E. coli* HMS174 (DE3). Die üN-Kulturen wurden in frisches Medium überimpft und ca. 3 h bei 37 °C bzw. 30 °C (Bacillen) bis zu einer OD₆₀₀ von ∼ 1 wachsen gelassen. Für den Test wurden die Stämme in einer Verdünnung von ca. 10³ KbE/ml eingesetzt. Die entsprechenden Verdünnungen wurden zur Kontrolle ausplattiert und gezählt. Der Testansatz hat 1 ml und wird in PBST (20 mM NaPhosphat, 120 mM NaCl, pH 7,4, 0,1 % Tween 20) durchgeführt. Als spezifisches Bindungsprotein wird JS-Tag-CBDBa in einer Konzentration von 20 µg/ml vorgelegt. Für die Kontrollversuche wurde statt Protein nur PBST zugegeben. Jeweils 10 µl der Bakterienvorverdünnung werden hinzugegeben, so dass die Konzentrationen der Bakterien im Ansatz jeweils ∼ 10³ KbE/ml betragen. Die Bakterien werden mit den Zellen für 1 Minute inkubiert. Danach werden 100 µg/ml magnetische PA-Streptavidin-Beads (Microcoat) zugegeben und die Ansätze für 20 min bei Raumtemperatur rollend inkubiert. Die Magnetbeads werden für 5 min im Magnetseparator gesammelt und die Überstände abgenommen. Die separierten Magnetpartikel werden 1 mal für 5 min mit 1 ml PBST gewaschen. Die Waschlösung wird mit den Überständen vereinigt. Die Magnetbeads werden mit PBST wieder zu 1 ml ergänzt. Von verschiedenen Verdünnungen werden je 100 µl zum einen auf CASO-Platten (Casein, Sojabohnenextrakt; Vollmedium, Merck) und zum anderen auf PEMBA-Platten (Selektivmedium für *Bacillus cereus,* enthält Polymyxin B, Eigelb, Mannitol) plattiert und bei 27 °C für ca. 18 h inkubiert und anschliessend ausgezählt. Es wurden jeweils Mittelwerte aus 2 Versuchen ermittelt. Die Gesamtzahl der wiedergefundenen Zellen ergab sich aus der Summe der an die Magnetpartikel gebundenen Zellen und den im Überstand bzw. der Waschlösung verbliebenen Zellen. Als Kontrolle wurden die ausplattierten Zellverdünnungen verwendet. Das Ergebnis ist in Figur 12 dargestellt. Es zeigte sich, dass nur bei Zugabe der spezifischen CBD *Bacillus cereus* aus der Probe angereichert wurde, während ohne Zugabe des Proteins keine Bakterien an die Magnetpartikel gebunden wurden. Ebenso zeigt sich, dass über die Beads nur *Bacillus cereus* gebunden wird, während die anderen Bakterien nur auf den CASO-Platten hochwachsen, auf denen der Überstand ausplattiert wurde.

### Experiment 15: Anreicherung von Bacillus cereus aus kohlenhydrathaltigen Lebensmitteln

Als Lebensmittelprobe wurde vorgegarter Express Reis (Spitzen-Langkorn Express Reis, Uncle Ben's) verwendet. 5 g vorgegarter Express Reis wurde steril in einen Stomacher-Beutel überführt, mit 50 ml TSPB-Medium (TS-Vollmedium mit 0,01 mg/ml Polymyxin B) versetzt und 1 min homogenisiert.Eine üN-Kultur von *Bacillus cereus* (DSMZ345) wurde bei 30 °C in TS-Medium angeimpft. 2 ml der üN-Kultur wurden in 10 ml frisches TSPB-Medium überführt und inkubiert bis sie OD₆₀₀ = 0,8 erreicht hatten. 990 µl Lebensmittelprobe aus dem Stomacherbeutel-Filtrat wurden mit je 10 µl aus verschiedenen Verdünnungen der Bacillenvorkultur bespikt, so dass die Bacillenkonzentration in der Lebensmittelprobe 10², 10³, oder 10⁴ KbE/ml betrug. Als Kontrolle wurde TSPB-Medium bespikt. Nach 5 min Inkubation bei Raumtemperatur wurde pro Ansatz 10 µg JS-Tag-CBD-Ba zugegeben und gut gemischt. Die Kontrollansätze erhielten kein Protein. Nach ca. 1 Minute wurden je 400 µg Streptavidin-PA-Magnetbeads (Microcoat) zugegeben und die Proben für 20 min bei Raumtemperatur rollierend inkubiert. Die Magnetbeads werden für 5 min im Magnetseparator gesammelt und die Überstände abgenommen. Die separierten Magnetpartikel werden 2 mal für 5 min mit 1 ml PBST gewaschen. Die Waschlösungen werden mit den Überständen vereinigt. Die Magnetbeads werden mit PBST wieder zu 1 ml ergänzt. Je 100 µl verschiedener Verdünnungen der Ansätze mit den Magnetpartikeln bzw. der Überstände werden auf selektive PEMBA-Platten ausgestrichen, anschliessend über Nacht bei 30 °C inkubiert und dann ausgezählt. Es wurden jeweils 2 Platten parallel ausgestrichen. Das Ergebnis ist in Figur 13 abgebildet. Es zeigte sich, dass praktisch alle *Bacillus cereus* Zellen selektiv sowohl aus der Lebensmittelprobe als auch aus TSPB-Medium an die JS-Tag-CBDBa und anschließend an die Magnetpartikel gebunden wurden, während im Überstand und in den Waschlösungen fast keine *Bacillus cereus* Zellen zurückblieben. In den Kontrollansätzen ohne Protein wurden keine Bacillen unspezifisch an die Magnetpartikel gebunden.

### Experiment 16: Anreicherung von Bacillus cereus aus Blut

*Bacillus cereus* (DSMZ345) wurde in üN (über Nacht)-Kultur bei 30 °C in TB-Medium angezogen. Frisches TB-Medium wurde 1:10 angeimpft und die Bakterien bis OD₆₀₀ ∼ 1 wachsen gelassen. Jeweils 0,5 ml Citrat-, EDTA- oder Heparin-Blut wurden mit je 0,5 ml PBST gemischt. Alle Zusätze zum Blut dienen als Antikoagulantien. Citrat-Blut: 0,106 M Citrat wird 1:10 mit Blut verdünnt. EDTA-Blut: 1,2 - 2 mg EDTA/ml Blut. Heparin-Blut: 10 - 30 I.E. Heparin/ml Blut. Die 1 ml Proben des gepufferten Blutes wurden mit je 10 µl *Bacillus cereus* Vorkultur so bespikt, dass eine Bakterienkonzentration von ca. 10³ KbE/ml vorlag. Dazu wurden 5 µl einer JS-Tag-CBD21-Proteinlösung (2 mg/ml) gegeben und kurz mit dem Vortex gemischt. Die Kontrollen erhielten kein Protein. Danach wurden 40 µl Magnetpartikel (Streptavidin-PA-Beads, Microcoat, 10 mg/ml) zugegeben und die Proben für 20 min bei RT rolliert. Die Separation der Magnetpartikel im Magnetseparator erfolgte für 5 min. Der Überstand enthaltend die nicht gebundenen Bakterien wurde abgenommen und die Beads 1 mal mit 1 ml PBST gewaschen (5 min). Die Waschlösung wurde nach erneuter Magnetseparation mit den Überständen vereinigt. Die Magnetbeads mit dem gebundenen Komplex aus JS-Tag-CBD und Bakterien wurden mit 1 ml PBST ergänzt. Jeweils 100 µl einer 1:10 Verdünnung aus den vereinigten Überständen und der Lösung enthaltend die Magnetpartikel wurde auf selektive PEMBA-Platten ausgestrichen, für ca. 18 h inkubiert und ausgezählt. Es wurden Mittelwerte aus je 2 Versuchen gebildet. Figur 14 zeigt das Ergebnis des Versuchs. Es zeigte sich, dass mit Hilfe der JS-Tag-CBD21 nahezu alle Bacillen aus dem Blut entfernt werden konnten. Bei den Kontrollen ohne Protein zeigte sich, dass lediglich bei Blutproben, denen Heparin zugesetzt wurde, ein gewisser Prozentsatz (ca. 25 %) an unspezifischer Bindung an die Magnetpartikel auftrat. Bei Citrat-Blut und EDTA-Blut war der Anteil an unspezifischer Bindung sehr gering.

### Experiment 17: Clostridienbindung mit JS-Tag-Konstrukten der CBD3626

Ausgehend von dem in der Literatur beschriebenen Endolysin des *Clostridium perfringens* Phagen Φ3626, Ply3626, (Zimmer et al., 2002, Appl. Environm. Microbiol., 68, 5311-5317) wurden mit molekularbiologischen Techniken verschiedene Varianten einer potentiell geeigneten CBD3626 mit JS-Tag hergestellt. Da sich anfangs alle Konstrukte sehr schlecht exprimieren ließen, wurde ein synthetisches Gen hergestellt, das auf die unterschiedliche Codonusage in Clostridien und E. coli hin optimiert war. Es stellte sich auch heraus, dass konventionelle Varianten, die mit His-Tag kloniert wurden, nur sehr schlecht löslich waren, die Varianten mit JS-Tag jedoch besser löslich waren. Die Varianten, die sich als geeignet herausstellten, um stabil und löslich exprimiert zu werden, so dass sie anschließend gereinigt und in einen funktionierenden Bindetest mit *Clostridium perfringens* Zellen eingesetzt werden konnten, sind in Tabelle 4 zusammengestellt. Die Klonierung erfolgte jeweils in den Vektor Pet21d. Die Expression erfolgte im Stamm *E. coli* HMS174 bei 30 °C in LB-Medium mit Ampicillin und Chloramphenicol unter Koexpression von birA auf einem weitem Plasmid.

**Tabelle 4**

| **Protein-Abkürzung** | **Anteil von Ply3626 (Aminosäure)** | **JS-Tag Variante** | **GFP** | **Molekular-gewicht (kDa)** |
|---|---|---|---|---|
| JS4a_GFP_CBD3626_150 | 150-347 | JS(4a) | + | 58 |
| JS10a_GFP _CBD3626_150 | 150-347 | JS(10a) | + | 58 |
| Js4a_GFP-CBD3626_178 | 178-347 | JS(4a) | + | 55 |
| Js10a_GFP-CBC3626_178 | 178-347 | JS(10a) | + | 55 |
| Js4a_CBD3626_150 | 150-347 | JS(4a) | - | 23 |
| Js10a_CBD3626_178 | 178-347 | JS(10a) | - | 20 |

Zum Bindetest wurde eine Vorkultur von *Clostridium perfringens* bei 45 °C über Nacht in TYG-Medium (Trypton, Hefeextrakt, Glucose) in einer Anaerobierbox gezogen. Die Bindetests wurden in 1ml PBST-Puffer bei Raumtemperatur durchgeführt. Clostridium Zellen wurden in einer Konzentration von ca. 10⁴ KbE/ml vorgelegt und die verschiedenen JS-Tag-CBD3626-Konstrukte wurden in einer Konzentration von 20 µg/ml hinzugegeben, gemischt und etwa 2 min inkubiert. Anschliessend wurde PA-Streptavidin-beads (Microcoat) in einer Konzentration von 100 µg/ml hinzugegeben und 10 min rollierend inkubiert. Von den CBD-Konstrukten, die GFP als Marker enthielten, wurden jeweils Proben entnommen und unter dem Fluoreszenzmikroskop auf Bindung analysiert. Das Ergebnis ist in Figur 15 abgebildet.

### Experiment 18: Bindung von Staphylokokken mit spezifischen JS-Tag-CBD-Konstrukten

ÜN-Kulturen von Staphylococcus-Stämmen wurden 1:10 in BHI-Medium verdünnt und bei 37 °C bis zu einer OD₆₀₀ von ca. 1 inkubiert. Die Zellen wurden in Medium verdünnt und in einer Konzentration von 10⁴ KbE/ml in den Test eingesetzt (Testansatz 500 µl, PBST-Puffer). Spezifisches Bindeprotein JS5b_CBDUSA wurde in einer Konzentration von 10 µg/ml zugegeben und 20 min bei RT mit den Zellen inkubiert. Anschliessend wurden PA-Streptavidin-Beads (Microcoat) in einer Konzentration von 200 µg/ml zugegeben und für 45 min im Über-Kopf-Rollator inkubiert. Die Magnetpartikel mit gebundenen Zellen wurden 5 min im Magnetseparator inkubiert, einmal mit 1 ml Puffer gewaschen und wieder in 500 µl Puffer resuspendiert. Die Magnetpartikel und die vereinigten Überstände wurden auf CASO-Platten ausplattiert und über Nacht bei 37 °C inkubiert. Die Platten mit *Staphylococcus* aureus und *S. haemolyticus* konnten nach 16 h ausgezählt werden, die Platten mit S. *epidermidis* erst nach 24 h. Untersucht wurde der Anteil an gebundenen Staphylokokken im Vergleich zu den insgesamt gefundenen Bakterien.

**Tabelle 5**

| **Stamm** | **JS5b_CBDUSA** |
|---|---|
| Staphylococcus aureus | +++ |
| Staphylococcus haemolyticus | ++ |
| Staphylococcus *epidermidis* | + |
| % Zellbindung: < 10 %: o; 10 % -25 % : +; 25 % - 50 %: ++; > 50 %: +++ | |

Mit dem JS5b-CBDUSA-Konstrukf können verschiedene Stämme von Staphylokokken gebunden werden, wobei *S*. *aureus* am besten bindet.

### Experiment 19: Erfindungsgemäße Polypeptidkonstrukte mit CBD und JS-Tag binden Staphylococcus Zellen im Bead-sbBindetest wesentlich besser als eine CBD mit einer Kombination aus Strep-Tag und His-Tag (NS-His).

Die Durchführung des Versuchs erfolgte im Zellbindetest analog zu Experiment 18. Erfindungsgemäße JS-Tag Polypeptidkonstrukte der Zellbindedomäne der CBDPitti26 (SEQ ID No 28) wurden im Vergleich zu einem Konstrukt eingesetzt, das sowohl einen Strep-Tag für die Biotinbindung sowie einen His-Tag (Kombination NS-His) enthielt. Die CBD Konstrukte besitzen den NS-His- bzw. Js-Tag (Variante 5b) am N-Terminus, gefolgt von einer Linkersequenz (AGAGAGAGSEL) und der CBDPitti26 Sequenz. Pitti26 ist ein Eigenisolat eines Staphylococcusphagen. Die Polypeptidkonstrukte wurden vor dem Test frisch dialysiert, die Proteinkonzentration per UV-Absorptionsmessung bestimmt und die Proteine im Test in einer Konzentration von 10 µg/ml eingesetzt. Tabelle 6 zeigt das Bindungsverhalten an 3 verschiedene *Staphylococcus* Stämme.

**Tabelle 6**

| CBD Konstrukt | Staphylococcus aureus (Patientenisolat) | Staphylococcus epidermidis (DSMZ 20044) | Staphylococcus haemolyticus (DSMZ 20263) |
|---|---|---|---|
| NS-His-CBDPitti26 | 0 % | 1,1 % | 0,0 % |
| JS-CBDPitti26 | 1,0 % | 61,3 % | 5,0 % |

Während mit dem erfindungsgemäßen JS-Tag-CBD-Konstrukt bei allen 3 *Staphylococcus* Stämmen Zellbindung nachgewiesen werden konnte, wenn auch mit deutlicher Präferenz für *Staphylococcus epidermidis* Zellen, konnte unter denselben Bedingungen beim Konstrukt mit Strep-Tag und His-Tag nur für die *Staphylococcus epidermidis* Zellen eine sehr schwache Bindung erreicht werden. Dies zeigt, dass die erfindungsgemäßen Konstrukte sich wesentlich besser für die Bindung von Bakterienzellen eignen als CBDs, die mit anderen Tags gekoppelt sind.

### Experiment 20: Nachweis der Staphylokokkenbindung durch Staphylococcus spezifische JS-Tag-CBD-Konstrukte im Zellbindetest.

Analog zu der in Experiment 18 beschriebenen Versuchsanordnung wurde eine Vielzahl von verschiedenen *Staphylococcus* Stämmen unter Verwendung von 3 verschiedenen für *Staphylococcus* spezifischen JS-Tag-CBD-Konstrukten getestet. Die CBD-Anteile der verschiedenen Konstrukte stammen aus dem Phagen ΦSA2usa - JS-CBDUSA, bzw. aus Endolysinen aus den Phageneigenisolaten PlyOpf - JS-CBDOpf - und PlyPitti20 - JS-CBDPitti20. Die Polypeptidsequenzen sind unter den SEQ ID NOs 21, 31, bzw 27 zu finden. Die Versuchsergebnisse sind in Abbildung 16 dargestellt.

Die erfindungsgemäßen Polypeptidkonstrukte JS-CBDUSA, JS-CBDOpf und JS-CBDPitti20 binden eine Vielzahl von *Staphylococcus* Stämmen spezifisch im Beadsbindetest. Dabei werden *Staphylococcus aureus* MRSA Stämme ähnlich gut gebunden wie nicht MRSA Stämme (Fig. 16A). Als Tendenz lässt sich jedoch feststellen, dass JS-CBDOpf die MRSA-Stämme besser bindet während JS-CBDUSA die nicht MRSA-Stämme besser bindet. Abbildung 16B fasst die Bindung der 3 erfindungsgemäßen *Staphylococcus* JS-CBD Konstrukte an eine Reihe weiterer *Staphylococcus* Stämme zusammen, die nicht der Spezies *Staphylococcus aureus* angehören. Stämme der Spezies *Staphylococcus carnosus, Staphylococcus sciuri* und *Staphylococcus equorum* (mit einer Ausnahme) werden dabei nicht spezifisch gebunden, Stämme der Spezies *Staphylococcus epidermidis, Staphylococcus haemolyticus, Staphylococcus saprophyticus, Staphylococcus simulans, Staphylococcus warneri* und *Staphylococcus xylosus* hingegen schon. Dies zeigt, dass die verwendeten erfindungsgemäßen JS-Tag-CBD-Konstrukte geeignet sind, um eine große Anzahl humanpathogener *Staphylococcus* Stämme spezifisch und effizient zu binden.

Experiment 21: Nachweis der Zellbindung an JS-Tag-CBD-Konstrukte mit Hilfe des Peroxidasetests. Der Peroxidasetest beruht auf dem Prinzip, dass ein Messsignal nur erhalten wird, wenn das verwendete StrepTactin-HRP(Horse Radish Peroxidase)-Konjugat (IBA, Göttingen) über den StrepTactin-Anteil an Biotin binden kann. Dieses Biotin befindet sich kovalent gebunden am biotinylierten JS-Tag der erfindungsgemäßen Polypeptidkonstrukte, die wiederum im Versuchsansatz spezifisch an Bakterienzellen gebunden haben und sich somit in den Pelletfraktionen befinden, die bei den Zentrifugationsschritten zurückgehalten werden. Nicht gebundene JS-Tag-CBD-Konstrukte würden mit den jeweiligen Zentrifugationsüberständen verworfen und deshalb kein Signal geben. Die erfindungsgemäßen JS-Tag-Polypeptidkonstrukte werden vor dem Einsatz im Peroxidasetest über Nacht gegen TE-Puffer (20 mM Tris, 50 mM NaCl, 5 mM EDTA; pH 7,0) dialysiert und anschließend die Proteinkonzentration über UV-Absorption anhand des spezifischen Extinktionskoeffizienten bestimmt. Mikrotiterplatten (Deepwell) werden durch Inkubation mit je 600 µl PBST für 1 h bei 37 °C blockiert. Anschließend werden je 200 µl Bakterienvorkultur bzw. BHI-Medium (als Kontrolle) in die Vertiefungen pipettiert und mit entsprechenden Mengen an Proteinlösung (Proteinkonzentration 10 µg/ml) bzw. Puffer (als Kontrolle) versetzt und für 15 min bei RT inkubiert. Es werden jeweils Dreifachbestimmungen durchgeführt. Nach Inkubation werden die Platten für 10 min bei 4 °C in einer Tischzentrifuge (3600 rpm) zentrifugiert, die Überstände abgenommen und verworfen und die Pellets nochmals mit 200 µl PBST gewaschen. Anschließend werden die Pellets in 200 µl StrepTactin-HRP-Konjugat-Lösung (1:5000 verdünnt in PBST) resuspendiert und 30 min bei RT inkubiert. Die Proben werden erneut zentrifugiert, noch 2x mit je 200 µl PBST gewaschen und die Überstände dabei jeweils verworfen. Nach dem 2. Waschschritt werden die Pellets in je 200 µl ABTS-Reaktionslösung aufgenommen und die Farbreaktion über die Absorption bei 405 nm (korrigiert um die Hintergrundabsorption bei 600 nm) über maximal 30 min gemessen. Die ABTS-Reaktionslösung setzt sich folgendermaßen zusammen: 18 ml Mc-Ilvains-Puffer (0,1 M Zitronensäure, 0,2 M Na₂HPO₄, pH 5,0) plus 2 ml 1 % ABTS (2,2 Azino-bis(3-ethyl)benzthiazolin 6-Sulfonsäure) in Wasser plus 100 µl H₂O₂ (1 % ige Lösung).
Peroxidasetestergebnisse unter Verwendung verschiedener erfindungsgemäßer CBD-Konstrukte sind in der Abbildung 17 dargestellt. Bei JS-CBDOpf und JS-CBDPitti20 handelt es sich um CBD-Konstrukte aus Staphylococcusphageneigenisolaten. JS-CBDUSA stammt aus dem Phagen ΦSA2usa. Die CBD-Anteile aus den staphylolytischen Enzymen ALE-1 und Lysostaphin in den Konstrukten JS-CBDALE-1 und JS-CBDLS stammen aus den Bakterienstämmen *Staphylococcus capitis* EPK1 bzw. *Staphylococcus simulans.* Die entsprechenden Gene wurden jedoch synthetisch hergestellt und zur besseren Expression der Proteine an die *E. coli* "Codon usage" angepasst.

In Abbildung 17A ist dargestellt, dass JS-CBDALE-1 (SEQ ID No 50) und JS-CBDLS (SEQ ID No 49) verschiedene Koagulase positive *S. aureus* Stämme (MRSA und nicht MRSA) sowie die Koagulase negativen *S. epidermidis* und *S*. *haemolyticus* spezifisch und mit hoher Effizienz binden. Beide JS-Tag-Konstrukte binden die Staphylokokken mit etwa gleicher Effizienz.

Abbildung 17B zeigt, dass die erfindungsgemäßen Polypeptide JS-CBDALE-1 (SEQ ID No 50), JS-CBDLS (SEQ ID No49), JS-CBDPitti20 (SEQ ID No 47), JS-CBDOpf (SEQ ID No 51) sowie JS-CBDUSA (SEQ ID No 46) verschiedene *Staphylococcus* Stämme wie *S. aureus* (MRSA und nicht MRSA) und *S. haemolyticus* Stämme im Peroxidasetest spezifisch binden, während der gram negative *E. coli* Stamm, aber auch der gram positive pathogene Stamm *Streptococcus nautans* nicht gebunden werden. Besonders hervorzuheben ist die starke Zellbindung durch das Polypeptidkonstrukt JS-CBDPitti20.

### Experiment 22: Nachweis der Zellbindung an Enterococcus spezifische JS-Tag-CBD-Konstrukte mit Hilfe des Peroxidasetests.

Die Durchführung des Peroxidasetests erfolgte wie in Experiment 21 beschrieben. Peroxidasetestergebnisse unter Verwendung verschiedener erfindungsgemäßer CBD-Konstrukte sind in der Abbildung 18 dargestellt. Bei JS-CBDEF0355 und JS-CBDEF1293 handelt es sich um 2 erfindungsgemäße JS-Tag-CBD-Konstrukte, bei denen die CBDs aus zwei putativen Prophagenendolysinen stammen, die in dem komplett veröffentlichten Genom des Stammes *Enterococcus faecalis* V583 (Accession Number: NC_004668) unter den "Locus Tags" EF_0355 bzw. EF_1293 zu finden sind. Die entsprechenden Gene wurden jedoch synthetisch hergestellt und zur besseren Expression der Proteine an die *E. coli* "Codon usage" angepasst. Die CBDs wurden so erhalten, dass aus der kompletten Endolysinsequenz die konservierten Domänen für die EADs deletiert wurden und danach mit Sequenzanalyseprogrammen nach potentiellen Domänenlinkern innerhalb des Proteins gesucht wurde. Als neuer Linker zwischen JS-Tag und CBD wurde dann jeweils die Sequenz AGAGAGAGSEL (SEQ ID No. 35) eingeführt. Es wurden jeweils 10 µg/ml JS-Tag-CBD-Konstrukt im Test eingesetzt. Die Versuchsergebnisse sind in Abbildung 18 dargestellt.

Abbildung 18A zeigt, dass alle getesteten *Enterococcus faecalis* Stämme im Peroxidasetest spezifisch gebunden werden, da die Kontrollen ohne Zugabe des JS-Tag-CBD-Konstruktes ein deutlich geringeres Messignal liefern. Die beiden Konstrukte JS-CBDEF0355 und JS-CBDEF1293 liefern bei den meisten Stämmen sehr ähnliche Ergebnisse.

Abbildung 18B zeigt, dass auch die getesteten *Enterococcus faecium* Stämme spezifisch gebunden werden, wenn sie auch zum Teil etwas geringere Absorptionssignale liefern. Auch ein *Staphylococcus aureus* Stamm wurde mit guter Effizienz gebunden, so dass die Bindung nicht sehr spezifisch für die Gattung *Enterococcus* ist. Dieses Verhalten war jedoch aus Yoong et al. (J. Bact., 2004, 186, 4808-4812) schon bekannt, wo gezeigt wurde, dass neben Enterokokken auch Streptokokken und Staphylokokken gebunden werden.

## Patentansprüche

1. Polypeptid umfassend:
i) eine enzymatisch nicht aktive, Zellwand-bindende Domäne eines Endolysins oder eines anderen Zellwand lysierenden Enzyms, und
ii) eine Sequenz gemäß SEQ ID NO: 1 oder Derivate gemäß SEQ ID NO: 2-18 davon,
wobei das Polypeptid neben der Zellwand-bindenden Domäne keine weiteren Domänen eines Endolysins oder anderen Zellwand lysierenden Enzyms umfasst und wobei die Zellwand-bindende Domäne gram-positive Bakterien bindet.

2. Polypeptid gemäß Anspruch 1, wobei das andere Zellwand lysierende Enzym ausgewählt wird aus der Gruppe bestehend aus Autolysinen, Bakteriocinen und Phagenschwanzproteinen.

3. Polypeptid gemäß einem der vorhergehenden Ansprüche, wobei das Polypeptid biotinyliert ist.

4. Polypeptid gemäß einem der vorhergehenden Ansprüche, wobei die Zellwand-bindende Domäne des Polypeptids oder des anderen Zellwand lysierenden Enzyms die Fähigkeit aufweist, gram positive Bakterien zu binden.

5. Polypeptid gemäß Anspruch 4, wobei die gram positiven Bakterien ausgewählt sind aus der Gruppe bestehend aus Clostridien, Bazillen, Listerien, Staphylokokken, Lactobazillen, Enterokokken, Aerokokken, Pediokokken, Streptokokken, Mycoplasmen und/oder Leuconostoc.

6. Polypeptid gemäß einem der vorhergehenden Ansprüche, wobei die Zellwand-bindende Domäne ausgewählt wird aus der Gruppe der Zellwand-bindende Domänen der Endolysine oder anderen Zellwand lysierenden Enzyme Ply511, Ply 500, Ply 118, PlyPSA, EGDe, PLyL, PlyG, PlyPH, PlyB, PlyBa, Ply21, Ply12, der *Enterococcus faecalis* V583 Prophagen Endolysine, Ply3626, Lysine aus Cl. perfringens Stamm 13 und Stamm SM101, ΦP1 Lysin, PlyV12, PlyC, PlyGBS, Cpl-1, Cpl-7, Cpl-9, Pal Amidase, Twort Amidase, *S*. *aureus* phage PVL amidase, P68 lys16, ΦSA2usa endolysin , Phi11 und Phi12 Endolysin, Zellwandhydrolyse des Staphylococcus aureus Phagen Phi 11, Phage B30 Endolysin, Phage 168 Endolysin, LysK, *S. simulans* Lysostaphin, *S*. *capitis* ALE-1 Endopeptidase, Phage PhiNIH1.1 Zellwandhydrolase, LytM, Atl, LytA aus Streptococcus pneumoniae, aus *Staphylococcus aureus* Stamm PS47 stammende Peptidoglykanhydrolase, Enterolysin A aus Enterococcus faecalis, Ami Autolysin von L. *monocytogenes,* Lactobacillus Lysine wie Lysin des Phagen A2 oder Phage PL-1 Amidase.

7. Polypeptid gemäß einem der vorhergehenden Ansprüche, wobei die enzymatisch nicht aktive Zellwand-bindende Domäne eines Endolysins eine Sequenz gemäß SEQ ID NO: 19-33 aufweist.

8. Polypeptid gemäß einem der vorhergehenden Ansprüche, das zwischen der enzymatisch nicht aktiven Zellwand-bindenden Domäne eines Endolysins, und der Sequenz gemäß SEQ ID NO: 1 oder Derivaten davon einen Spacer aufweist.

9. Verwendung eines der Polypeptide gemäß einem der Ansprüche 1 bis 8, um gram-positive Bakterien zu binden, anzureichern, aus einer Probe zu entfernen, einzufangen und/oder nachzuweisen.

10. Verfahren zur Anreicherung, zur Entfernung, zum Einfangen und/oder dem Nachweis von gram-positiven Bakterien aus einer Probe, umfassend die Schritte:
a) In Kontakt Bringen und/oder Inkubation einer Probe mit einem biotinylierten Polypeptid gemäß einem der Ansprüche 1 bis 8,
b) In Kontakt Bringen und/oder Inkubation der durch Schritt a) erhaltenen Polypeptid-Bakterien-Komplexe mit einem Träger, der mit einer Biotin-bindenden Substanz versehen ist.
c) Trennen des durch Schritt b) erhaltenen Träger-Polypeptid-Balcterien-Komplexes von der Probe,
d) gegebenenfalls Abwaschen unspezifisch anhaftender Bestandteile der Probe von dem Träger-Polypeptid-Bakterien-Komplex,
e) gegebenenfalls Abtrennen des Trägers vom Polypeptid-Bakterien Komplex, und
f) gegebenenfalls Nachweis der Bakterien.

11. Verfahren nach Anspruch 10, wobei die biotin-bindende Substanz Avidin oder Streptavidin umfasst.

12. Verfahren nach Anspruch 11, wobei das Verfahren mittels magnetischen, chromatographischen oder "Batch"-Verfahren erfolgt.

13. Verfahren nach einem der Ansprüche 10 bis 12, wobei der Nachweis in Schutt f) mittels selektiven Wachstumsbedingungen, nukleinsäurebasierten Methoden, Nachweis der Bakterien-Zellwand bzw. ihrer Komponenten, Nachweis von Bakterien-Bestandteilen, mittels einer weiteren spezifischen Zellwand-bindenden Domäne, die mit einem Marker gekoppelt ist, und/oder mittels einer Kombination aus mikrobiologischen, morphologischen und/oder biochemischer Nachweismethoden erfolgt.

14. Verfahren nach Anspruch 13, wobei die nukleinsäurebasierten Methoden ausgewählt sind aus PCR, RT-PCR, PCR-RFLP, rep-PCR-fingerprinting, NASBA, DNA-Hybridisierungsmethoden, Multilocus Sequenz Typisierung (MLST) und rRNA-Vergleichen.

15. Verfahren nach Anspruch 13, wobei der Nachweis der Bakterien-Zellwand bzw. ihrer Komponenten über Zellbindedomänen von Endolysinen, Antikörper oder per FTIR erfolgt.

16. Verfahren nach Anspruch 13, wobei der Nachweis der Bakterien-Bestandteile über ELISA, Enzymaktivität, Multilocus Enzymelektrophorese (MEE) oder einen Biolumineszenzassay erfolgt.

## Claims

1. Polypeptide comprising:
i) an enzymatic non-active cell wall binding domain of an endolysin or another cell wall lysing enzyme, and
ii) a sequence according to SEQ ID NO: 1 or derivatives according to SEQ ID NOs: 2-18 thereof,
wherein the polypeptide comprises besides the cell wall binding domain no further domains of an endolysin or another cell wall lysing enzyme, and wherein the cell wall binding domain binds gram-positive bacteria.

2. Polypeptide according to claim 1, wherein the other cell wall lysing enzyme is selected from the group consisting of autolysins, bacteriocins and phage tail proteins.

3. Polypeptide according to any one of the preceding claims, wherein the polypeptide is biotinylated.

4. Polypeptide according to any one of the preceding claims, wherein the cell wall binding domain of the polypeptide or the other cell wall lysing enzyme exhibits the capability to bind gram-positive bacteria.

5. Polypeptide according to claim 4, wherein the gram-positive bacteria are selected from the group consisting of clostridii, bacilli, listeria, staphylococci, lactobacilli, enterococci, aerococci, pediococci, streptococci, mycoplasma and/or leuconostoc.

6. Polypeptide according to any one of the preceding claims, wherein the cell wall binding domain is selected from the group of the cell wall binding domains of the endolysins or other cell wall lysing enzymes Ply511, Ply500, Ply118, PlyPSA, EGDe, PlyL, PlyG, PlyPH, PlyB, PlyBa, Ply21, Ply12, of the *Enterococcus faecalis* V583 prophage endolysins, Ply3626, lysins from Cl. perfringens strain 13 and strain SM101, ΦP1 lysin, PlyV12, PlyC, PlyGBS , Cpl-1, Cpl-7, Cpl-9, Pal Amidase, Twort Amidase, *S. aureus* phage PVL amidase, P68 lys16, ΦSA2usa endolysin, Phi11 and Phi12 endolysin, cell wall hydrolysis of the Staphylococcus aureus phage Phi 11, phage B30 endolysin, phage 168 endolysin, LysK, *S*. *simulans* Lysostaphin, *S*. *capitis* ALE-1 endopeptidase, phage PhiNIH1.1 cell wall hydrolase, LytM, Atl, LytA from Streptococcus pneumoniae, from *Staphylococcus aureus* strain PS47 deriving peptidoglycan hydrolase, enterolysin A from Enterococcus faecalis, ami autolysin from *L*. *monocytogenes,* lactobacillus lysin such as lysin of the phage A2 or phage PL-1 amidase.

7. Polypeptide according to any one of the preceding claims, wherein the enzymatic non-active cell wall binding domain of an endolysin exhibits a sequence according to SEQ ID NO: 19-33.

8. Polypeptide according to any one of the preceding claims, exhibiting a spacer between the enzymatic non-active cell wall binding domain of an endolysin and the sequence according to SEQ ID NO: 1 or derivatives thereof.

9. Use of one of the polypeptides according to any one of the claims 1 to 8 to bind, enrich, remove from a sample, capture and/or detect gram-positive bacteria.

10. Method for the enrichment, for the removal, for the capture and/or for the detection of gram-positive bacteria from a sample, comprising the steps:
a) Contacting and/or incubating a sample with a biotinylated polypeptide according to any one of the claims 1 to 8,
b) Contacting and/or incubating the polypeptide-bacteria-complexes obtained in step a), with a carrier supplied with a biotin-binding substance.
c) Separating the carrier-polypeptide-bacteria-complex obtained in step b), from the sample,
d) optionally washing unspecifically attached components of the sample from the carrier-polypeptide-bacteria-complex,
e) optionally separation of the carrier from the polypeptide-bacteria-complex, and
f) optionally detection of the bacteria.

11. Method according to claim 10, wherein the biotin-binding substance comprises avidin or streptavidin.

12. Method according to claim 11, wherein the method is carried out via magnetic, chromatographic or batch-methods.

13. Method according to any one of the claims 10 to 12, wherein the detection in step f) is carried out via selective growth conditions, nucleic acid based methods, detection of the bacteria cell wall and its components, respectively, detection of bacteria components via a further specific cell wall binding domain coupled to a marker, and/or via a combination of microbiological, morphological and/or biochemical detection methods.

14. Method according to claim 13, wherein the nucleic acid based methods are selected from PCR, RT-PCR, PCR-RFLP, rep-PCR-fingerprinting, NASBA, DNA-hybridisation methods, multi-locus sequence typing (MLST) and rRNA-comparisons.

15. Method according to claim 13, wherein the detection of the bacteria cell wall and its components, respectively, is carried out via cell binding domains of endolysins, antibodies or via FTIR.

16. Method according to claim 13, wherein the detection of the bacteria components is carried out via ELISA, enzyme activity, multi-locus enzyme electrophoresis (MEE) or a bioluminescence assay.

## Revendications

1. Polypeptide comprenant :
i) un domaine enzymatiquement non actif se liant à la paroi cellulaire d'une endolysine ou d'une autre enzyme lysant la paroi cellulaire, et
ii) une séquence selon SEQ ID NO: 1 ou des dérivés de celle-ci selon SEQ ID NO: 2-18,
où le polypeptide ne comprend aucun autre domaine d'une endolysine ou d'une autre enzyme lysant la paroi cellulaire à côte du domaine se liant à la paroi cellulaire, et où le domaine se liant à la paroi cellulaire lie des bactéries à Gram positif.

2. Polypeptide selon la revendication 1, où l'autre enzyme lysant la paroi cellulaire est sélectionnée dans le groupe constitué par les autolysines, les bactériocines et les protéines de la queue du phage.

3. Polypeptide selon l'une des revendications précédentes, où le polypeptide est biotinylé.

4. Polypeptide selon l'une des revendications précédentes, où le domaine se liant à la paroi cellulaire du polypeptide ou de l'autre enzyme lysant la paroi cellulaire a la capacité de lier des bactéries à Gram positif.

5. Polypeptide selon la revendication 4, où les bactéries à Gram positif sont sélectionnées dans le groupe constitué par les clostridies, bacilles, listéries, staphylocoques, lactobacilles, entérocoques, aérocoques, pédiocoques, streptocoques, mycoplasmes et/ou leuconostocs.

6. Polypeptide selon l'une des revendications précédentes, où le domaine se liant à la paroi cellulaire est sélectionné dans le groupe constitué par les domaines se liant à la paroi cellulaire des endolysines ou autres enzymes lysant la paroi cellulaire PlyB511, Ply 500, Ply 118, PlyPSA, EGDe, PLyL, PlyG, PlyPH, PlyB, PlyBa, PlyC21, Ply12, des endolysines du prophage *Enterococcus faecalis* V583, Ply3626, lysines de souche 13 et souche SM101 de Cl. perfringens, lysine ΦP1, PlyV12, PlyC, PlyGBS, Cpl-1, Cpl-7, Cpl-9, amidase Pal, amidase de Twort, amidase du phage PVL S. *aureus,* P68 lys16, endolysine ΦSA2usa, endolysine Phi11 et Phi12, hydrolyse de la paroi cellulaire du phage Phi 11 Staphilococcus aureus, endolysine du phage B30, endolysine du phage 168, LysK, lysostaphine de S. *simulans,* endopeptidase ALE-1 de *S*. *capitis,* hydrolase de la paroi cellulaire du phage PhiNIH1.1, LytM, Atl, LytA de Streptococcus pneumoniae, hydrolase de peptidoglycane provenant de la souche PS47 de *Staphilococcus aureus,* entérolysine A d'Enterococcus faecalis, autolysine Ami de *L. monocytogenes,* lysines de Lactobacillus telles que lysine du phage A2 ou amidase du phage PL-1.

7. Polypeptide selon l'une des revendications précédentes, où le domaine enzymatiquement non actif se liant à la paroi cellulaire d'une endolysine présente une séquence selon SEQ ID NO: 19-33.

8. Polypeptide selon l'une des revendications précédentes, qui comporte un espaceur entre le domaine enzymatiquement non actif se liant à la paroi cellulaire d'une endolysine et la séquence selon SEQ ID NO: 1 ou des dérivés de celle-ci.

9. Utilisation d'un des polypeptides selon l'une des revendications 1 à 8 pour lier des bactéries à Gram positif, les enrichir, les éliminer d'un échantillon, les capturer et/ou les détecter.

10. Procédé pour l'enrichissement, l'élimination, la capture et/ou la détection de bactéries à Gram positif dans un échantillon, comprenant les étapes suivantes :
a) mise en contact et/ou incubation d'un échantillon avec un polypeptide biotinylé selon l'une des revendications 1 à 8,
b) mise en contact et/ou incubation des complexes polypeptide-bactéries obtenus à l'étape a) avec un support doté d'une substance liant la biotine,
c) séparation du complexe polypeptide-bactéries-support obtenu à l'étape b) de l'échantillon,
d) le cas échéant, lavage des composants non spécifiquement adhérents de l'échantillon du complexe polypeptide-bactéries-support,
e) le cas échéant, séparation du support du complexe polypeptide-bactéries, et
f) le cas échéant, détection des bactéries.

11. Procédé selon la revendication 10, où la substance liant la biotine comprend de l'avidine ou de la streptavidine.

12. Procédé selon la revendication 11, où le procédé est exécuté au moyen de procédés magnétiques, chromatographiques ou « batch ».

13. Procédé selon l'une des revendications 10 à 12, où la détection de l'étape f) est effectuée au moyen de conditions de croissance sélectives, de méthodes à base d'acide nucléique, de la détection de la paroi cellulaire des bactéries ou de ses composants, de la détection de composants des bactéries, au moyen d'un autre domaine spécifique se liant à la paroi cellulaire couplé à un marqueur, et/ou au moyen d'une combinaison de méthodes de détection microbiologiques, morphologiques et/ou biochimiques.

14. Procédé selon la revendication 13, où les méthodes à base d'acide nucléique sont sélectionnées parmi PCR, RT-PCR, PCR-RFLP, prise d'empreintes par rep-PCR, NASBA, méthodes d'hybridation ADN, typage par séquençage multilocus (MLST) et comparaisons d'ARNr.

15. Procédé selon la revendication 13, où la détection de la paroi cellulaire des bactéries ou de ses composants est effectuée au moyen de domaines liants cellulaires d'endolysines, d'anticorps ou par FTIR.

16. Procédé selon la revendication 13, où la détection de composants des bactéries est effectuée par ELISA, activité enzymatique, électrophorèse enzymatique multilocus (MEE) ou d'un test de bioluminescence.
